# EUROPEAN PATENT SPECIFICATION

(11) **EP 2 762 562 B1**
(45) Date of publication and mention of the grant of the patent: **22.05.2019**
(21) Application number: 12832061.1
(22) Date of filing: 14.09.2012
(51) Int. Cl.: C12Q 1/6844

(54) **METHOD FOR DETECTING TARGET NUCLEIC ACID**
VERFAHREN ZUM NACHWEIS EINER ZIELNUKLEINSÄURE
PROCÉDÉ DE DÉTECTION D'UN ACIDE NUCLÉIQUE CIBLE

(30) Priority: 14.09.2011 WO PCT/JP2011/071048; 03.08.2012 JP 2012173347
(43) Date of publication of application: 06.08.2014
(73) Proprietor: NGK Insulators, Ltd., Nagoya-shi, Aichi 467-8530 (JP)
(72) Inventor: NIWA, Kousuke, Nagoya-shi Aichi 467-8530 (JP); HIROTA, Toshikazu, Nagoya-shi Aichi 467-8530 (JP); KAWASE, Mitsuo, Nagoya-shi Aichi 467-8530 (JP)
(74) Representative: Mewburn Ellis LLP
(86) International application number: PCT/JP2012/073710
(87) International publication number: WO 2013/039228

(56) References cited:
- WO-A1-92/08728
- WO-A1-03/050305
- WO-A2-00/20556
- WO-A2-02/24944
- WO-A2-97/24455
- WO-A2-99/32663
- WO-A2-2004/039825
- WO-A2-2011/100057
- JP-A- 2010 014 507
- JP-A- 2010 014 507
- US-A- 6 037 127
- URDEA, MICKEY S. ET AL.: 'A comparison of non-radioisotopic hybridization assay methods using fluorescent, chemiluminescent and enzyme labeled synthetic oligodeoxyribonucleotide probes.' NUCLEIC ACIDS RESEARCH vol. 16, no. 11, 1988, pages 4937 - 4956, XP001080119

### SEQUENCE LISTING

<110> NGK INSULATORS, LTD.
<120> Method of Detecting Target Nucleotide
<130> K12-359-PCT
<160> 136
<170> PatentIn version 3.3
<210> 1
   <211> 23
   <212> DNA
   <213> Artificial
<220>
   <223> probe
<400> 1
   tgttctctga ccaatgaatc tgc 23
<210> 2
   <211> 23
   <212> DNA
   <213> Artificial
<220>
   <223> probe
<400> 2
   tggaactggg aacgctttag atg 23
<210> 3
   <211> 23
   <212> DNA
   <213> Artificial
<220>
   <223> probe
<400> 3
   ttcgcttcgt tgtaatttcg gac 23
<210> 4
   <211> 23
   <212> DNA
   <213> Artificial
<220>
   <223> probe
<400> 4
   aggcatccta agaaatcgct act 23
<210> 5
   <211> 23
   <212> DNA
   <213> Artificial
<220>
   <223> probe
<400> 5
   tagcccagtg atttatgaca tgc 23
<210> 6
   <211> 23
   <212> DNA
   <213> Artificial
<220>
   <223> probe
<400> 6
   cgctctggtt actattggac gtt 23
<210> 7
   <211> 23
   <212> DNA
   <213> Artificial
<220>
   <223> probe
<400> 7
   tagccaactc taaataacgg acg 23
<210> 8
   <211> 23
   <212> DNA
   <213> Artificial
<220>
   <223> probe
<400> 8
   ttcggttgtc gatatgagga tct 23
<210> 9
   <211> 23
   <212> DNA
   <213> Artificial
<220>
   <223> probe
<400> 9
   ggggggtact tcatacaaga tgc 23
<210> 10
   <211> 23
   <212> DNA
   <213> Artificial
<220>
   <223> probe
<400> 10
   gagtagcagg caaataccct aga 23
<210> 11
   <211> 23
   <212> DNA
   <213> Artificial
<220>
   <223> probe
<400> 11
   gcctattaag gtctacgtca tcg 23
<210> 12
   <211> 23
   <212> DNA
   <213> Artificial
<220>
   <223> probe
<400> 12
   agtcatacag tgaggaccaa atg 23
<210> 13
   <211> 23
   <212> DNA
   <213> Artificial
<220>
   <223> probe
<400> 13
   cattcgacat aagctgttga tgc 23
<210> 14
   <211> 23
   <212> DNA
   <213> Artificial
<220>
   <223> probe
<400> 14
   tgctcactta cattacgtcc atg 23
<210> 15
   <211> 23
   <212> DNA
   <213> Artificial
<220>
   <223> probe
<400> 15
   tacacctatc aactcgtaga gca 23
<210> 16
   <211> 23
   <212> DNA
   <213> Artificial
<220>
   <223> probe
<400> 16
   aggtccggta gtaatttagg tgc 23
<210> 17
   <211> 23
   <212> DNA
   <213> Artificial
<220>
   <223> probe
<400> 17
   tgcactctga tatatacagg cca 23
<210> 18
   <211> 23
   <212> DNA
   <213> Artificial
<220>
   <223> probe
<400> 18
   gcagccctta tagataacgg gac 23
<210> 19
   <211> 23
   <212> DNA
   <213> Artificial
<220>
   <223> probe
<400> 19
   gaagccatga tactgttcag ggt 23
<210> 20
   <211> 23
   <212> DNA
   <213> Artificial
<220>
   <223> probe
<400> 20
   tattctacca acgacatcac tgc 23
<210> 21
   <211> 23
   <212> DNA
   <213> Artificial
<220>
   <223> probe
<400> 21
   ccatcagtta ttcggaggga ctc 23
<210> 22
   <211> 23
   <212> DNA
   <213> Artificial
<220>
   <223> probe
<400> 22
   ccatatccga ttattagcga cgg 23
<210> 23
   <211> 23
   <212> DNA
   <213> Artificial
<220>
   <223> probe
<400> 23
   catctccaag aattgaccca cca 23
<210> 24
   <211> 23
   <212> DNA
   <213> Artificial
<220>
   <223> probe
<400> 24
   ccgtcgtgtt attaaagacc cct 23
<210> 25
   <211> 23
   <212> DNA
   <213> Artificial
<220>
   <223> probe
<400> 25
   gaaggatcgc ttttatctgg cat 23
<210> 26
   <211> 23
   <212> DNA
   <213> Artificial
<220>
   <223> probe
<400> 26
   catttgtcag gtacagtcca ctt 23
<210> 27
   <211> 23
   <212> DNA
   <213> Artificial
<220>
   <223> probe
<400> 27
   gcccacactc ttacttatcg act 23
<210> 28
   <211> 23
   <212> DNA
   <213> Artificial
<220>
   <223> probe
<400> 28
   cgctgttact gtaagcgtac tag 23
<210> 29
   <211> 23
   <212> DNA
   <213> Artificial
<220>
   <223> probe
<400> 29
   cgcgattcct attgattgat ccc 23
<210> 30
   <211> 23
   <212> DNA
   <213> Artificial
<220>
   <223> probe
<400> 30
   ccgtctgggt taaagattgc tag 23
<210> 31
   <211> 23
   <212> DNA
   <213> Artificial
<220>
   <223> probe
<400> 31
   agtcagtcca aatctcagga tgg 23
<210> 32
   <211> 23
   <212> DNA
   <213> Artificial
<220>
   <223> probe
<400> 32
   cgcctaaatg aaactcactc tgc 23
<210> 33
   <211> 23
   <212> DNA
   <213> Artificial
<220>
   <223> probe
<400> 33
   ggggtcaaac caacaattga tct 23
<210> 34
   <211> 23
   <212> DNA
   <213> Artificial
<220>
   <223> probe
<400> 34
   gcccattgat agaattacga ggc 23
<210> 35
   <211> 23
   <212> DNA
   <213> Artificial
<220>
   <223> probe
<400> 35
   atgccgttgt caagagttat ggt 23
<210> 36
   <211> 23
   <212> DNA
   <213> Artificial
<220>
   <223> probe
<400> 36
   tgccggctat cgtaagtata tgc 23
<210> 37
   <211> 23
   <212> DNA
   <213> Artificial
<220>
   <223> probe
<400> 37
   gcacctcata ccttcataga gca 23
<210> 38
   <211> 23
   <212> DNA
   <213> Artificial
<220>
   <223> probe
<400> 38
   cgcgacattt agtccaggag atg 23
<210> 39
   <211> 23
   <212> DNA
   <213> Artificial
<220>
   <223> probe
<400> 39
   ctagtccatt gtaacgaagg cca 23
<210> 40
   <211> 23
   <212> DNA
   <213> Artificial
<220>
   <223> probe
<400> 40
   agacaattag aatcagtgcc cct 23
<210> 41
   <211> 23
   <212> DNA
   <213> Artificial
<220>
   <223> probe
<400> 41
   gcattgaggt attgttgctc cca 23
<210> 42
   <211> 23
   <212> DNA
   <213> Artificial
<220>
   <223> probe
<400> 42
   cgagagtctg taatagccga tgc 23
<210> 43
   <211> 23
   <212> DNA
   <213> Artificial
<220>
   <223> probe
<400> 43
   tgccgtgata cttaactacg cta 23
<210> 44
   <211> 23
   <212> DNA
   <213> Artificial
<220>
   <223> probe
<400> 44
   gagtccgcaa aaatatagga ggc 23
<210> 45
   <211> 23
   <212> DNA
   <213> Artificial
<220>
   <223> probe
<400> 45
   gcctcacata actggagaaa cct 23
<210> 46
   <211> 23
   <212> DNA
   <213> Artificial
<220>
   <223> probe
<400> 46
   cgccaatgac aataagttga ggc 23
<210> 47
   <211> 23
   <212> DNA
   <213> Artificial
<220>
   <223> probe
<400> 47
   cgcgatataa cattaaccga ggc 23
<210> 48
   <211> 23
   <212> DNA
   <213> Artificial
<220>
   <223> probe
<400> 48
   cacgcttagt tcctacctta ggc 23
<210> 49
   <211> 23
   <212> DNA
   <213> Artificial
<220>
   <223> probe
<400> 49
   cgcgtcgaat tacttaatca cca 23
<210> 50
   <211> 23
   <212> DNA
   <213> Artificial
<220>
   <223> probe
<400> 50
   gggataggta ttatgctcca gcc 23
<210> 51
   <211> 23
   <212> DNA
   <213> Artificial
<220>
   <223> probe
<400> 51
   cgccattata caacggttca tgc 23
<210> 52
   <211> 23
   <212> DNA
   <213> Artificial
<220>
   <223> probe
<400> 52
   gcctatatga accaagccac tgc 23
<210> 53
   <211> 23
   <212> DNA
   <213> Artificial
<220>
   <223> probe
<400> 53
   cgccgtcagt acttgtatag atg 23
<210> 54
   <211> 23
   <212> DNA
   <213> Artificial
<220>
   <223> probe
<400> 54
   gtcggtatcg aaaaggtact gca 23
<210> 55
   <211> 23
   <212> DNA
   <213> Artificial
<220>
   <223> probe
<400> 55
   aggcagttca acctatatct gcg 23
<210> 56
   <211> 23
   <212> DNA
   <213> Artificial
<220>
   <223> probe
<400> 56
   ggtcgtaaca ttgagaggag acg 23
<210> 57
   <211> 23
   <212> DNA
   <213> Artificial
<220>
   <223> probe
<400> 57
   ggcgatttat tgctaactgg cta 23
<210> 58
   <211> 23
   <212> DNA
   <213> Artificial
<220>
   <223> probe
<400> 58
   gcactaccgc taactatacg cta 23
<210> 59
   <211> 23
   <212> DNA
   <213> Artificial
<220>
   <223> probe
<400> 59
   ggctcgtagt actccttaca tgc 23
<210> 60
   <211> 23
   <212> DNA
   <213> Artificial
<220>
   <223> probe
<400> 60
   ggctctacaa acttgtgtcc atg 23
<210> 61
   <211> 23
   <212> DNA
   <213> Artificial
<220>
   <223> probe
<400> 61
   ggtggagtga atctcactag act 23
<210> 62
   <211> 23
   <212> DNA
   <213> Artificial
<220>
   <223> probe
<400> 62
   ctagcacaat taatcaatcc gcc 23
<210> 63
   <211> 23
   <212> DNA
   <213> Artificial
<220>
   <223> probe
<400> 63
   gcagctgaat tgctatgatc acc 23
<210> 64
   <211> 23
   <212> DNA
   <213> Artificial
<220>
   <223> probe
<400> 64
   gcctatagtg tcgattgtcc tcg 23
<210> 65
   <211> 23
   <212> DNA
   <213> Artificial
<220>
   <223> probe
<400> 65
   cgatcacgga ttaatgtcac ccc 23
<210> 66
   <211> 23
   <212> DNA
   <213> Artificial
<220>
   <223> probe
<400> 66
   aagagattta acttgagctc gcc 23
<210> 67
   <211> 23
   <212> DNA
   <213> Artificial
<220>
   <223> probe
<400> 67
   tttgttgttc gatatcaggc gtg 23
<210> 68
   <211> 23
   <212> DNA
   <213> Artificial
<220>
   <223> probe
<400> 68
   gcccgggaat agattataac gca 23
<210> 69
   <211> 23
   <212> DNA
   <213> Artificial
<220>
   <223> probe
<400> 69
   gcatttttag taatccgagc gcc 23
<210> 70
   <211> 23
   <212> DNA
   <213> Artificial
<220>
   <223> probe
<400> 70
   catggataag ttttcaagct gcg 23
<210> 71
   <211> 23
   <212> DNA
   <213> Artificial
<220>
   <223> probe
<400> 71
   gagacaggta aaccctcaga gca 23
<210> 72
   <211> 23
   <212> DNA
   <213> Artificial
<220>
   <223> probe
<400> 72
   tagcacccgt taaaacggaa atg 23
<210> 73
   <211> 23
   <212> DNA
   <213> Artificial
<220>
   <223> probe
<400> 73
   tatgtttagt tgttgaaccg gcg 23
<210> 74
   <211> 23
   <212> DNA
   <213> Artificial
<220>
   <223> probe
<400> 74
   cgatcagctc tatttccctc cca 23
<210> 75
   <211> 23
   <212> DNA
   <213> Artificial
<220>
   <223> probe
<400> 75
   agtcagttaa tcagacgtga gca 23
<210> 76
   <211> 23
   <212> DNA
   <213> Artificial
<220>
   <223> probe
<400> 76
   tggcaataca ataacgtatc gcg 23
<210> 77
   <211> 23
   <212> DNA
   <213> Artificial
<220>
   <223> probe
<400> 77
   cgcagtttgc aagaacgaac aaa 23
<210> 78
   <211> 23
   <212> DNA
   <213> Artificial
<220>
   <223> probe
<400> 78
   cgcgataatt gatacctacg ggc 23
<210> 79
   <211> 23
   <212> DNA
   <213> Artificial
<220>
   <223> probe
<400> 79
   ggggtgtgag agctttttag acg 23
<210> 80
   <211> 23
   <212> DNA
   <213> Artificial
<220>
   <223> probe
<400> 80
   gggatccgta acaagtgtgt tag 23
<210> 81
   <211> 23
   <212> DNA
   <213> Artificial
<220>
   <223> probe
<400> 81
   accactatga ttgaggaaac gcg 23
<210> 82
   <211> 23
   <212> DNA
   <213> Artificial
<220>
   <223> probe
<400> 82
   cgtctttagt atcaaccctc cgc 23
<210> 83
   <211> 23
   <212> DNA
   <213> Artificial
<220>
   <223> probe
<400> 83
   gcatacgaac ttctatatcg gcg 23
<210> 84
   <211> 23
   <212> DNA
   <213> Artificial
<220>
   <223> probe
<400> 84
   ccgtgtgtat gagtatgaca gca 23
<210> 85
   <211> 23
   <212> DNA
   <213> Artificial
<220>
   <223> probe
<400> 85
   tgctgtcttc gtgttttacc tag 23
<210> 86
   <211> 23
   <212> DNA
   <213> Artificial
<220>
   <223> probe
<400> 86
   cgatcatgta aagctaactc gcg 23
<210> 87
   <211> 23
   <212> DNA
   <213> Artificial
<220>
   <223> probe
<400> 87
   tgccgtcatt taaacgtaag ggt 23
<210> 88
   <211> 23
   <212> DNA
   <213> Artificial
<220>
   <223> probe
<400> 88
   tggcaattac agttgttaac gca 23
<210> 89
   <211> 23
   <212> DNA
   <213> Artificial
<220>
   <223> probe
<400> 89
   gagtcgaaga cctcctccta ctc 23
<210> 90
   <211> 23
   <212> DNA
   <213> Artificial
<220>
   <223> probe
<400> 90
   atgccaatat gtactcgtga ctc 23
<210> 91
   <211> 23
   <212> DNA
   <213> Artificial
<220>
   <223> probe
<400> 91
   gcatatagtg acggtaaggc gaa 23
<210> 92
   <211> 23
   <212> DNA
   <213> Artificial
<220>
   <223> probe
<400> 92
   gcctcacttg taataagcgg gac 23
<210> 93
   <211> 23
   <212> DNA
   <213> Artificial
<220>
   <223> probe
<400> 93
   gtcccaaaag cttcttacgg acg 23
<210> 94
   <211> 23
   <212> DNA
   <213> Artificial
<220>
   <223> probe
<400> 94
   ctaggtacaa caccaactgt ctc 23
<210> 95
   <211> 23
   <212> DNA
   <213> Artificial
<220>
   <223> probe
<400> 95
   tgccggttat acctttaagg acg 23
<210> 96
   <211> 23
   <212> DNA
   <213> Artificial
<220>
   <223> probe
<400> 96
   ggctggttaa atgtaaatcc gcg 23
<210> 97
   <211> 23
   <212> DNA
   <213> Artificial
<220>
   <223> probe
<400> 97
   cgcggtacta ttagaaaggg cta 23
<210> 98
   <211> 23
   <212> DNA
   <213> Artificial
<220>
   <223> probe
<400> 98
   agtcgcttaa ttactccgga tgg 23
<210> 99
   <211> 23
   <212> DNA
   <213> Artificial
<220>
   <223> probe
<400> 99
   cgctgttggt attaccttcc tcg 23
<210> 100
   <211> 23
   <212> DNA
   <213> Artificial
<220>
   <223> probe
<400> 100
   tgcagtgtaa gcaactattg tct 23
<210> 101
   <211> 45
   <212> DNA
   <213> Artificial
<220>
   <223> Primer
<400> 101
   accaaagaat atggctgaat ttagtagtgt tttaaataat tttaa 45
<210> 102
   <211> 45
   <212> DNA
   <213> Artificial
<220>
   <223> Primer
<400> 102
   acctgctaat gagatgatcc cttattttga aaacaactat tccta 45
<210> 103
   <211> 44
   <212> DNA
   <213> Artificial
<220>
   <223> Primer
<400> 103
   agcctagatt cattattcaa agatatgaaa ttttaaaatg cata 44
<210> 104
   <211> 37
   <212> DNA
   <213> Artificial
<220>
   <223> Pr
<400> 104
   ctagagatac tacagagcct gtccgtccaa ggtcata 37
<210> 105
   <211> 43
   <212> DNA
   <213> Artificial
<220>
   <223> Probe
<400> 105
   cattttaata ttgggtagaa aaatcaagaa tgcattgctc ata 43
<210> 106
   <211> 40
   <212> DNA
   <213> Artificial
<220>
   <223> Primer
<400> 106
   tgttaatctt attaaggttg ctcagctcta agattctata 40
<210> 107
   <211> 42
   <212> DNA
   <213> Artificial
<220>
   <223> Primer
<400> 107
   cattcatata tttatgcatt cctccattca aaagatctta tt 42
<210> 108
   <211> 41
   <212> DNA
   <213> Artificial
<220>
   <223> Primer
<400> 108
   gtttaagtaa aggatacaga ggtttataaa agtttgaaaa c 41
<210> 109
   <211> 35
   <212> DNA
   <213> Artificial
<220>
   <223> Primer
<400> 109
   tctgtggcaa taagatccct atgactgaag atgcc 35
<210> 110
   <211> 40
   <212> DNA
   <213> Artificial
<220>
   <223> Primer
<400> 110
   ggataaacct taatatagaa ggaattagag ctgccacagc 40
<210> 111
   <211> 36
   <212> DNA
   <213> Artificial
<220>
   <223> Primer
<400> 111
   gagaaccttt gaggcatccc tgctgttctc gagata 36
<210> 112
   <211> 35
   <212> DNA
   <213> Artificial
<220>
   <223> Primer
<400> 112
   aaacatgtga ggcgttcaca gaaagggttc aggaa 35
<210> 113
   <211> 68
   <212> DNA
   <213> Artificial
<220>
   <223> Primer
<400> 113
   aggtttttct agagtggaca cggaccaaag aatatggctg aatttagtag tgttttaaat 60
   aattttaa 68
<210> 114
   <211> 68
   <212> DNA
   <213> Artificial
<220>
   <223> Primer
<400> 114
   tgttctctga ccaatgaatc tgcacctgct aatgagatga tcccttattt tgaaaacaac 60
   tattccta 68
<210> 115
   <211> 67
   <212> DNA
   <213> Artificial
<220>
   <223> Primer
<400> 115
   aggtttttct agagtggaca cggagcctag attcattatt caaagatatg aaattttaaa 60
   atgcata 67
<210> 116
   <211> 60
   <212> DNA
   <213> Artificial
<220>
   <223> Primer
<400> 116
   tggaactggg aacgctttag atgctagaga tactacagag cctgtccgtc caaggtcata 60
<210> 117
   <211> 66
   <212> DNA
   <213> Artificial
<220>
   <223> Primer
<400> 117
   aggtttttct agagtggaca cggcatttta atattgggta gaaaaatcaa gaatgcattg 60
   ctcata 66
<210> 118
   <211> 63
   <212> DNA
   <213> Artificial
<220>
   <223> Primer
<400> 118
   ttcgcttcgt tgtaatttcg gactgttaat cttattaagg ttgctcagct ctaagattct 60
   ata 63
<210> 119
   <211> 65
   <212> DNA
   <213> Artificial
<220>
   <223> Primer
<400> 119
   aggtttttct agagtggaca cggcattcat atatttatgc attcctccat tcaaaagatc 60
   ttatt 65
<210> 120
   <211> 64
   <212> DNA
   <213> Artificial
<220>
   <223> Primer
<400> 120
   aggcatccta agaaatcgct actgtttaag taaaggatac agaggtttat aaaagtttga 60
   aaac 64
<210> 121
   <211> 58
   <212> DNA
   <213> Artificial
<220>
   <223> Primer
<400> 121
   aggtttttct agagtggaca cggtctgtgg caataagatc cctatgactg aagatgcc 58
<210> 122
   <211> 63
   <212> DNA
   <213> Artificial
<220>
   <223> Primer
<400> 122
   tagcccagtg atttatgaca tgcggataaa ccttaatata gaaggaatta gagctgccac 60
   agc 63
<210> 123
   <211> 59
   <212> DNA
   <213> Artificial
<220>
   <223> Primer
<400> 123
   aggtttttct agagtggaca cgggagaacc tttgaggcat ccctgctgtt ctcgagata 59
<210> 124
   <211> 58
   <212> DNA
   <213> Artificial
<220>
   <223> Primer
<400> 124
   cgctctggtt actattggac gttaaacatg tgaggcgttc acagaaaggg ttcaggaa 58
<210> 125
   <211> 69
   <212> DNA
   <213> Artificial
<220>
   <223> Primer
<220>
   <221> misc_feature
   <222> (24)..(24)
   <223> conjugation part
<400> 125
   aggtttttct agagtggaca cggnaccaaa gaatatggct gaatttagta gtgttttaaa 60
   taattttaa 69
<210> 126
   <211> 69
   <212> DNA
   <213> Artificial
<220>
   <223> Primer
<220>
   <221> misc_feature
   <222> (24)..(24)
   <223> conjugation part
<400> 126
   gcagattcat tggtcagaga acanacctgc taatgagatg atcccttatt ttgaaaacaa 60
   ctattccta 69
<210> 127
   <211> 68
   <212> DNA
   <213> Artificial
<220>
   <223> Primer
<220>
   <221> misc_feature
   <222> (24)..(24)
   <223> conjugation part
<400> 127
   aggtttttct agagtggaca cggnagccta gattcattat tcaaagatat gaaattttaa 60
   aatgcata 68
<210> 128
   <211> 61
   <212> DNA
   <213> Artificial
<220>
   <223> Primer
<220>
   <221> misc_feature
   <222> (24)..(24)
   <223> n is a, c, g, or t
<400> 128
   catctaaagc gttcccagtt ccanctagag atactacaga gcctgtccgt ccaaggtcat 60
   a 61
<210> 129
   <211> 67
   <212> DNA
   <213> Artificial
<220>
   <223> Primer
<220>
   <221> misc_feature
   <222> (24)..(24)
   <223> conjugation part
<400> 129
   aggtttttct agagtggaca cggncatttt aatattgggt agaaaaatca agaatgcatt 60
   gctcata 67
<210> 130
   <211> 64
   <212> DNA
   <213> Artificial
<220>
   <223> Primer
<220>
   <221> misc_feature
   <222> (24)..(24)
   <223> conjugation part
<400> 130
   gtccgaaatt acaacgaagc gaantgttaa tcttattaag gttgctcagc tctaagattc 60
   tata 64
<210> 131
   <211> 66
   <212> DNA
   <213> Artificial
<220>
   <223> Primer
<220>
   <221> misc_feature
   <222> (24)..(24)
   <223> conjugation part
<400> 131
   aggtttttct agagtggaca cggncattca tatatttatg cattcctcca ttcaaaagat 60
   cttatt 66
<210> 132
   <211> 65
   <212> DNA
   <213> Artificial
<220>
   <223> Primer
<220>
   <221> misc_feature
   <222> (24)..(24)
   <223> conjufgation part
<400> 132
   agtagcgatt tcttaggatg cctngtttaa gtaaaggata cagaggttta taaaagtttg 60
   aaaac 65
<210> 133
   <211> 59
   <212> DNA
   <213> Artificial
<220>
   <223> Primer
<220>
   <221> misc_feature
   <222> (24)..(24)
   <223> conjugation part
<400> 133
   aggtttttct agagtggaca cggntctgtg gcaataagat ccctatgact gaagatgcc 59
<210> 134
   <211> 64
   <212> DNA
   <213> Artificial
<220>
   <223> Primer
<220>
   <221> misc_feature
   <222> (24)..(24)
   <223> conjugation part
<400> 134
   gcatgtcata aatcactggg ctanggataa accttaatat agaaggaatt agagctgcca 60
   cagc 64
<210> 135
   <211> 60
   <212> DNA
   <213> Artificial
<220>
   <223> Primer
<220>
   <221> misc_feature
   <222> (24)..(24)
   <223> conjugation part
<400> 135
   aggtttttct agagtggaca cggngagaac ctttgaggca tccctgctgt tctcgagata 60
<210> 136
   <211> 59
   <212> DNA
   <213> Artificial
<220>
   <223> Primer
<220>
   <221> misc_feature
   <222> (24).. (24)
   <223> conjugation part
<400> 136
   aacgtccaat agtaaccaga gcgnaaacat gtgaggcgtt cacagaaagg gttcaggaa 59

## Description

### TECHNICAL FIELD

The present description relates to a technique for detecting a target nucleic acid.

### DESCRIPTION OF RELATED ART

To date, methods have been proposed for exhaustively detecting, identifying and assaying nucleic acid sequences as a method of analyzing the genes of individual organisms or investigating the presences of viruses, bacteria and the like in biological samples. In such analysis and the like, normally a probe or the like associated with a target nucleic acid sequence is prepared in advance, and hybridization of the probe or the like with a DNA fragment amplified by a nucleic acid amplification method from a biological sample is used to detect the target nucleic acid by means of a label bound to the probe and the DNA fragment.

For example, a method is described in which primers are designed so as to include base sequences to which a specific substance can bind at both ends of a DNA fragment amplified by a nucleic acid amplification method, and this specific substance is used to detect the DNA fragment (patent document 1).

Specialized arrays have also been developed for detecting single nucleotide polymorphisms (SNPs) (see for example patent documents 2 and 3, and non-patent document 1). In these methods, a detection probe having an artificial base sequence is prepared in advance, and the sample preparation step is designed to allow amplification of those DNA fragments having base sequences that bind to this artificial nucleotide sequence.

Moreover, a method has also been disclosed for using an array of DNA extracted from bacteria to visually detect the drug sensitivity of tuberculosis bacteria (non-patent document 2).

PCR and other nucleic acid amplification methods normally amplify target double-stranded parts. Therefore, the nucleic acid amplification product is also double-stranded. To determine whether the target amplification product has been produced, the double strand is heat denatured into single strands, and some of the single strands are hybridized with an oligonucleotide or other probe to detect the product.

However, hybridization efficiency may decline when nucleic acids are heat denatured into single strands and hybridized with a probe.

### CITATION LIST

### Patent Literatures

Patent Literature 1: WO 2009/034842
Patent Literature 2: Japanese Patent Application Publication No. 2006-211982
Patent Literature 3: Japanese Patent Application Publication No. 2006-101844

### Non Patent Literatures

Non Patent Literature 1: Analytical Biochemistry 364 (2007), 78-85
Non Patent Literature 2: Proceedings of the 14th Annual Meeting of the Association for the Rapid Method and Automation in Microbiology (ARMAM): 45-50

### SUMMARY OF INVENTION

In the method described in patent document 1 above, a specific substance binding site (specific base sequence bindable with a specific substance) on an amplified DNA fragment is recognized by a specific substance, which binds to the specific base sequence. However, in the amplified DNA fragment even the specific base sequence is in the form of a double strand with a complementary strand, rather than being a single strand that can interact easily with the specific substance. Therefore, recognition of the specific base sequence by the specific substance is not very efficient, and it is necessary to concentrate the double-stranded fragment bound to the specific substance.

In the methods described in patent documents 2 and 3 and non-patent document 1, meanwhile, reactivity is improved by performing amplification with a relatively higher concentration of one primer (called asymmetric PCR) or the like in the labeling step, so as to deliberately and selectively amplify the DNA strand that reacts with the probe on the array. However, in such asymmetric PCR the amplification efficiency itself tends to be lower.

Moreover, although the tuberculosis bacterium can be detected visually with the method described in non-patent document 2, the DNA extracted from the bacterium must be heat denatured before being applied to the array.

In ordinary probe hybridization, the DNA amplified fragments used as the sample are of course double-stranded fragments, and are normally denatured with heat or alkali to obtain single strands in order to achieve efficient hybridization with the probe. However, there is a risk that denatured amplified fragments will gradually return to their double-stranded form, reducing hybridization efficiency. To control this, it may be necessary to optimize the hybridization time, temperature and other conditions.

Another possible method is one in which partially double-stranded DNA comprising single strands (hereunder called tag strands) at the nucleic acid 5' ends of the DNA double strand is obtained as the amplification product of a nucleic acid amplification reaction. The partially double-stranded DNA obtained as the amplification product in this method can hybridize with other DNA strands and the like at both ends. Thus, the tag strand at one end of the partially double-stranded DNA can be used to hybridize with a probe, while the other tag strand can be used to hybridize with a labeling probe or the like. This method has the advantage of eliminating the heat-denaturing step, but the inventors have found the following problems. Mainly, the problem is that when using such partially double-stranded DNA, it becomes more difficult to design the base sequences of the tag strands. That is, since partially double-stranded DNA has two tag strands, in order to detect a single target nucleic acid using this partial double strand, one tag strand must be a sequence that hybridizes specifically with the target nucleic acid, while the other tag strand must be a sequence that does not hybridize with the target nucleic acid or inhibit (interfere with) hybridization between the first tag strand and the nucleic acid. When the aim is to detect multiple target nucleic acids simultaneously, moreover, both tag strands must be designed not to interfere with other target nucleic acids not targeted by this particular partially double-stranded DNA. In addition, the base sequences of both tag strands must be designed so that they do not hybridize with one another.

Moreover, in this method it is also more difficult to design the amplification conditions. This is because the excess tag strands that do not participate in amplification are bound to the forward and reverse primers, respectively.

As a result, in this method there is a risk of decreased efficiency in the amplification step and nucleic acid chromatography hybridization step, and of reduced detection accuracy due to mis-hybridization. In particular, because movement and evaporation of the developing medium are factors in nucleic acid chromatography, there has been a problem of low hybridization efficiency in comparison with immersion hybridization, in which the array is immersed in the hybridization solution.

In light of these circumstances, this Description provide a target nucleic acid detection method whereby problems with sample DNA fragments in conventional probe hybridization can be resolved to achieve efficient probe hybridization, and a gene amplification agent and hybridization composition using this method.

This Description also provides a target nucleic acid detection method and kit that are more suitable for practical use, or in other words that allow a target nucleic acid to be detected more accurately with an easy operation.

The inventors investigated modifications to nucleic acid amplification methods from the perspective of improving the sensitivity and efficiency of hybridization with the probe when applied to probe hybridization. As a result of much research, it was found that detection sensitivity could be improved with high hybridization efficiency by introducing a site capable of inhibiting or arresting the progress of a polymerase reaction into a part of a primer used in nucleic acid amplification. It was also found that hybridization could be accomplished with high sensitivity in a short amount of time without the need for heat denaturing. This Description provides the following means based on these findings.

A method for detecting a target nucleic acid, the method comprising:
a hybridization step in which one or two or more partially double-stranded nucleic acids associated with one or two or more target nucleic acids are brought into contact with one or two or more probes that are on a solid phase body carrier and are associated with the one or two or
more target nucleic acids, under conditions that allow hybridization, wherein the hybridization step is performed by nucleic acid chromatography; and
a detection step in which the hybridization product produced in the hybridization step is detected, wherein
each of the one or two or more partially double-stranded nucleic acids has a single-stranded tag part at only the 5' end of a first strand, which consists solely of natural nucleic acids and which is a tag sequence capable of hybridizing specifically with one of the probes, and has a label or
label binding substance at the 5' end of a second strand,
before the hybridization step an amplification step in which the partially double-stranded nucleic acid is obtained as a product of an amplification reaction performed on a target nucleic acid using a first primer having the tag sequence and a first recognition sequence that recognizes a first base sequence in the target nucleic acid and having a linking site capable of inhibiting or
arresting a DNA polymerase reaction disposed between the tag sequence and the first recognition sequence, and a second primer having a second recognition sequence that recognizes a second base sequence in the target nucleic acid and the label or label binding substance;wherein the linking site comprises an optionally substituted alkylene chain with an element number of 2 to 40, adjoining a nucleotide in the primer via a phosphate diester bond; and wherein the linking site is represented by the following formula:

   5'-O-CₘH₂ₘ-O-3' Formula (1)
(where 5' represents the oxygen atom of a phosphate diester bond at the 5' end, 3' represents the phosphorus atom of a phosphate diester bond at the 3' end, and m is an integer from 3-12).

The method optionally further comprises the use of the specific sequence of the detection probe is selected from the base sequences represented by SEQ ID NOS:1 to 100 represented by the SEQ ID NOS in the following **table** and complementary sequences thereof.

**[Table 1]**

| Name | Seq(5→3') | SEQ.ID. |
|---|---|---|
| D1-001 | TGTTCTCTGACCAATGAATCTGC | 1 |
| D1-002 | TGGAACTGGGAACGCTTTAGATG | 2 |
| D1-003 | TTCGCTTCGTTGTAATTTCGGAC | 3 |
| D1-005 | TAGCCCAGTGATTTATGACATGC | 5 |
| D1-006 | CGCTCTGGTTACTATTGGACGTT | 6 |
| D1-010 | GAGTAGCAGGCAAATACCCTAGA | 10 |
| D1-012 | AGTCATACAGTGAGGACCAAATG | 12 |
| D1-014 | TGCTCACTTACATTACGTCCATG | 14 |
| D1-016 | AGGTCCGGTAGTAATTTAGGTGC | 16 |
| D1-020 | TATTCTACCAACGACATCACTGC | 20 |
| D1-023 | CATCTCCAAGAATTGACCCACCA | 23 |
| D1-025 | GAAGGATCGCTTTTATCTGGCAT | 25 |
| D1-026 | CATTTGTCAGGTACAGTCCACTT | 26 |
| D1-027 | GCCCACACTCTTACTTATCGACT | 27 |
| D1-030 | CCGTCTGGGTTAAAGATTGCTAG | 30 |
| D1-035 | ATGCCGTTGTCAAGAGTTATGGT | 35 |
| D1-038 | CGCGACATTTAGTCCAGGAGATG | 38 |
| D1-040 | AGACAATTAGAATCAGTGCCCCT | 40 |
| D1-041 | GCATTGAGGTATTGTTGCTCCCA | 41 |
| D1-044 | GAGTCCGCAAAAATATAGGAGGC | 44 |
| D1-045 | GCCTCACATAACTGGAGAAACCT | 45 |
| D1-050 | GGGATAGGTATTATGCTCCAGCC | 50 |
| D1-052 | GCCTATATGAACCAAGCCACTGC | 52 |
| D1-062 | CTAGCACAATTAATCAATCCGCC | 62 |
| D1-064 | GCCTATAGTGTCGATTGTCCTCG | 64 |
| D1-065 | CGATCACGGATTAATGTCACCCC | 65 |
| D1-077 | CGCAGTTTGCAAGAACGAACAAA | 77 |
| D1-084 | CCGTGTGTATGAGTATGACAGCA | 84 |
| D1-089 | GAGTCGAAGACCTCCTCCTACTC | 89 |
| D1-090 | ATGCCAATATGTACTCGTGACTC | 90 |
| D1-095 | TGCCGGTTATACCTTTAAGGACG | 95 |
| D1-097 | CGCGGTACTATTAGAAAGGGCTA | 97 |
| D1-100 | TGCAGTGTAAGCAACTATTGTCT | 100 |

The present disclosure further discloses that the hybridization step is optionally a step of supplying a liquid containing the amplified fragment as a mobile phase to a solid phase body containing a plurality of the detection probe, and expanding the mobile phase in the solid phase body.

A nucleic acid amplification agent for use in a nucleic acid amplification method is described herein, comprising, in order from the 5' end, a first arbitrary base sequence and a first recognition sequence that recognizes a first base sequence in a nucleic acid to be amplified, and also comprising an oligonucleotide derivative having a linking site capable of inhibiting or arresting a DNA polymerase reaction, disposed between the first arbitrary base sequence and the first recognition sequence.

The nucleic acid amplification agent may be one wherein the first base sequence has a label bound thereto.
A nucleic acid amplification kit containing two or more of the nucleic acid amplification agent is described herein.

A composition for probe hybridization is described herein, comprising a double-stranded DNA fragment having a single-stranded part on the 5' side of at least one strand and a double-stranded part formed by base pairing, wherein at least one of the DNA strands has a linking site capable of inhibiting or arresting a DNA polymerase reaction disposed between the single-stranded part and the double-stranded binding part, and the single-stranded part has a recognition sequence that recognizes a base sequence in the probe.
The composition for probe hybridization optionally further comprises a single-stranded part on the 5' side of the other strand, and having a label linked to this single-stranded part.

A double-stranded DNA fragment having a single-stranded part on the 5' side of at least one strand and a double-stranded part formed by base pairing is described herein, wherein at least one of the DNA strands has a linking site capable of inhibiting or arresting a DNA polymerase reaction disposed between the single-stranded part and the double-stranded binding part.

A method for amplifying a target nucleic acid in a sample is described herein,
the method comprising a step of performing nucleic acid amplification on the sample using at least a first primer having a first arbitrary base sequence and a first recognition sequence that recognizes a first base sequence in the target nucleic acid, and having a linking site capable of inhibiting or arresting a DNA polymerase reaction disposed between the first arbitrary base sequence and the first recognition sequence.

The inventors investigated modifications to nucleic acid amplification methods from the perspective of more practical detection of the target nucleic acid. As a result of profound research, it was found that specifying a structure of nucleic acid to be provided for hybridization via nucleic acid chromatography enables simplification of primer design and processes thereof in amplification step that takes place prior to the specification, and enables an increase in the efficiency of hybridization rate in hybridization step while suppressing mis-hybridization. This Description provides the following means based on these findings.

A method for detecting a target nucleic acid by nucleic acid chromatography is described herein,
the method comprising:
a hybridization step in which one or two or more partially double-stranded nucleic acids associated with one or two or more target nucleic acids are brought into contact with one or two or more probes that are on a solid phase body carrier and are associated with the one or two or more target nucleic acids, under conditions that allow hybridization by nucleic acid chromatography; and
a detection step in which the hybridization product produced in the hybridization step is detected, wherein
each of the one or two or more partially double-stranded nucleic acids has a single-stranded tag part at the 5' end of a first chain, which is a tag sequence capable of hybridizing specifically with one of the probes, and
at least part of the double-stranded nucleic acid has a label or label binding substance. Described herein is a method wherein the partially double-stranded nucleic acid is provided with the label or label binding substance at the 5' end of a second strand.

Described herein is a method comprising before the hybridization step an amplification step in which the partially double-stranded nucleic acid is obtained as a product of an amplification reaction performed on a target nucleic acid using a first primer having the tag sequence and a first recognition sequence that recognizes a first base sequence in the target nucleic acid and having a linking part capable of inhibiting or arresting a DNA polymerase reaction disposed between the tag sequence and the first recognition sequence, and a second primer having a second recognition sequence that recognizes a second base sequence in the target nucleic acid and. the label or label binding substance

Described herein is a method comprising before the hybridization step an amplification step in which the partially double-stranded nucleic acid is obtained as the product of an amplification reaction performed on a target nucleic acid using a first primer having the tag sequence and a first recognition sequence that recognizes a first base sequence in the target nucleic acid and having a linking site capable of inhibiting or arresting a DNA polymerase reaction disposed between the tag sequence and the first recognition sequence, a second primer I having a labeling sequence and a second recognition sequence that recognizes a second base sequence in the target nucleic acid, and a second primer II having the label or label binding substance and the labeling sequence.

Described herein is a method comprising before the hybridization step an amplification step in which an amplification reaction is performed on a target nucleic acid in the presence of a labeling probe having the label or label binding sequence and a sequence that hybridizes specifically with the labeling sequence in use of a first primer having the tag sequence and a first recognition sequence that recognizes a first base sequence in the target nucleic acid and having a linking site capable of inhibiting or arresting a DNA polymerase reaction disposed between the tag sequence and the first recognition sequence, and a second primer I having a labeling sequence and a second recognition sequence that recognizes a second base sequence in the target nucleic acid, whereby a complex of the partially double-stranded nucleic acid and the labeling probe is formed.

Described herein is a method wherein the partially double-stranded nucleic acid is provided with the label or label binding substance in the double-stranded part.

Described herein is a method comprising before the hybridization step an amplification step in which the partially double-stranded nucleic acid is obtained as the amplification product of an amplification reaction performed on a target nucleic acid in use of a first primer having the tag sequence and a first recognition sequence that recognizes a first base sequence in the target nucleic acid and also having a linking site capable of inhibiting or arresting a DNA polymerase reaction disposed between the tag sequence and the first recognition sequence, and a second primer provided with a second recognition sequence that recognizes a second base sequences in the target nucleic acid, and in use of a nucleoside triphosphate containing a nucleoside derivative triphosphate having the label or label binding substance.

Described herein is a method wherein the hybridization step is performed by bringing an developing medium comprising an amplification reaction solution containing the amplification product of the amplification step into contact with a part of the solid phase body carrier.

Described herein is a method wherein the hybridization step is performed by preparing the developing medium comprising an amplification reaction solution containing the amplification product of the amplification step together with a label for binding to the label binding substance, and brining this developing medium into contact with at least a part of the solid phase body carrier.

Described herein is a method wherein the amplification step is performed in a cavity, the developing medium is prepared by supplying at least the label to the cavity holding the amplification reaction solution, and the developing medium is brought into contact with part of the solid phase body carrier in the cavity.

Described herein is a method wherein
the label binding substance is one or two or more selected from the group consisting of the antibodies in antigen-antibody reactions and biotin, digoxigenin, and FITC and other haptens, and
the label is provided with a site capable of binding with the label binding substance, and is a label that uses one or two or more selected from fluorescence, radioactivity, enzymes, phosphorescence, chemical luminescence and coloration.

A chromatography unit for use in the nucleic acid detection method according to the present disclosure is described herein, provided with
a solid phase body carrier,
3 or more band-shaped probe regions with the probes fixed thereto in parallel to one another at different locations on the solid phase body carrier, and
2 or more position marker regions located parallel to one another and also to the probe regions in positions different from the 3 or more probe regions on the solid phase body carrier, wherein
three of the three or more probe regions are disposed at equal intervals between two position marker regions out of the two or more position marker regions.

The chromatography unit may be one wherein one or more probe regions are disposed at intervals equal to the intervals between the three probe regions on the opposite side of the two position markers to the three fixed probe regions.

The chromatography unit may be one wherein the solid phase body carrier has a tapering liquid contact part or liquid contact-forming marker at one end thereof to contact with the developing medium for nucleic acid chromatography.

The chromatography unit may be one wherein the liquid contact part-forming marker is a marker that makes visible a cutting site for forming the liquid contact part by cutting a part of the solid phase body carrier.

The chromatography unit may be one wherein the marker is sufficiently weak to allow the solid phase body carrier to be cut along the marker.

### BRIEF DESCRIPTION OF DRAWINGS

**FIG.** 1A shows an outline of one example of an amplification step in the detection method of the description;
**FIG.** 1B shows an outline of one example of another amplification step in the detection method of the description;
**FIG.** 2A shows one example of the flow of the detection method of the description;
**FIG.** 2B shows another example of the flow of the detection method of the description;
**FIG.** 2C shows another example of the flow of the detection method of the description;
**FIG.** 3 shows one example of a partially double-stranded nucleic acid;
**FIG.** 4 shows one example of a primer set and nucleic acid amplification step for obtaining a partially double-stranded nucleic acid;
**FIG.** 5 shows another primer set, etc. for obtaining a partially double-stranded nucleic acid;
**FIG.** 6 shows another primer set, etc. for obtaining a partially double-stranded nucleic acid;
**FIG.** 7 shows another primer set, etc. for obtaining a partially double-stranded nucleic acid;
**FIG.** 8 shows another set, etc. for obtaining an equivalent of a partially double-stranded nucleic acid;
**FIG.** 9 shows the detailed structure of a chromatography unit;
**FIG.** 10 is a plane view of a chromatography unit;
**FIG.** 11 shows the liquid contact part of a chromatography unit;
**FIG.** 12 shows results for amplification of genome DNA in an example;
**FIG.** 13 shows detection results obtained in an example of the description
**FIG.** 14 shows detection results obtained in an example of the description
**FIG.** 15 shows a membrane-type array prepared in an example;
**FIG.** 16 shows detection results obtained in an example of the description
**FIG.** 17 shows a membrane-type array (for chromatography) prepared in an example; **FIG.** 18 shows detection results obtained in an example of the description
**FIG.** 19 shows a chromatography unit prepared in an example;
**FIG.** 20 shows electrophoresis results for an amplification product;
**FIG.** 21 shows yield of an amplification product; and
**FIG.** 22 shows detection results for a target nucleic acid from nucleic acid chromatography using a partially double-stranded nucleic acid.
**FIG.** 23 shows electrophoresis results for an amplification product;
**FIG.** 24 shows yield of an amplification product; and
**FIG.** 25 shows detection results for a target nucleic acid from nucleic acid chromatography using a partially double-stranded nucleic acid.
**FIG.** 26 shows electrophoresis results for an amplification product;
**FIG.** 27 shows yield of an amplification product; and
**FIG.** 28 shows detection results for a target nucleic acid from nucleic acid chromatography using a partially double-stranded nucleic acid.
**FIG.** 29 shows electrophoresis results for an amplification product;
**FIG.** 30 shows yield of an amplification product; and
**FIG.** 31 shows detection results for a target nucleic acid from nucleic acid chromatography using a partially double-stranded nucleic acid.

### DESCRIPTION OF EMBODIMENTS

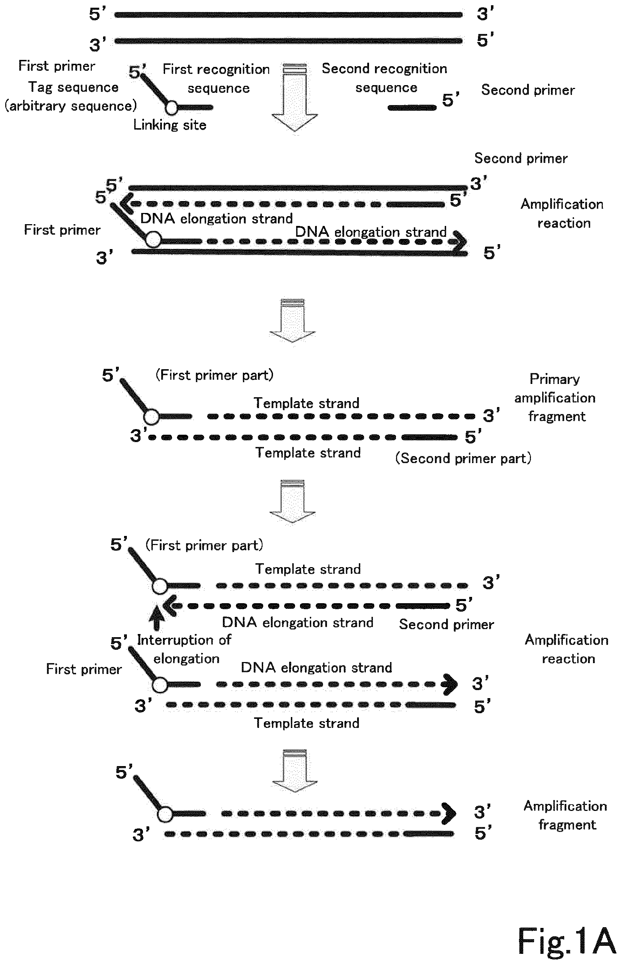

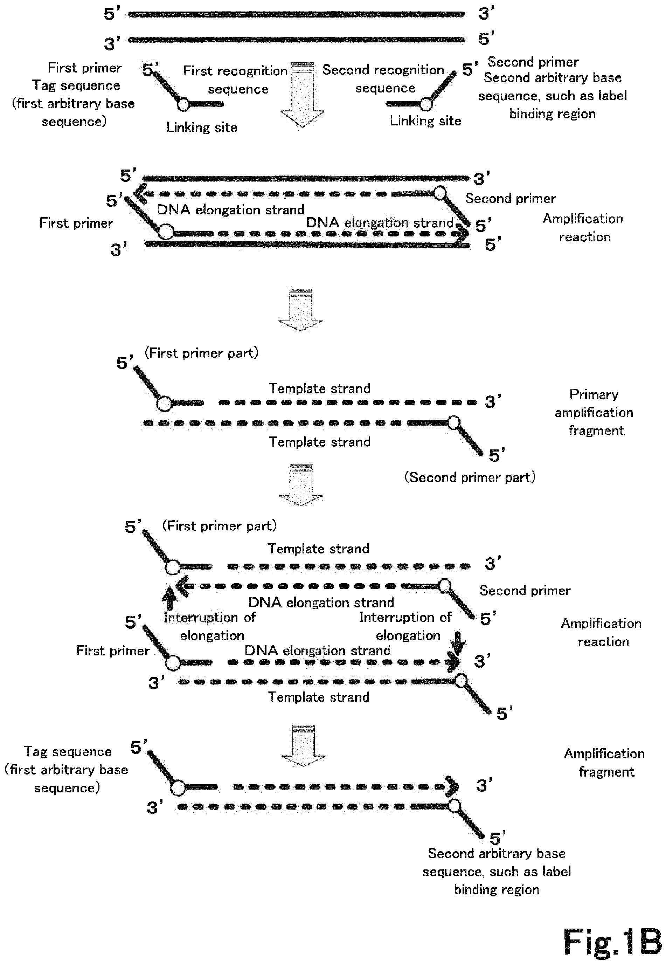

The present disclosure relates to a method for detecting a target nucleic acid, and to a nucleic acid amplification agent and the like. The method for detecting a target nucleic acid of the present method features the use of the following first primer and second primer. **FIG.** 1A and **FIG.** 1B illustrate one example of the amplification step in the detection present method.

As shown in **FIG.** 1A, the first primer comprises an arbitrary first base sequence, such as a tag sequence complementary to a detection probe pre-associated with the target nucleic acid, and a first recognition sequence that recognizes a first base sequence in the target nucleic acid, and has a linking site capable of inhibiting or arresting a DNA polymerase reaction disposed between the first arbitrary base sequence and the first recognition sequence, while the second primer contains a second recognition sequence that recognizes a second base sequence in the target nucleic acid.

The linking site inhibits or arrests the DNA polymerase reaction. That is, because this linking site contains no natural bases among other reasons, it cannot be a template for a DNA elongation reaction by a DNA polymerase. Thus, when the single-stranded DNA amplified by the first primer becomes a template strand, and is then amplified with the second primer as shown in **FIG.** 1A, the DNA elongation reaction from the second primer is inhibited or arrested at the 3' end beginning at the site paired with the linking site. Consequently, it is believed that the amplified fragment (double-stranded DNA fragment) obtained in the amplification step is provided at one end with the first arbitrary base sequence as a protruding single strand, and with a double-stranded part formed by base pairing.

**FIG.** 1B shows the amplification process when the second primer also has a second arbitrary base sequence, and has the linking site disposed between the second arbitrary base sequence and a second recognition sequence. As in the case of the first primer shown in **FIG.** 1A, when single-stranded DNA amplified by the second primer becomes a template strand, and is then amplified with the first primer as shown in **FIG.** 1B, the DNA elongation reaction from the first primer is inhibited or arrested at the 3' end beginning at the site paired with the linking site. Consequently, it is believed that the amplified fragments (double-stranded DNA fragments) obtained in the amplification step are provided at one end with a tag sequence as a protruding single strand, at the other end with an arbitrary base sequence as a protruding single strand, and with a double-stranded part formed by base pairing. Developing

This supports the idea that a target nucleic acid can be detected rapidly and with extremely high sensitivity when an amplified fragment obtained by performing an amplification step with a DNA polymerase on a sample containing the target nucleic acid, using a primer set that has a first arbitrary base sequence as a tag sequence complementary to a detection probe pre-associated with the target nucleic acid, is hybridized as is with the detection probe without being denatured. As shown in **FIG.** 1A and **FIG.** 1B, it is thought that because the resulting double-stranded DNA fragment comprises double-stranded parts formed with the first base sequence and second base sequence of the target nucleic acid, with the tag sequence protruding at the end as a single strand, this single strand can hybridize efficiently with the probe. Sensitivity is improved when hybridization efficiency is increased.

At least one of the following effects is achieved with the detection present method.
(1) More efficient (rapid) hybridization
(2) More efficient labeling
(3) Greater detection sensitivity
(4) A simpler (more rapid) process-particular by omission of the step of denaturing the double strand into a single strand

An oligonucleotide derivative having a base sequence containing such a linking site is itself useful as a primer or other nucleic acid amplifying agent. Moreover, a nucleic acid amplifying method using such a primer, the resulting double-stranded DNA fragment, and a hybridization composition containing this fragment can also have at least one effect according to their embodiments.

The Description relates separately to a method of detecting a target nucleic acid by nucleic acid chromatography, and a solid phase body or the like suited to this method. With the method of detecting a target nucleic acid disclosed in this Description, it is possible to specify a partially double-stranded nucleic acid that is desirable for hybridization by nucleic acid chromatography, and thereby achieve efficient and highly accurate hybridization. Unlike hybridization on an ordinary array, hybridization in nucleic acid chromatography involves movement of a developing medium based on the capillary effect and evaporation of the developing medium, making desirable hybridization hard to achieve. However, by preparing the partially double-stranded nucleic acid disclosed in this Description for the purpose of detecting a target nucleic acid in nucleic acid chromatography, it is possible to achieve efficient and highly accurate hybridization.

With the method disclosed in this Description, moreover, because hybridization is performed by bringing part of a solid phase body into contact with a developing medium containing an amplification reaction solution and causing the developing medium to move on a solid phase body carrier, it is possible to effectively prevent contamination and the like that occurs when part of a nucleic acid amplification reaction solution is supplied to a solid phase body, and to simplify the operations for the hybridization step.

Moreover, because the chromatography unit disclosed in this Description has position markers, a probe region corresponding to a target nucleic acid can be specified easily even when multiple target nucleic acids are being detected simultaneously, and the ease and precision of detection can be improved simultaneously. This is particularly suited to visual detection, so the presence or absence of the target nucleic acid can be detected at a glance with a visual detection system.

The various embodiments disclosed in this Description are explained in detail below.

In this Description, a "nucleic acid" is a polymer made up of nucleotides, the number of which is not limited. Nucleic acids encompass oligonucleotides formed by linking tens of nucleotides, as well as longer polynucleotides. In addition to single-stranded or double-stranded DNA, nucleic acids include single-stranded or double-stranded RNA, as well as DNA/RNA hybrids, DNA/RNA chimeras and the like. Nucleic acids may consist of natural bases, nucleotides and nucleosides, but may also consist partly of non-natural bases, nucleotides and nucleosides. In addition to all kind of DNA and RNA including cDNA, genome DNA, synthetic DNA, mRNA, total RNA, hnRNA and synthetic RNA, nucleic acids include peptide nucleic acids, morpholino nucleic acids methylphosphonate nucleic acids, S-oligonucleic acids and other artificially synthesized nucleic acids. These may be single-stranded or double-stranded.

In this Description, a "target nucleic acid" is any nucleic acid the existence or amount of which is to be detected, without any particular limitations. A target nucleic acid may be natural or artificially synthesized. Natural target nucleic acids include bases or base sequences that are genetic markers for physical properties and genetic diseases, and for the occurrence, diagnosis, prognosis, and drug and treatment selection for cancer and other specific disorders in humans, non-human animals and other organisms. Typical examples include SNPs and other polymorphisms and congenital and acquired mutations. Nucleic acids derived from pathogens, viruses and other microorganisms are also considered target nucleic acids. Examples of synthetic target nucleic acids include nucleic acids synthesized artificially for some kind of recognition purposes. They also include amplification products obtained by nucleic acid amplification reactions from some kind of natural or artificial nucleic acids.

The sample discussed below or a nucleic acid fraction thereof may be used as is for the target nucleic acid, but it is desirable to use an amplification product obtained by amplifying multiple target nucleic acids in a PCR amplification reaction, and preferably a multiplex PCR amplification reaction.

In this Description, a "sample" is a sample that may contain a target nucleic acid. There are no particular limitations on the source of the sample, but examples of samples that may contain target nucleic acids include various biological samples (blood, urine, sputum, saliva, tissue, cells (including various cultured animal cells, cultured plant cells and cultured microbial cells) and the like), or extract samples of DNA extracted from such biological samples. They also include DNA samples and the like obtained by extracting RNA from such biological samples and converting it to DNA. Fractions containing nucleic acids from these various samples can be obtained by a person skilled in the art on the basis of appropriate prior art.

In this Description, a "target sequence" is a sequence consisting of one or two or more bases specific to the target nucleic acid that is the object of detection. For example, it may be a partial sequence with low homology among the target nucleic acids, or a sequence with low complementarity or homology with other nucleic acids that may be contained in the sample. The target sequence may be a sequence specific to the target nucleic acid. The target sequence like this may have been artificially modified.

In this Description, nucleic acid chromatography is chromatography using a porous solid phase body carrier capable of dispersing and moving a liquid (developing medium) by capillary action, in which a nucleic acid is moved by this liquid inside the solid phase body carrier, forms a hybridization product by specific base pairing with a previously-prepared probe in the solid phase body carrier, and is thereby captured by the solid phase body carrier.

Typical and non-limiting specific examples of the disclosures of this Description are explained in detail below with reference to the drawings. These detailed explanations are aimed simply at showing preferred examples of the disclosures of the Description in detail so that they can be implemented by a person skilled in the art, and are not intended to limit the scope of the disclosures of the Description. The additional features and disclosures disclosed below may be used separately or together with other features to provide a further improved method of detecting a target nucleic acid or the like.

The combinations of features and steps disclosed in the detailed explanations below are not essential for implementing the disclosures of the Description in the broadest sense, and are presented only for purposes of explaining typical examples of the disclosures of the Description in particular. Moreover, the various features of the typical examples above and below and the various features described in the independent and dependent claims do not have to be combined in the same way as in the specific examples described here, or in the listed order, when providing addition useful embodiments of the disclosures of the Description.

All features described in the Description and/or Claims are intended as individual and independent disclosures restricting the initial disclosures and the claimed matter specifying the present invention separately from the constitution of features described in the Examples and/or Claims. Moreover, all descriptions of numerical ranges and groups or sets are intended to include intermediate configurations for purposes of restricting the initial disclosures and the claimed matter specifying the present method.

### [Method of detecting target sequence in target nucleic acid]

The detection method disclosed in this Description is provided with a step of preparing a solid phase bodycomprising a detection probe, a step of using a first primer and second primer to perform nucleic acid amplification on the sample, a hybridization step in which an amplified fragment obtained in the amplification step is brought into contact with the detection probe in such a way that it is hybridizable by means of the tag sequence, and a detection step in which the hybridization product of the amplified fragment and the detection probe is detected on the solid phase body. The detection method disclosed in this Description is applicable to one or two or more kinds of nucleic acids, and more specifically, is used to detect target sequences associated with characteristic sequences in these nucleic acids. A series of steps for one kind of target nucleic acid is mainly explained below, but the following steps are also applicable to simultaneous detection of multiple or many target nucleic acids.

### (Solid phase body preparation step)

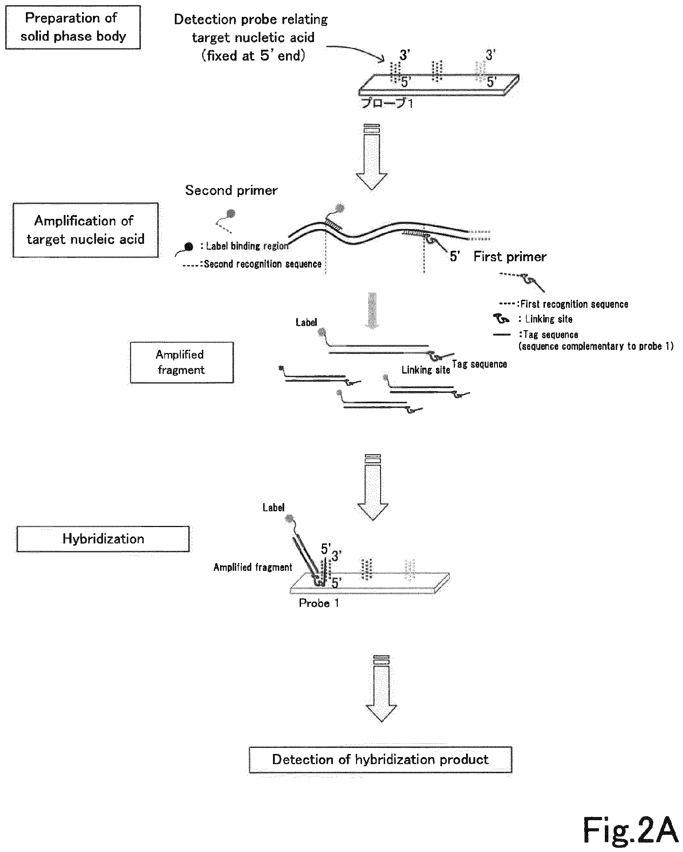

As shown in **FIG.** 2A, the detection method disclosed in this Description (also called simply "the detection method" below) may comprise a step of preparing a solid phase body. Such a solid phase body may be prepared in advance before implementing the detection method, or may be obtained commercially, or may be prepared each time the detection method is implemented.

As shown in **FIG.** 2A, the solid phase body may comprise multiple detection probes, each having a different unique base sequence as a detection sequence, disposed on a carrier. By preparing such a solid phase body, it is possible to avoid research into probe design and synthesis, array preparation, and hybridization conditions.

**FIG.** 2A shows one example of a solid phase body. Each detection probe has a detection sequence, which is a unique base sequence for purposes of probing. Such a detection sequence can be designed independently of the characteristic sequence of the target nucleic acid, or in other words of the target sequence. Designing the sequence independently of the target sequence makes it possible to inhibit or eliminate non-specific binding between the detection sequences of multiple detection probes, and to incorporate considerations of optimum hybridization temperature, time and other hybridization conditions into the design. It also makes it possible to always use the same detection probe independently of the kind of target nucleic acid.

The preferred length of the detection sequence is not specifically limited but is preferably 20 bases to 50 bases. The detection sequence having the bases in length can ensure both of the specificity of each of the detection sequence and hybridization efficiency. For example, the detection sequence having the bases in length may be selected from 46 bases sequence which base sequences described by SEQ IDs: 1 to 100 and their complementary sequences are combined and their modified sequences which addition or deletion of base is made. More preferably, the length of the detection sequence is 20 bases to 25 bases. For example, the detection sequence having such number of bases in length may be selected from sequences of 23 bases, each of which is described by any one of SEQ IDs: 1 to 100 and their complementary sequences and their modified sequences which addition or deletion of base is made arbitrarily. Tag sequence of the first primer is the sequence which pairs the detection sequence. Therefore, the length of the tag sequence is preferably 20 to 50 bases, more preferably 20 to 25 bases as same as the detection sequence.

The detection sequence in the detection probe may be base sequences of SEQ ID NO: 1 to SEQ ID NO: 100 or their complementary base sequences. These base sequences have the same base length and have a melting temperature (Tm) of 40°C or higher and 80°C or lower, more preferably 50°C or higher and 70°C or lower, thereby giving homogeneous hybridization results under the same hybridization conditions. As discussed above, two different sequences selected from this group of base sequences may be combined together. Also, bases may be added, deleted, substituted or the like in these sequences as long as specificity is not lost. Detection sequences for detection probes to be used simultaneously are preferably selected from either the nucleotide sequences (group) represented by SEQ ID NOS:1∼100, or from the nucleotide sequences (group) complementary to these sequences.

The detection sequences of the detection probes may be selected and used appropriately from such candidate base sequences or their complementary sequences, but of these, it is desirable to use a probe set consisting only of one or two or more probes each having as a detection sequence one or two or more base sequences selected from the base sequences listed in the following **table** or their complementary sequences, or a probe set consisting only of probes each having all of the following base sequences or their complementary sequences as detection sequences. By selecting such base sequences as probe sequences, it is possible to shorten the hybridization time, resulting in more rapid hybridization.

**[Table 2]**

| Name | Se(5→3') | SEQ.ID. |
|---|---|---|
| D1-001 | TGTTCTCTGACCAATGAATCTGC | 1 |
| D1-002 | TGGAACTGGGAACGCTTTAGATG | 2 |
| D1-003 | TTCGCTTCGTTGTAATTTCGGAC | 3 |
| D1-005 | TAGCCCAGTGATTTATGACATGC | 5 |
| D1-006 | CGCTCTGGTTACTATTGGACGTT | 6 |
| D1-010 | GAGTAGCAGGCAAATACCCTAGA | 10 |
| D1-012 | AGTCATACAGTGAGGACCAAATG | 12 |
| D1-014 | TGCTCACTTACATTACGTCCATG | 14 |
| D1-016 | AGGTCCGGTAGTAATTTAGGTGC | 16 |
| D1-020 | TATTCTACCAACGACATCACTGC | 20 |
| D1-023 | CATCTCCAAGAATTGACCCACCA | 23 |
| D1-025 | GAAGGATCGCTTTTATCTGGCAT | 25 |
| D1-026 | CATTTGTCAGGTACAGTCCACTT | 26 |
| D1-027 | GCCCACACTCTTACTTATCGACT | 27 |
| D1-030 | CCGTCTGGGTTAAAGATTGCTAG | 30 |
| D1-035 | ATGCCGTTGTCAAGAGTTATGGT | 35 |
| D1-038 | CGCGACATTTAGTCCAGGAGATG | 38 |
| D1-040 | AGACAATTAGAATCAGTGCCCCT | 40 |
| D1-041 | GCATTGAGGTATTGTTGCTCCCA | 41 |
| D1-044 | GAGTCCGCAAAAATATAGGAGGC | 44 |
| D1-045 | GCCTCACATAACTGGAGAAACCT | 45 |
| D1-050 | GGGATAGGTATTATGCTCCAGCC | 50 |
| D1-052 | GCCTATATGAACCAAGCCACTGC | 52 |
| D1-062 | CTAGCACAATTAATCAATCCGCC | 62 |
| D1-064 | GCCTATAGTGTCGATTGTCCTCG | 64 |
| D1-065 | CGATCACGGATTAATGTCACCCC | 65 |
| D1-077 | CGCAGTTTGCAAGAACGAACAAA | 77 |
| D1-084 | CCGTGTGTATGAGTATGACAGCA | 84 |
| D1-089 | GAGTCGAAGACCTCCTCCTACTC | 89 |
| D1-090 | ATGCCAATATGTACTCGTGACTC | 90 |
| D1-095 | TGCCGGTTATACCTTTAAGGACG | 95 |
| D1-097 | CGCGGTACTATTAGAAAGGGCTA | 97 |
| D1-100 | TGCAGTGTAAGCAACTATTGTCT | 100 |

The detection sequence in the detection probe is called also as an orthonormalization sequence and is designed based on the calculations on a consecutive identical length, melting temperature prediction by the Nearest-Neighbor method, a Hamming distance, secondary structure prediction on DNA sequences having certain base lengths obtained from random numbers. The orthonormalization sequences are base sequences of nucleic acids which have homogeneous melting temperatures and thus are designed so as to have the melting temperatures in a constant range, which do not inhibit hybridization with the complementary sequences because nucleic acids are structured intramolecularly, and which do not stably hybridize with base sequences other than complementary base sequences. Sequences contained in one orthonormalization sequence group hardly react or do not react to sequences other than a desired combination or within their sequences. When orthonormalization sequences are amplified by PCR, the amount of the nucleic acids quantitatively amplified correspond to an initial amount of the nucleic acids having the orthonormalization sequences without influenced by a problem such as cross-hybridization as mentioned above. Such orthonormalization sequences are reviewed in H. Yoshida and A. Suyama, "Solution to 3-SAT by breadth first search", DIMACS Vol.54, 9-20 (2000) and Japanese Patent Application No. 2003-108126. The orthonormalization sequences can be designed by using the methods described in these documents.

The detection probe is fixed on a carrier. A solid phase body carrier may be used as such a carrier. For example, the material of the carrier is not particularly limited, and may be plastic or glass. It may also be a porous material such as cellulose, nitrocellulose, nylon or the like. This kind of porous carrier is particularly suited to hybridizing a detection probe fixed to a solid phase body carrier with an amplified fragment by affinity chromatography.

There are no particularly limitations on the form of the carrier, which may be in the form of beads or a flat plate as shown in **FIG.** 1. Preferably the solid phase body is an array (especially a microarray) of multiple detection probes fixed in a specific arrangement, with the carrier in the form of a solid flat plate. Because multiple detection probes can be fixed in an array, it is convenient for exhaustively detecting various different target nucleic acids simultaneously. The solid phase body may also be provided with multiple compartmentalized array regions on the carrier. All of these multiple array regions may have a detection probe set of the same combination fixed thereto, or each may be have a detection probe set of a different combination. If detection probe sets of different combinations are fixed to the multiple array regions, each individual array region may be assigned to detecting a target nucleic acid in a different gene.

The mode of hybridization (discussed below) may be considered in designing the form of the carrier. For example, when hybridization is performed in a microtube such as the Eppendorf tube™, which is widely used in testing and research, the array regions on the carrier are preferably of a size and form that allow them to be immersed in the hybridization solution contained in the tube. Such a carrier typically has a plane area of 150 mm² or less, an aspect ratio of 1.5 to 20, and a thickness of 0.01 mm to 0.3 mm.

When hybridization is performed using the principles of affinity chromatography with a detection probe fixed on a porous solid phase body carrier, it is desirable that at least the end of the carrier be of a size (width) and form that allow it to be immersed in a hybridization solution supplied to a microtube such as the Eppendorf tube™, which is widely used in testing and research. More preferably, it is in a long thin form provided with a site that can be contained within this kind of tube from near the bottom to the top of the tube.

The detection probes may be fixed by any mode without limitation. The detection probes may be fixed at the 3'end or 5' end on the carrier. The modes may be covalent or non-covalent. The detection probes may be fixed on the surface of the carrier by any various well-known methods in the art. For example, detection probe are provided so that droplets of probe solution draw predetermined planner pattern on the carrier using a method for ejecting fine droplets of the probe solution. Then, the detection probe can be dried by heating as necessary and fixed. Further, for example, for the immobilization of the detection probe to the solid carrier, amino group may be added to the detection probe. For increasing the adhesion of the probe to the carrier, protein, such as albumin may be linked. Further, it is possible to improve the adhesion with various types of radiation such as UV irradiation or heat treatment.

The detection probe may also be provided with a suitable linker sequence for linking with the surface of the carrier. The linker sequence is preferably has the same length and the same base sequence in all detection probes.

The detection probe is supplied to the solid phase body carrier in a specific pattern according to the mode of hybridization (discussed below). When the entire solid phase body is to be immersed in the hybridization solution, this is typically a pattern of dots corresponding to the individual detection probes. When the solid phase body is to be developed with the hybridization solution as a mobile phase, streams (bands) corresponding to individual detection probes are typically arranged at one or two or more developing sites in the direction of developing.

### (Amplification step)

As shown in **FIG.** 2A, the amplification step is performed using a first primer and a second primer. Various known methods for obtaining double-stranded DNA fragments by amplifying DNA by a DNA polymerase reaction such as PCR can be used as the nucleic acid amplification method in the nucleic acid amplification step.

### (First primer)

The first primer comprises a tag sequence complementary to the detection probe associated with the target nucleic acid, and a first recognition sequence that recognizes a first base sequence in the target nucleic acid. The lengths of these base sequences are not particularly limited, and can be determined appropriately according to the nature of the target sequence of the target nucleic acid.

### (First recognition sequence)

The first recognition sequence is a sequence for amplifying the target nucleic acid by nucleic acid amplification, and can hybridize specifically with a first base sequence constituting part of the target sequence of the target nucleic acid. The first recognition sequence is designed with a degree of complementarity that allows it to hybridize very selectively with the first base sequence. Preferably it is designed to be entirely complementary (specific).

### (Tag sequence)

A tag sequence is a sequence that makes it possible for the amplified fragment to hybridize with the detection probe, and because it detects the target nucleic acid, a tag sequence is designed so that it can hybridize with the detection sequence of the detection probe for each target nucleic acid. Typically, it is a base sequence complementary to the detection sequence. Therefore, one target nucleic acid is matched to one detection probe. As explained above, the base length of the tag sequence preferably matches the base length of the detection sequence of the detection probe, and is preferably 20 to 50 bases or more preferably 20 to 25 bases.

The first base sequence and second base sequence in the target nucleic acid may be configured in any way relative to the target nucleic acid. For example, when detecting a mutation on DNA, they may be made to contain mutation sites in one or two or more bases only in one base sequence, or may contain mutation sites in both base sequences. The first primer has such a tag sequence and first recognition sequence, and together with the natural bases or artificial homologs of natural bases that constitute these base sequences, it also has a backbone that allows base pairing with a natural nucleic acid. Typically, it is an oligonucleotide or derivative thereof.

### (Linking site)

The part of the primer having the tag sequence is not directly linked to the other part of the primer having the first recognition site, and there is a linking site between the two. When incorporated into the template strand, the linking site is a site capable of inhibiting or arresting a DNA polymerase reaction. The DNA polymerase reaction does not further elongate the DNA strand when there is no nucleic acid (or bases) to serve as a template. Therefore, the linking site of the disclosure has a structure that cannot serve as a template during DNA elongation by DNA polymerase. That is, this linking site does not contain natural bases or natural base derivatives (natural bases or the like) that pair with natural bases. By not including such natural bases and the like, it is possible to prevent the site from being a template, and inhibit or prevent DNA strands from being elongated by DNA polymerase. Therefore, this linking site may consist solely of a simple skeletal strand having no natural bases or the like. That is, it may be a sugar-phosphate backbone or other known backbone used in artificial oligonucleotides. The DNA polymerase includes various known DNA polymerases. Typical examples include DNA polymerases used in various kinds of PCR and other nucleic acid amplification methods.

This linking site may also be a chain-like linking group containing a single-stranded structure with an element number of 2 to 40, adjoining a nucleotide via a phosphate diester bond. If the element number is 1 or less, the DNA polymerase reaction may not be completely inhibited or arrested, while if the element number is over 40, nucleotide solubility may be diminished. Considering the effect of inhibiting or arresting the DNA polymerase reaction, the element number of the chain-like linking group is preferably 2 to 36 or more preferably 3 to 16.

This linking site contains a single bond in order to facilitate rotation at the linking site, and examples of single bonds include carbon-carbon, carbon-oxygen, carbon-nitrogen and S-S single bonds. This linking site preferably consists primarily of this single bond. As long as the linking site contains a single bond an aromatic ring or cycloalkane may also be included in part of the site.

An optionally substituted alkylene chain or polyoxyalkylene chain with an element number of 2 to 40 is preferably included as this linking site. Such a chain-like linking structure is structurally simple and easy to introduce as a linking site.

One example of this linking site is the linking site represented by Formula (1) below:

5'-O-CₘH₂ₘ-O-3' Formula (1)

(wherein 5' represents the oxygen atom of a phosphate diester bond at the 5' end, 3' represents the phosphorus atom of a phosphate diester bond at the 3' end, and m is an integer from 2 to 40).

In Formula (1), m is more preferably 2 to 36, or still more preferably 3 to 16. A substituent of H in Formula (1) is typically an alkyl group, alkoxy group, hydroxyl group or the like. The number of carbon atoms in an alkyl group or alkoxy group is preferably 1 to 8, or more preferably 1 to 4. When there are 2 or more substituents, they may be the same or different. Preferably there are no substituents.

Another example of a linking site is the linking site represented by Formula (2) below:

5'-(OCₙH₂ₙ)ₗ-v3' Formula (2)

(wherein 5' represents the oxygen atom of a phosphate diester bond at the 5' end, 3' represents the phosphorus atom of a phosphate diester bond at the 3' end, n is an integer from 2 to 4, 1 is 2 or an integer greater than 2, and (n + 1) x 1 is 40 or an integer smaller than 40).

In Formula (2), (n + 1) x 1 is preferably 2 to 36, or more preferably 3 to 16. A substituent of H in Formula (2) may be similar to the substituent in Formula (1).

The chain-like site shown below is an example of this linking site.

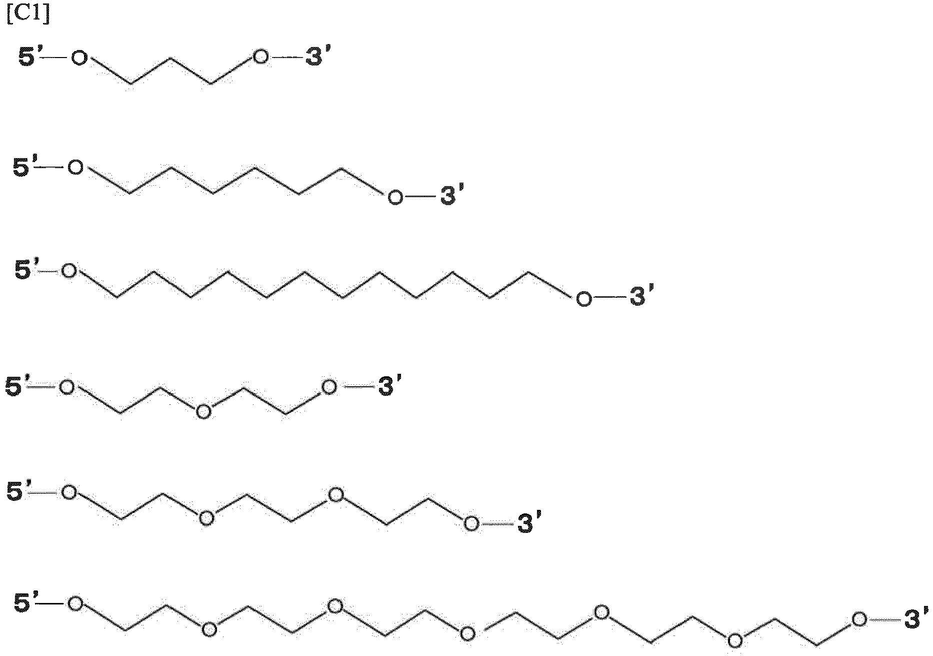

The chain-like site shown below is another example of this linking site.

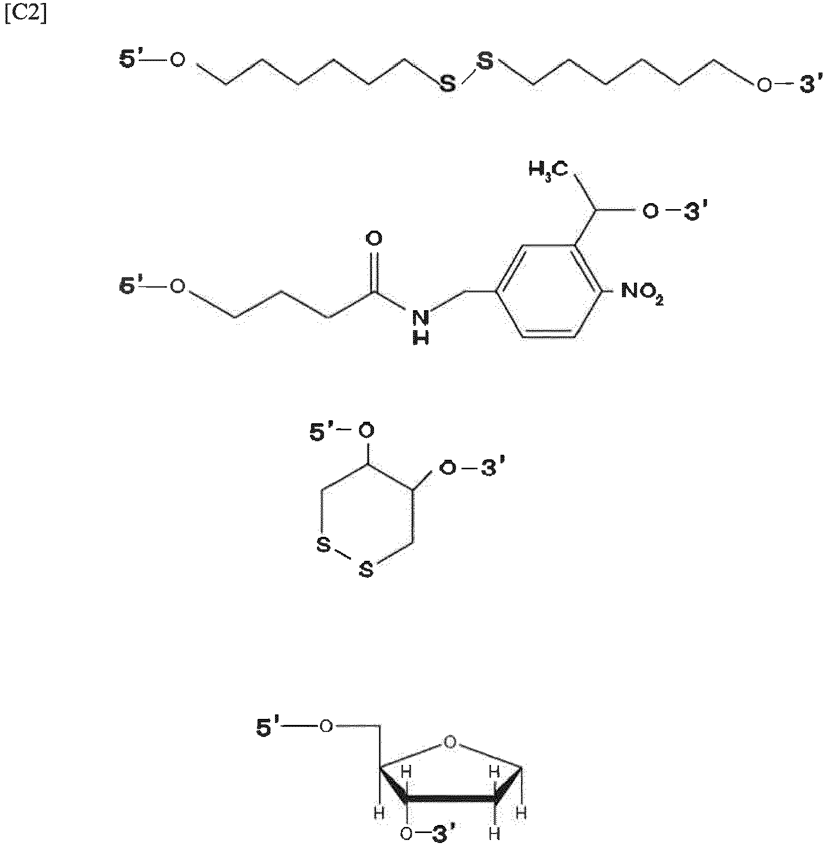

The first primer has a first recognition sequence and a tag sequence, and apart from the natural bases or artificial homologs of natural bases that constitute these base sequences, it consists principally of a backbone that allows base pairing with a natural nucleic acid. Typically it is an oligonucleotide or derivative thereof.

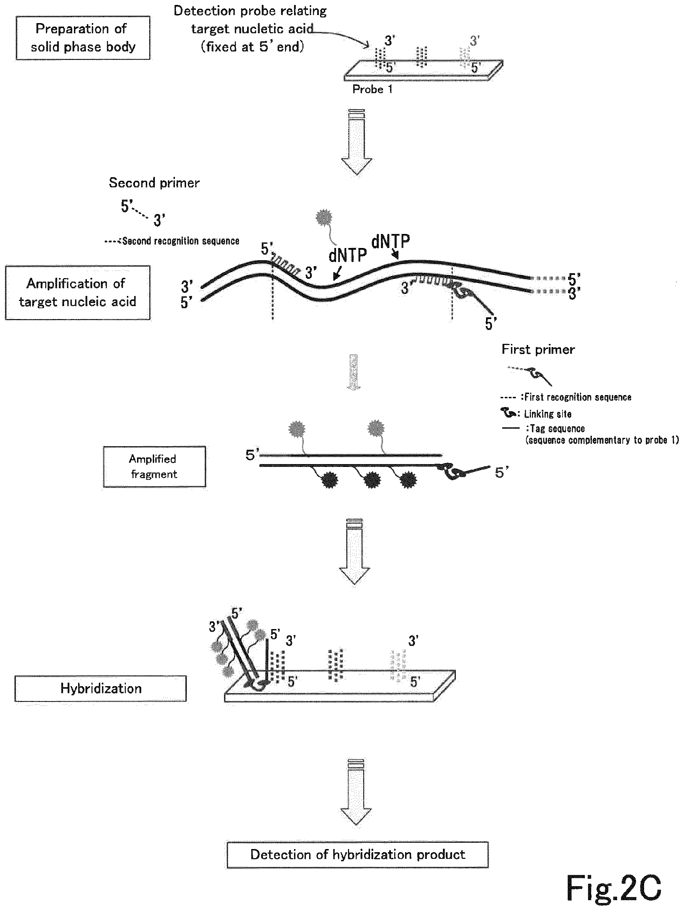

The first primer preferably has a tag sequence, a linking site and a first recognition sequence in that order from the 5' end. With such a configuration, when a DNA strand amplified by such a primer becomes a template strand for amplification, the elongation reaction is inhibited or arrested on the 5' side of the linking site derived from the first primer in the template strand, or in other words further towards the 3' end in the DNA strand elongated with DNA polymerase. This results in an amplified fragment having as its 3' end a base that pairs with a base (or a base near a base) of a nucleotide adjacent to the 3' side of the linking site derived from the first primer in the template strand, or with a base adjacent to that base, and lacking a complement strand to the tag sequence in the first primer (**FIGS.** 1A and 1B, **FIGS.** 2A and 2C).

A sequence unrelated to the tag sequence or first recognition sequence may also be included near the linking site, or in other words on the 3' and 5' side of the linking site. This makes it possible to reduce or avoid the effect of unintentionally promoting or arresting the DNA elongation reaction with respect to the tag sequence or first recognition sequence in the elongated strand due to the presence of the linking site when the first primer becomes a template strand.

### (Second primer)

As shown in **FIG.** 2A, the second primer contains a second recognition sequence that recognizes a second base sequence in the target nucleic acid. The lengths of these base sequences are not particularly limited, and may be determined appropriately according to the nature of the target sequence in the target nucleic acid.

### (Second recognition sequence)

The second recognition sequence is a sequence for amplifying a target nucleic acid with a first primer by nucleic acid amplification, and is capable of hybridizing specifically with a second base sequence constituting another part of the target sequence in the target nucleic acid. The second recognition sequence is designed to be sufficiently complementary so that it can hybridize very selectively with the second base sequence. Preferably it is designed to be completely complementary (specific).

### (Label-binding region)

As shown in **FIG.** 2A, the label-binding region may be provided in advance with a label. The purpose of the label is to detect a double-stranded DNA fragment bound to the detection probe on the solid phase body. A conventionally known substance may be selected and used appropriately as the label. Possibilities include various dyes including fluorescent substances that emit a fluorescent signal by themselves when excited, as well as substances that emit various signals in combination with second components by enzyme reactions or antigen-antibody reactions. Typically, a fluorescent label such as Cy3, Alexa555, Cy5 or Alexa647 may be used. The label-binding region is provided with the label linked to the second base sequence by a known method, either directly or via a suitable linker.

In this Description, a "label" is a substance that allows a substance or molecule that is an object of detection to be distinguished from others. The label is not particularly limited, but typical examples include labels using fluorescence, radioactivity, enzymes (for example, peroxidase, alkaline phosphatase, etc.), phosphorescence, chemoluminescence, coloration and the like.

The label is preferably a luminescent substance or chromogenic substance that presents luminescence or coloration that is detectable with the naked eye. That is, it is preferably a substance that can produce a signal that is itself directly visible to the naked eye, without the need for another component. This makes the detection process easier and more rapid. Typical examples of such substances include various pigments, dyes and other colorants. Equivalent substances include gold, silver and other precious metals, and copper and various other metals and alloys, as well as organic compounds containing these metals (which may be complex compounds). Mica and other inorganic compounds are also equivalent to colorants.

Typical examples of such labels include various dyes and pigments and chemoluminescent substances including luminol, isoluminol, acridinium compounds, olefins, enol ethers, enamines, aryl vinyl ethers, dioxenes, aryl imidazole, lucigenin, luciferin and aequorin. Other examples include latex or other particles labeled with these labels. Other examples include colloids or sols including gold colloids or sols and silver colloids or sols. Metal particles, inorganic particles and the like are also possible.

As discussed above, part of the label may be provided with particles. The average particle diameter of latex or other particles constituting part of the label is not particularly limited, but the mean particle diameter may be 20 nm to 20 µm for example, and is typically 40 nm to 10 µm, or preferably 0.1 µm to 10 µm, or more preferably 0.1 µm to 5 µm, or still more preferably 0.15 µm to 2 µm. Depending on the pore diameter of the solid phase body carrier 210, 500 nm or less is desirable, 250 nm or less is more desirable, 100 nm or less is still more desirable, and 50 nm or less is most desirable. The lower limit is preferably 0.1 nm or more, or more preferably 1 nm or more. For example, 0.1 nm to 250 nm is more desirable, and 1 nm to 250 nm is still more desirable. 0.1 nm to 100 nm is yet more desirable, and 1 nm to 50 nm is most desirable.

Preferred are particles made of a water-insoluble polymer material that can be suspended in an aqueous solution. Examples include polyethylene, polystyrene, styrene-styrene sulfonate copolymer, acrylic acid polymers, methacrylic acid polymers, acrylonitrile polymers, acrylonitrile-butadiene-styrene, polyvinyl acetate-acrylate, polyvinyl pyrrolidone or vinyl chloride-acrylate. Latex particles of such polymers having surface carboxyl, amino or aldehyde groups or other active groups are also possible.

The label-binding region may also be provided with a molecule or substance (hereunder sometimes called the label binding substance) capable of binding these so as to allow ultimate recognition by the label. Protein-protein interactions and interactions between proteins and low-molecular-weight compounds and the like may also be used for example. Examples include antibodies in antigen-antibody reactions, biotin in avidin (streptavidin)-biotin systems, digoxigenin in anti-digoxigenin (DIG)-digoxigenin (DIG) systems, and haptens such as FITC and the like in anti-FITC-FITC systems. In this case, the label ultimately used in detection is modified so that it also functions as a site for binding between the label binding substance and another molecule or substance (an antigen for example, such as streptavidin, anti-FITC or the like) that interacts with the label binding substance. When the amplification product has a label binding substance, a complex can be formed between the label binding substance of the amplification product and a label provided with a site that binds with the label binding substance, either during the hybridization step or before or after this step, and the amplification product can be detected by means of the label.

Such labels and label binding substances can be obtained commercially, and methods for manufacturing labels and label binding substances and for labeling particles with labels and the like are well known, and these can be obtained by a person skilled in the art using appropriate known techniques. Moreover, binding between such labels or labeled particles or label binding substances and DNA and other oligonucleotides can be accomplished as necessary via amino groups and other functional groups, and these matters are well known in the field.

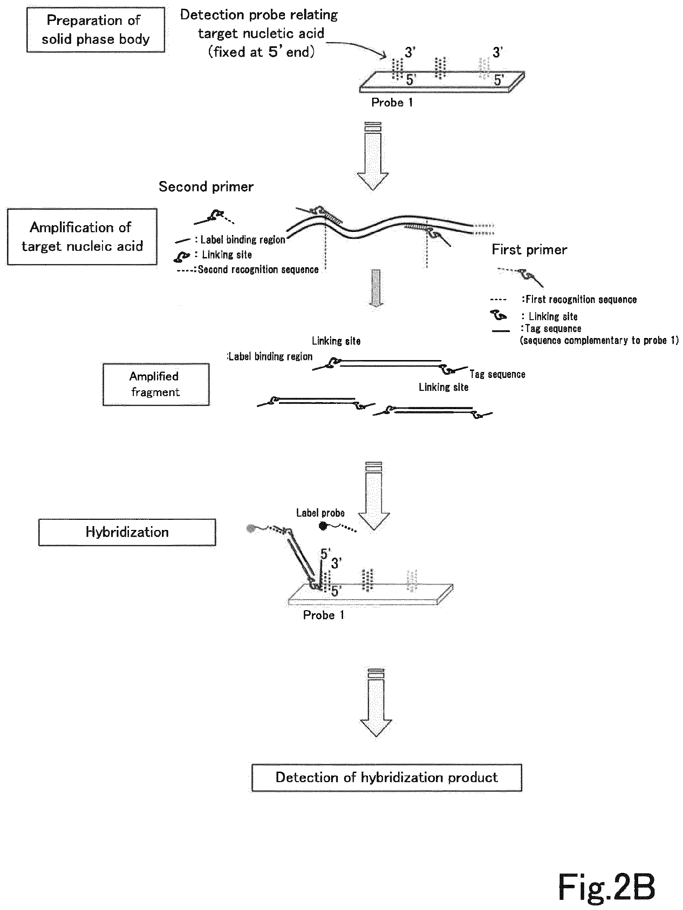

As shown in **FIG.** 2B, the second primer may be configured so that the label-binding region can bind the label or label binding substance. That is, it may have a specific base sequence as well as a label or label binding substance, and be capable of binding a labeling probe having a base sequence that recognizes the label binding sequence. Such a labeling probe is supplied to a double-stranded DNA fragment that has hybridized with the detection probe on the solid phase body in the hybridization step and detection step (discussed below), and can label the fragment.

As shown in **FIG.** 2C, the second primer may also lack a label-binding region. That is, an amplified fragment labeled with an introduced label at the DNA elongation site of the amplified fragment can be obtained by performing nucleic acid amplification using a nucleoside triphosphate containing a nucleoside derivative triphosphate provided with the label in the amplification step.

The second primer has a label-binding region as necessary in addition to the second recognition sequence, and has natural nucleotides or artificial bases homologous to these making up the base sequence of the second recognition sequence, as well as a backbone that allows base pairing with natural nucleic acids. Typically, it is an oligonucleotide or derivative thereof.

### (Linking site)

When there is a label-binding region, the label-binding region and the second recognition sequence may be linked directly, or there may be a linking site between the two. As shown in FIG. 2B, this is desirable when the label-binding region has a base sequence that interacts with and binds the labeling probe. The linking site has been explained already with reference to the first primer.

In the second primer, the label-binding region, linking site and second recognition sequence are preferably arranged in this order from the 5' end. With this configuration, when a DNA strand amplified by the second primer becomes a template strand for amplification by the first primer, the elongation reaction is arrested or inhibited further towards the 5' end from the linking site derived from the second primer in the template strand, or in other words further toward the 3' end in the new DNA strand elongated by the DNA polymerase. This results in a DNA amplified fragment having as its 5' end a base that pairs with a base (or a base near a base) of a nucleotide adjacent to the 3' end of the linking group derived from the second primer in the template strand, and lacking a complement strand to the (base sequence of) the label-binding region in the second primer (**FIG.** 1B, **FIG.** 2B).

A sequence unrelated to the label-binding region or second recognition sequence may also be included near the linking site, or in other words on the 3' or 5' side of the linking site. This makes it possible to reduce or avoid the effect of unintentionally promoting or arresting the DNA elongation reaction with respect to the label-binding region or second recognition sequence in the elongated strand due to the presence of the linking site when the second primer becomes a template strand.

Such a primer can be synthesized in accordance with ordinary oligonucleotide synthesis methods. For example, the linking site may be synthesized using a phosphoramidite reagent having an alkylene chain. Such reagents are well known, and can be obtained from GlenResearch or the like for example. Examples include the following reagent. In the formula below, DMT represents a typical dimethoxytrityl group as a hydroxyl protecting group, but this may also be another known hydroxyl protecting group. PA in the formula below represents a phosphoramidite group.

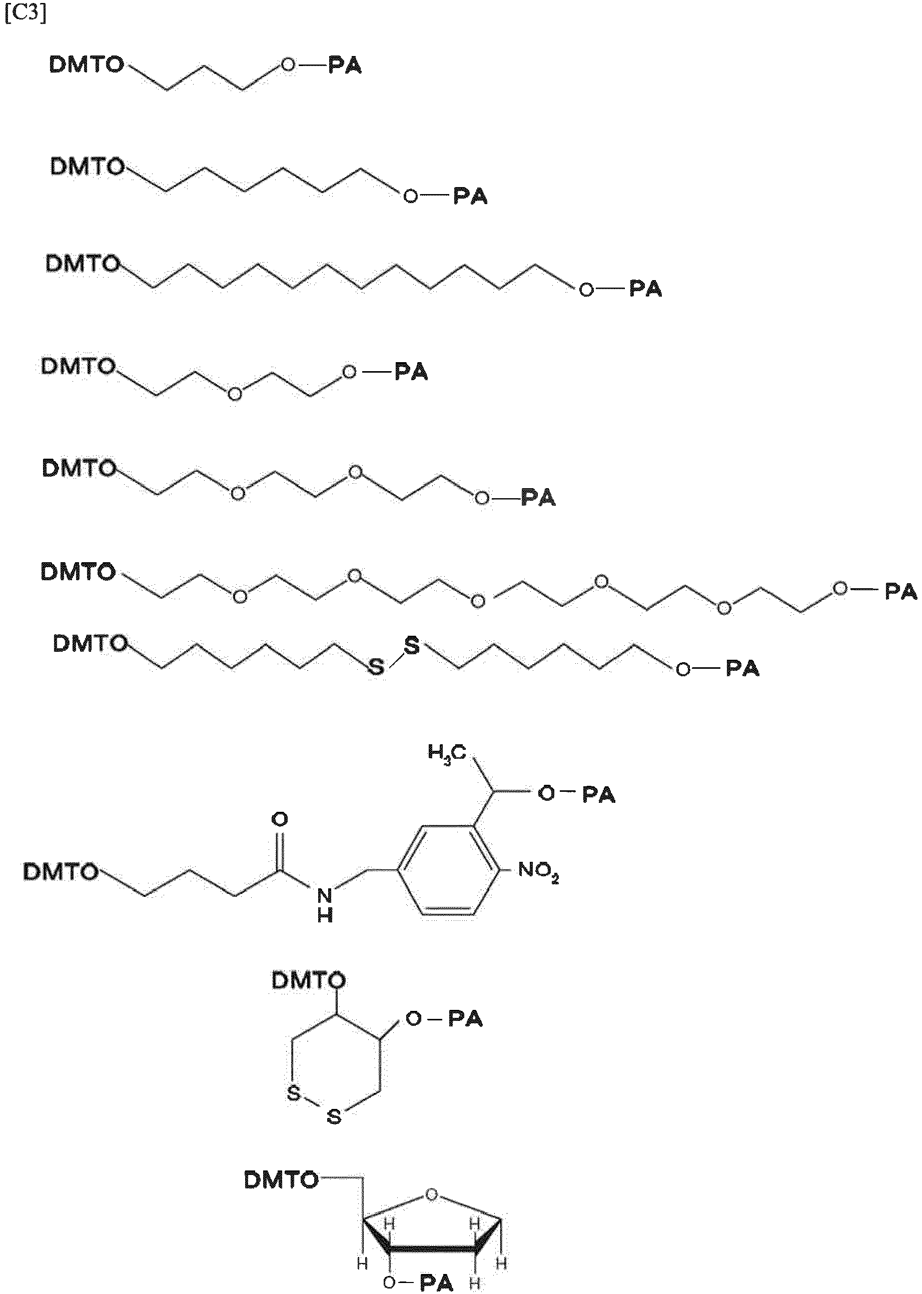

Nucleic acid amplification is performed using these primers. Various known methods may be applied to nucleic acid amplification as explained above, but various kinds of PCR such as multiplex PCR are typical. When performing the nucleic acid amplification step, the solution composition, temperature control and the like can be set appropriately by a person skilled in the art.

As explained above, when a first primer having a tag sequence, a linking site and a first recognition sequence in that order from the 5' end and a second primer having a label-binding region, a linking site and a second recognition sequence in that order from the 5' end are used to perform PCR on a sample that may contain a target nucleic acid, template strands derived from the first and second primers and containing those primers are formed by a DNA elongation reaction with a DNA polymerase as shown in (a) through (c) of **FIG.** 1B.

A DNA elongation reaction with a DNA polymerase is then performed again using a second primer and first primer different from the primers from which the template strands were derived. As shown in (d) and (e) of **FIG.** 1B, in a DNA elongation reaction using a DNA polymerase on template strands beginning with the second primer and including the first primer, DNA elongation is inhibited or arrested on the 5' side of the linking site derived from the first primer in the template strands, or in other words towards the 3' end from the linking site in the elongated strand.

As shown in (d) and (e) of **FIG.** 1B, moreover, in a DNA elongation reaction using a DNA polymerase on template strands beginning with the first primer and including the second primer, DNA elongation is inhibited or arrested on the 5' side of the linking site derived from the second primer in the template strands, or in other words towards the 3' end from the linking site in the elongated strand.

As shown in **FIG.** 2B, the resulting amplified fragment is a double-stranded DNA fragment having a single-stranded tag sequence and label-binding region protruding at either 5' end, and having a double-stranded part that includes the first recognition sequence and second recognition sequence. That is, in this double-stranded DNA fragment the tag sequence protrudes as a single strand on the 5' side of one DNA strand, and the labeling-binding region protrudes on the 5' side of the other DNA strand.

When the second primer used has a label bound in advance to the label-binding region as shown in **FIG.** 2A, this results in a double-stranded DNA fragment having the label at the 5' end of one DNA strand and the tag sequence protruding on the 5' side of the other DNA strand, together with a double strand that includes the first and second recognition sequences.

As shown in **FIG.** 2C, moreover, this results in a double-stranded DNA fragment having the label at the DNA strand elongation sites and a tag sequence protruding on the 5' side of one DNA strand, with the first and second recognition sequences included in a double strand.

### (Hybridization step)

Next, the hybridization step is performed as shown in **FIGS.** 2A to 2C. The hybridization step is a step of bringing the amplified fragment obtained in the amplification step into contact with a detection probe so that they can be hybridized by means of the tag sequence. In the hybridization step, as shown in **FIGS.** 2A to 2C, when the tag sequence of the double-stranded DNA fragment obtained in the amplification step and the recognition sequence of the detection probe on the solid phase body are sufficiently complementary to allow specific hybridization under fixed conditions, they hybridize to form a double strand on a specific detection probe on the solid phase body. A suitable washing step may be included after the hybridization step.

A double-stranded DNA fragment corresponding to the target nucleic acid specifically amplified in the amplification step is supplied to the hybridization step. This fragment has a tag sequence specific to the pre-associated detection probe protruding as a single strain. Therefore, it can react easily with the detection probe without being reduced to a single strand in a heat denaturing or other denaturing step following the amplification step. Hybridization efficiency is thereby improved, resulting in improved sensitivity and stability. Sensitivity is preferably improved 5-fold or more or more preferably 10-fold or more by providing a linking site in the first primer. Hybridization speed is also improved. It has been shown that hybridization time is reduced to about 1/10 by providing a linking site in the first primer.

As shown in **FIG.** 2A, when the double-stranded DNA fragment supplied to the hybridization step has a label-binding region provided with a direct label, there is no need for a special labeling step. As shown in **FIG.** 2C, the same applies when the double-stranded DNA fragment is provided with a label in the amplification step. Moreover, as shown in **FIG.** 2B, when the label-binding region contains a base sequence that binds with the labeling probe, this base sequence part protrudes as a single strand on the 5' end opposite the tag sequence. This allows for efficient hybridization with the labeling probe, and thus for rapid, easy and sensitive labeling. There is thus no need for a special labeling step, and the labeling probe is either supplied to the solid phase body at the same time as the double-stranded DNA fragment in the hybridization step, or may also be supplied to the solid phase body before or after the double-stranded DNA fragment (that is, either before or after hybridization).

The double-stranded DNA fragment only hybridizes with a specific detection probe based on its tag sequence. Because the tag sequence and the detection sequence of the detection probe are designed with a high degree of selectivity, mis-hybridization is strongly inhibited, and non-specific hybridization of double-stranded fragments with detection probes is strongly inhibited in the hybridization step.

The hybridization step is not limited to a mode of hybridization in **FIG.** 2 in which the hybridization solution is supplied to the entire solid phase body (immersion hybridization). For example, another option is a form of chromatography (developing hybridization) in which the hybridization solution is supplied as a mobile phase to part of the solid phase body, and expanded in a specific orientation relative to the solid phase body.

### (Detection step)

The detection step is a step of detecting the product of hybridization between the amplified fragment and the detection probe on the solid phase body.

The detection step is a step in which signal strength data is obtained for the target nucleic acid based on the label retained on the hybridization product on the solid phase body after hybridization, to thereby detect the hybridization product. Signal strength data can be obtained by detecting a label signal from the label. Because the location of the detection probe associated with the target nucleic acid on the solid phase body is already known, it is possible to assess the presence or absence and proportion of the target nucleic acid by detecting the label signal.

To obtain signal strength data, conventional known methods can be selected and applied appropriately according to the form of the solid phase body and the type of label. Typically, oligonucleotides and the like that have not hybridized are first removed from the solid phase body by a washing operation or the like, and the fluorescent signal from the added label is then detected with an array scanner or the like, or a chemoluminescence reaction is performed on the label. Detection methods using flow cytometry are applicable when beads are used as the carrier.

In this detection step, the presence or absence, proportion and the like of the target nucleic acid in the sample can be detected based on the signal strength data for the label. With this method, it is possible to reliably detect the target nucleic acid that is the object of detection even when detecting multiple target nucleic acids simultaneously. In this method, because the double-stranded DNA fragment obtained in the amplification step is adapted to efficient hybridization and efficient labeling, detection can be highly efficient and sensitive, and complex denaturing steps can be omitted.

In this detection method, amplified fragments corresponding to multiple target nucleic acids are preferably amplified from a sample by multiplex PCR, and detected at once on a solid phase body. That is, preferably nucleic acid amplification is performed using multiple primer sets each consisting of a first primer and a second primer, so as to allow detection by multiple detection probes pre-associated with multiple target nucleic acids, the multiple amplified fragments obtained in the amplification step are brought into contact with multiple detection probes on the solid phase body so that hybridization can occur, and the hybridization products of the multiple amplified fragments and multiple detection probes on the solid phase body are detected.

### (Nucleic acid amplification agent)

As shown by the first primer and the like in **FIG.** 1A, the nucleic acid amplification agent comprises an oligonucleotide derivative containing, in order from the 5' end, a first arbitrary base sequence and a first recognition sequence that recognizes a first base sequence in a nucleic acid to be amplified, and having a linking site capable of inhibited or arresting a DNA polymerase reaction disposed between the first base sequence and the first recognition sequence. Because this nucleic acid amplification agent has such a linking site, when the nucleic acid amplification agent is used as at least one primer or the like in a nucleic acid amplification method, and a DNA strand obtained by an amplification reaction and containing the nucleic acid amplification agent becomes a template strand, the linking site functions as an inhibition or arrest point for the DNA polymerase reaction in the elongated strand, so that the template strand no longer functions as such beyond the linking site. As a result, no elongated strand complementary to the template strand is formed beyond the linking site. The double-stranded DNA fragment obtained as a result is double-stranded DNA having the first arbitrary base sequence as a single strand at one 5' end as shown in **FIG.** 1A.

Like the first primer, as shown in **FIG.** 1B, the other primer (second primer) may also be an oligonucleotide derivative containing, in order from the 5' end, a second arbitrary base sequence and a second recognition sequence that recognizes a second base sequence in a nucleic acid to be amplified, and also having a linking site capable of inhibited or arresting a DNA polymerase reaction, disposed between the second base sequence and the second recognition sequence. As shown in **FIG.** 1B, this results in double-stranded DNA having the first arbitrary base sequence and second arbitrary base sequence as single strands at either 5' end, respectively.

Double-stranded DNA having such protruding single strands is used for various applications including hybridization. The nucleic acid amplification agent can typically be used as a primer in various nucleic acid amplification methods.

The first arbitrary base sequence and/or second arbitrary base sequence may be the tag sequence of the disclosure or may be a base sequence that has a bound label or is capable of hybridizing with a labeling probe. When the first arbitrary base sequence is associated with a label in this way, it can simultaneously amplify and label a target nucleic acid.

The various embodiments of linking sites explained above with respect to the detection method can also be applied to the linking site in the nucleic acid amplification agent. Moreover, various embodiments of the tag sequence and first recognition sequence and the label-binding region and second recognition sequence in the first primer and second primer as explained above with reference to the present detection method may also be applied to the first arbitrary base sequence and first recognition sequence of the nucleic acid amplification site. That is, the nucleic acid amplification agent includes the first primer and second primer as embodiments.

The present disclosure also provides a kit including one or two or more nucleic acid amplification agents. This kit may also contain a solid phase body for hybridizing with DNA fragments obtained using the first primer and second primer.

The disclosure also provides a double-stranded DNA fragment obtained in the detection method, or in other words a double-stranded DNA fragment having a single-stranded part on the 5' side of at least one strand together with a double-stranded part formed by base pairing, wherein at least one of the DNA strands has a linking part capable of inhibiting or arresting a DNA polymerase reaction, disposed between the single-stranded part and the double-stranded binding part, and the single-stranded part has a tag sequence complementary to the base sequence of the detection probe. It also provides a double-stranded DNA fragment also having a single-stranded part on the 5' side of the other strand, and having a label linked to this single-stranded part. Because such double-stranded DNA fragments are suited to probe hybridization, moreover, the disclosure also provides a probe hybridization composition containing these. The composition is used in the above method.

The present disclosure also provides a method for amplifying a target nucleic acid in a sample. That is, it provides a method comprising a step of subjecting the sample to nucleic acid amplification using at least a first primer comprising a first arbitrary base sequence and a first recognition sequence that recognizes a first base sequence in the target nucleic acid, with a linking site capable of inhibiting or arresting a DNA polymerase reaction disposed between the first arbitrary base sequence and the first recognition site. As shown in FIG. 1A, the amplified fragment obtained by this method is a double-stranded DNA fragment having a first arbitrary base sequence protruding as a single strand at the 5' end of at least one strand. In this amplification method, moreover, it is possible to use as another primer a second primer containing a second arbitrary base sequence and a second recognition sequence that recognizes a second base sequence in the target nucleic acid, with a linking site capable of inhibiting or arresting a DNA polymerase reaction disposed between the second arbitrary base sequence and the second recognition site. In this case, as shown in **FIG.** 1B, it is possible to obtain a double-stranded DNA fragment having single-stranded first arbitrary base sequences protruding at the 5' ends of both strands. In the first primer, the first arbitrary base sequence may be provided with a label, or with a base sequence capable of binding with a labeling probe. The same applies to the second primer.

The various embodiments of the detection method explained above may also be applied to the first primer, second primer and linking sites in this amplification method.

This amplification method may also be provided as a method of producing a double-stranded DNA fragment provided with a single strand at the 5' end of at least one DNA chain. The amplification method may also be implemented as a target nucleic acid labeling method. It may also be implemented as a nucleic acid detection method having such a labeling step. That is, by using this amplification step (labeling step) in place of the labeling steps of the SNP and other detection methods disclosed in Japanese Patent Application Publication No. 2008-306941, Japanese Patent Application Publication No. 2009-24 and Analytical Biochemistry 364 (2007), 78-85, it is possible to eliminate the subsequent denaturing step and achieve highly efficient and sensitive hybridization.

### (Method of detecting target nucleic acid by nucleic acid chromatography)

The method of detecting a target nucleic acid by nucleic acid chromatography (hereunder sometimes called simply the detection method) disclosed in this Description comprises a hybridization step in which a partially double-stranded nucleic acid is brought into contact with a probe on a solid phase body carrier under conditions that allow hybridization by nucleic acid chromatography, and a step of detecting the hybridization product produced in the hybridization step. The partially double-stranded nucleic acid shown in **FIG.** 1A is explained first below, and the hybridization step and detection step are explained next.

### (Partially double-stranded nucleic acid)

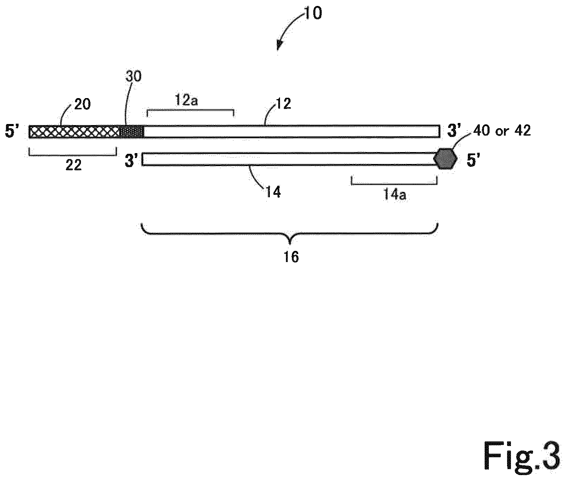

As shown in Detail in **FIG.** 3, the partially double-stranded nucleic acid 10 in this Description is a nucleic acid comprising a first strand 12 and a second strand 14, with a single-stranded part 20 at the 5' end of the first strand 12, wherein the part other than the single-stranded part 20 is a double-stranded part 16 formed by hydrogen bonds. The single-stranded part 20 (or in other words tag 20) of the first strand 12 of the partially double-stranded nucleic acid 10 in this Description is in the form of a dangling strand protruding from the double-stranded part 16.

The double-stranded part 16 of the partially double-stranded nucleic acid 10, or in other words the double-stranded part 16 formed by base pairing between the first strand 12 and the second strand 14, preferably has a structure comprising nucleotides provided with natural bases (adenine, guanine, thymine and cytosine) that can provide a substrate for DNA strand elongation reaction by a DNA polymerase, linked to each other by phosphate diester bonds. That is, the double-stranded part 16 preferably has a natural ribose-phosphate backbone, and also has natural nucleic acids with natural bases, and more preferably consists solely of natural nucleic acids. As discussed below, it is preferably synthesized by the nucleic acid amplification reaction used to obtain the partially double-stranded nucleic acid 10 associated with the target nucleic acid.

The partially double-stranded nucleic acid 10 is associated with a target nucleic acid. "Associated with a target nucleic acid" here means that it has a double-stranded part 16 containing at least part of the double-stranded part of the target nucleic acid. This double-stranded part 16 can be obtained for example by amplification with a primer set that hybridizes specifically with part of a target sequence in the target nucleic acid.

### (Tag part)

The same configuration used for the double-stranded part 16 can be adopted for the tag part 20 of the partially double-stranded nucleic acid 10 so that it can be synthesized by a nucleic acid amplification reaction. That is, the tag part 20 may have a structure comprising nucleotides having natural bases capable of providing a template for a DNA strand elongation reaction by a DNA polymerase, linked to each other by phosphate diester bonds, and may contain natural nucleic acids or consist solely of natural nucleic acids. The tag part 20 may also be an oligonucleotide chain formed by non-natural synthesis, without a nucleic acid amplification reaction. Either a natural ribose-phosphate backbone or a PNA (peptide nucleic acid) backbone, BNA (bridged nucleic acid) backbone or other known artificial backbone may be adopted as the backbone of this nucleotide chain. For the bases, since they need only hybridize specifically with the probe, moreover, it is possible to include non-natural L-DNA or the like as long as this is matched to the probe, or the bases may consist solely of L-DNA. Non-natural bases may also be included, or the bases may consist solely of non-natural bases. Although the tag is associated with the target nucleic acid, it need not be synthesized by a nucleic acid amplification reaction, unlike the double-stranded part 16. Thus, the tag part 20 can be derived from the primer in the nucleic acid amplification reaction.

The tag part 20 of the partially double-stranded nucleic acid 10 has a tag sequence capable of hybridizing specifically with a probe provided on a solid phase body carrier. Tag sequence 22 is a sequence that allows hybridization with a probe, and detects a target nucleic acid. Therefore, it is designed so as to hybridize with the detection sequence of the probe for each target nucleic acid. Typically, it is a base sequence complementary to the detection sequence. As a result, one probe is associated with one target nucleic acid. The base length of the tag sequence 22 matches the base length of the detection sequence of the probe, and is preferably 20 to 50 bases, or more preferably 20 to 25 bases.

### (Linking site)

A linking site 30 capable of inhibiting or arresting a DNA strand elongation reaction by a DNA polymerase is preferably provided between the tag part 20 of the first strand 12 and the part corresponding to the double-stranded part 16 of the partially double-stranded nucleic acid 10. As discussed above, providing the linking site 30 makes it possible to synthesize a partially double-stranded nucleic acid 10 with a tag part 20 by a nucleic acid amplification reaction. Providing such a linking site 30 also improves the autonomous mobility of the tag part 20, thereby allowing more efficient hybridization with the probe.

Other examples of the linking site 30 include nucleic acid sequences having three-dimensional structures that inhibit the progress of the polymerase, such as strong hairpin structures and pseudoknot structures, as well as L-shaped nucleic acids, artificial nucleic acids and other target nucleic acid natural nucleic acids, and RNA, aliphatic chains and other non-nucleic acid structures. Examples of artificial nucleic acids include peptide nucleic acids, bridged nucleic acids, azobenzenes and the like.

### (Label)

The partially double-stranded nucleic acid 10 is provided with a label 40 or a label binding substance 42 as explained above. In this detection method, the partially double-stranded nucleic acid 10 is used in nucleic acid chromatography, and is provided only with a tag part 20 for hybridizing with the probe, but not with a single strand for the label 40.

The label 40 or label binding substance 42 can be provided in any part of the partially double-stranded nucleic acid 10. For example, as shown in **FIG.** 3, it is provided at the 5' end of one strand. It can also be provided on all or part of the first strand 12 and second strand 14. The label 40 or label binding substance 42 is normally incorporated into the partially double-stranded nucleic acid 10 in the nucleic acid amplification reaction of the partially double-stranded nucleic acid 10.

### (Amplification step)

The partially double-stranded nucleic acid 10 supplied to the hybridization step is preferably obtained by a nucleic acid amplification reaction. One example of a nucleic acid amplification step for obtaining the partially double-stranded nucleic acid 10 is explained below.

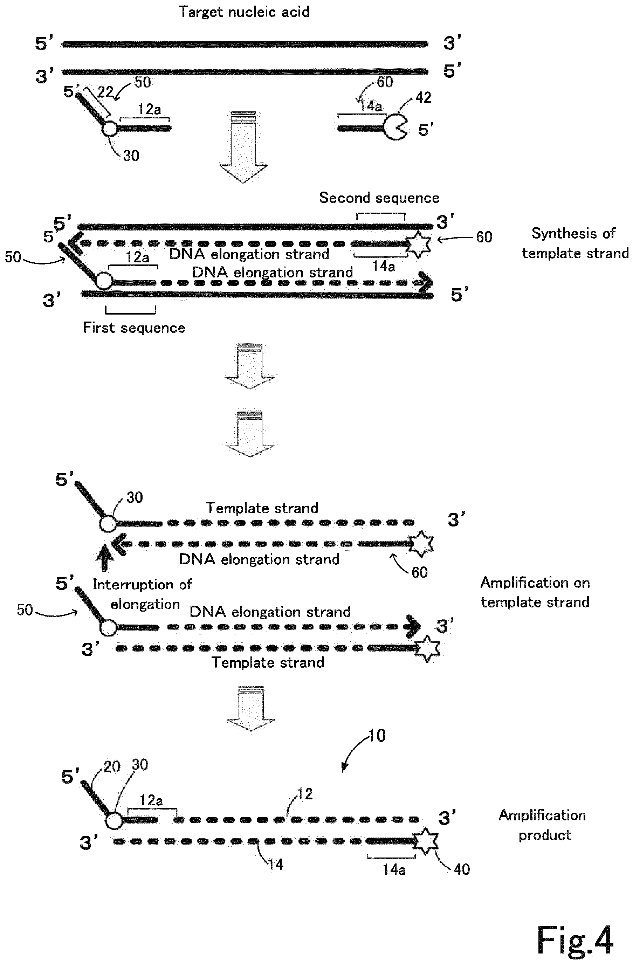

As shown in **FIG.** 4, the amplification step for obtaining the partially double-stranded nucleic acid 10 is performed using a first primer 50 and a second primer 60. The nucleic acid amplification method in the nucleic acid amplification step may be a known method such as PCR, in which a double-stranded DNA fragment is obtained by amplifying DNA in a DNA polymerase reaction.

### (First primer)

The first primer 50 is a primer for obtaining the first strand 12 of the partially double-stranded nucleic acid 10. As discussed above, the first primer 50 contains a tag sequence 22 complementary to a probe pre-associated with the target nucleic acid, and a first recognition sequence 12a that recognizes a first base sequence in the target nucleic acid. The tag sequence 22 corresponds to the tag sequence 22 of the tag part 20 of the partially double-stranded nucleic acid 10. The first primer 50 has a linking site 30 between the first recognition sequence 12a and the tag sequence 22. The linking site 30 is as explained above.

The first primer 50 preferably has the tag sequence 22, linking site 30 and first recognition sequence 12a in that order from the 5' end. In this way, as shown in **FIG.** 4, a second strand 14 is obtained having as its 3' end a base that pairs with a base of a nucleotide adjacent to the 3' side of the linking site 30 from the first primer 50, or with a base adjacent to that base, and lacking a complementary strand to the tag sequence 22 of the first primer 50.

### (Second primer)

The second primer 60 is a primer for obtaining the second strand 14 of the partially double-stranded nucleic acid 10. As shown in **FIG.** 4, the second primer 60 contains a second recognition sequence 14a for recognizing a second base sequence in the target nucleic acid as discussed above.

As shown in **FIG.** 4, the second primer 60 can be provided in advance with a label 40. The purpose of the label 40 is to detect the partially double-stranded nucleic acid 10 bound to the probe on the solid phase body carrier. A conventionally known substance may be selected appropriately and used as the label 40. The label 40 is preferably provided at the 5' end of the second primer 60.

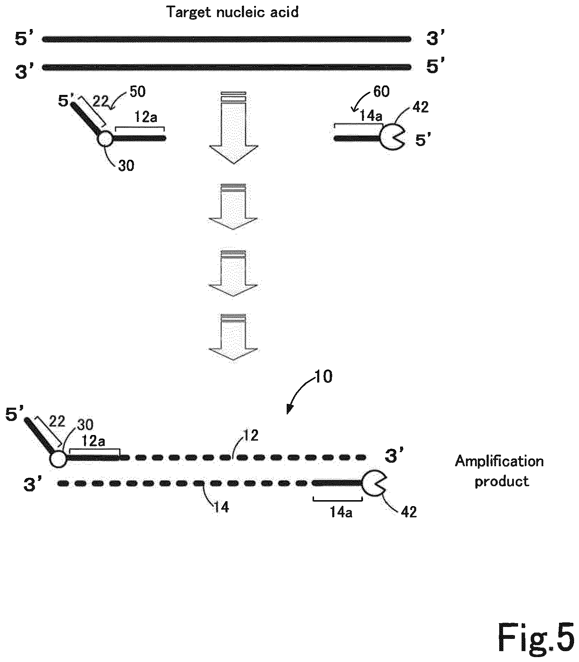

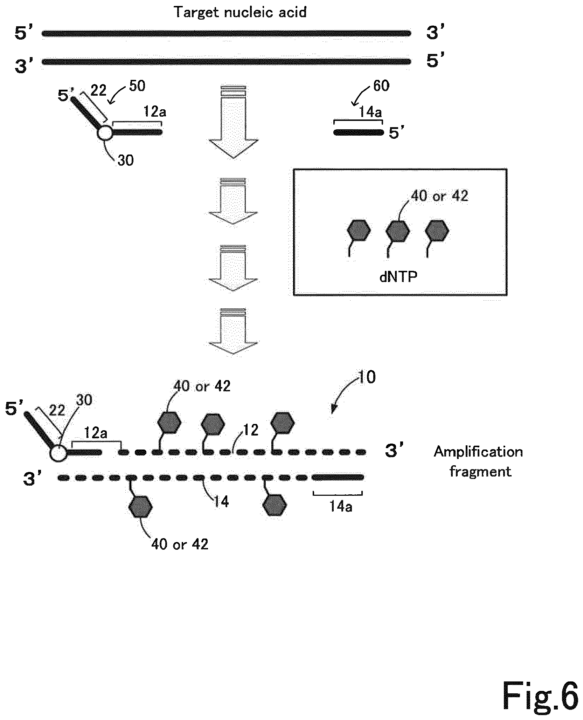

The second primer 60 may also be provided with a label binding substance 42 as shown in **FIG.** 5. The label binding substance 42 is preferably provided at the 5' end of the second primer 60. Moreover, as shown in **FIG.** 6, the second primer 60 may have neither a label 40 nor a label binding substance 42. This is because a partially double-stranded nucleic acid 10 labeled with an introduced label 40 or label binding substance 42 at the DNA elongation sites can be obtained in the amplification step by performing a nucleic acid amplification reaction with a nucleoside triphosphate composition containing a nucleoside derivative triphosphate provided with the label 40 or label binding substance 42.

The second primer 60 preferably has the label 40 or label binding substance 42 and second recognition sequence 14a in that order from the 5' end. In this way, it is possible to obtain a second strand 14 having the label 40 or label binding substance 42 at the 5' end **(****FIG.** 4, **FIG.** 5).

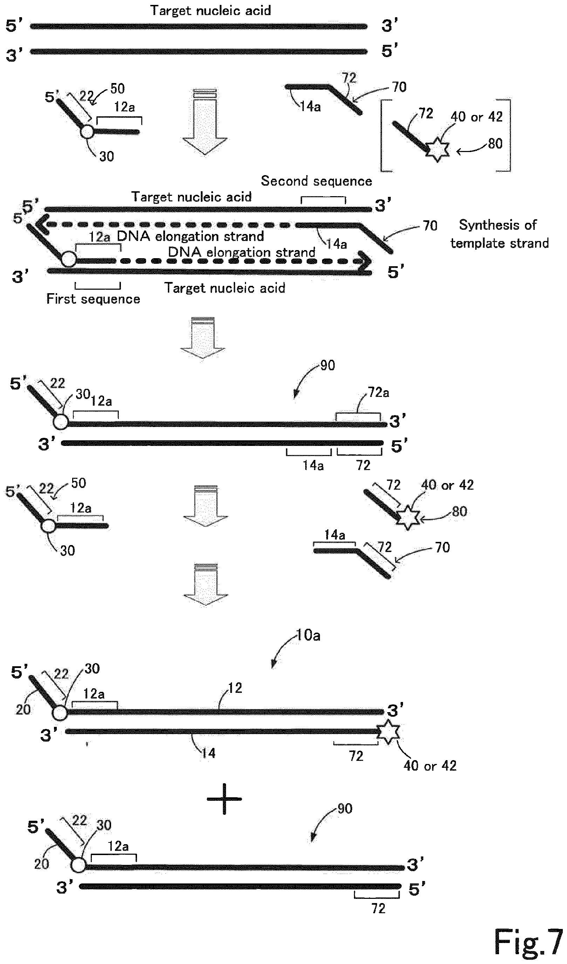

Moreover, a partially double-stranded nucleic acid 10 of the form shown in **FIG.** 4 and **FIG.** 5 (hereunder, a partially double-stranded nucleic acid of this type is called partially double-stranded nucleic acid 10a) can be obtained by performing an amplification step using the first primer explained above and the two kinds of second primers I and II explained below. The primers and amplification steps used are shown in **FIG.** 7.

### (Second primer I)

As shown in **FIG.** 7, the second primer I 70 comprises a labeling sequence 72 and a second recognition sequence 14a that recognizes the second base sequence. The second recognition sequence 14a is as explained previously. The labeling sequence 72 is for introducing a new base sequence for labeling purposes into the amplification product obtained with the second primer I 70, and can be designed without reference to the base sequence of the target nucleic acid or detection probe. It also does not need to be associated. Thus, the labeling sequence 72 does not need to be different for each target nucleic acid, and a common base sequence can be used for all target nucleic acids, but when two or more labels or the like are used, different base sequences may be used for the different labels. That is, the labeling sequence 72 may be standardized rather than being unique for each target nucleic acid. It may also be a completely artificial base sequence designed to optimize reactivity in the amplification reaction for example. The second primer I 70 preferable comprises the labeling sequence 72 and second recognition sequence in that order from the 5' end.

### (Second primer II)

The second primer II 80 contains the label 40 or label binding substance 42 and a labeling sequence 72. The label 40 or label binding substance 42 is as explained above. The label 40 or label binding substance 42 is linked to the 5' end of the second primer II. The labeling sequence 72 is as explained with respect to the second primer I. In the second primer II 80, the base sequence of the second primer II 80 with the bound label or the like is a common sequence. It is thus possible to provide the second primer II 80 with the bound label inexpensively.

As shown in **FIG.** 7, a partially double-stranded nucleic acid 90 having the tag sequence 22 as a single-stranded part and having the labeling sequence 72 together with its complementary sequence 72a is obtained as an amplification product by performing a nucleic acid amplification reaction on a target nucleic acid with the first primer 50 and second primer I 70. Once this partially double-stranded nucleic acid 90 has been obtained, the second primer II 80 hybridizes with one strand of the partially double-stranded nucleic acid 90 (the strand having the sequence 72a complementary to the labeling sequence 72) in place of the second primer I 70, producing a nucleic acid amplification reaction. As a result, a partially double-stranded nucleic acid 10a having a label or the like is produced as shown in **FIG.** 7. If the second primer I 70 is present in the amplification reaction system, the second primer I 70 also acts on the partially double-stranded nucleic acid 90, and the partially double-stranded nucleic acid 90 is also synthesized.

Using the second primer I 70 and the second primer II 80 in place of the second primer 60 produces an efficient nucleic acid amplification reaction, resulting in good detection sensitivity. That is, depending on the base sequence of the target nucleic acid, in some cases the matching between the second primer 60 and target nucleic acid may be poor and the nucleic acid amplification reaction may not progress well, resulting in poor detection sensitivity. Even in such cases, if the partially double-stranded nucleic acid 90 can once be obtained with the primer set consisting of the first primer 50 and second primer I 70, the second primer II 80 then acts on the strand having the strand complementary to the labeling sequence 72 in the partially double-stranded nucleic acid 100, and an efficient nucleic acid amplification reaction can be achieved with the first primer 50 and second primer II 80.

The order in which the first primer 60, second primer I 70 and second primer II 80 are supplied can be determined appropriately. For example, they may be supplied simultaneously to act on the target nucleic acid in the nucleic acid amplification reaction system, or for example the first primer 60 and second primer I 70 can be supplied first, followed by the second primer II 80. They may also be supplied simultaneously to the nucleic acid amplification reaction system.

By including the second primer II 80 in excess over the second primer I 70, it is possible to synthesize a partially double-stranded nucleic acid 110 with high efficiency. For example, the second primer II 80 is supplied to the reaction system in the amount of 1 to 10 times or preferably 1 to 5 times the amount of the second primer I.

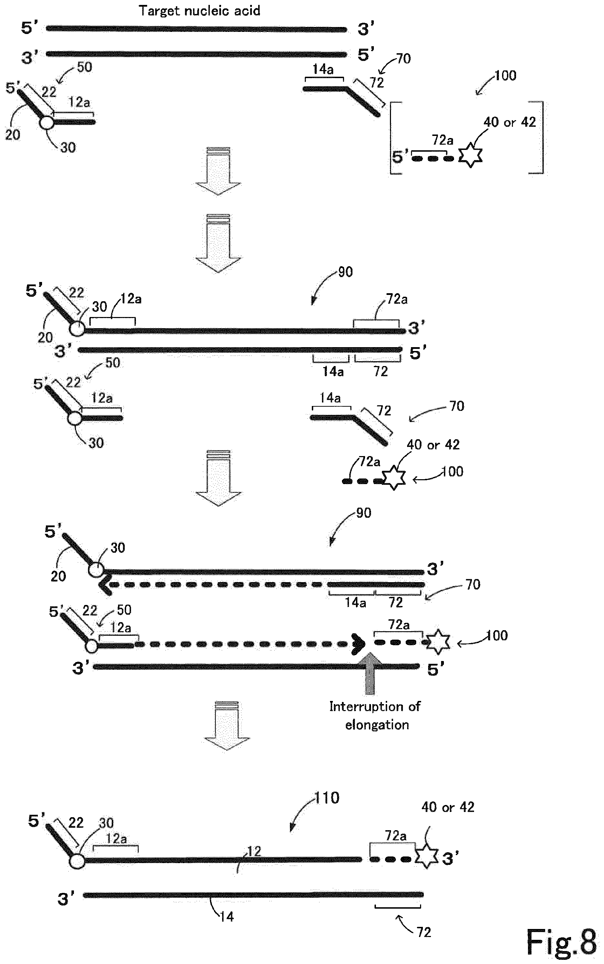

Moreover, as shown in **FIG.** 8, an equivalent of the partially double-stranded nucleic acid 10a can be obtained by using a labeling probe 100 provided with the label 40 or label binding substance 42 shown below, without using the second primer II 80. That is, it can also be obtained by performing a nucleic acid amplification reaction on the target nucleic acid using the first primer 50 and second primer I 70 in the presence of the labeling probe 100. The primers and probes used and the amplification steps are shown in **FIG.** 8.

### (Labeling probe)

As shown in **FIG.** 8, the labeling probe 120 is provided with a complementary strand 72a capable of hybridizing specifically with the labeling sequence 72 of the second primer I 70, and also with a label 40 or label binding substance 42 at the 3' end. Labeling sequence 72 itself is not specific or associated with the target nucleic acid, and since it can be a common sequence irrespective of the target nucleic acid, complementary sequence 72a is also made a common sequence for the same reasons. Thus, the labeling probe 100 can also be supplied inexpensively as in the case of the second primer II 80.

As shown in **FIG.** 8, an amplification step is performed on the target nucleic acid using the first primer 50 and second primer I 70 in the presence of the labeling probe 100. As in **FIG.** 7, a partially double-stranded nucleic acid 90 is synthesized having the tag sequence 22 as a single-stranded part, and having the complementary sequence 72a at the 3' end of the DNA strand that includes the tag sequence 22. In the subsequent amplification reaction, the first primer 50 acts on the single strand that serves as the template (having the labeling sequence 72 at the 5' end), elongating the DNA. During this DNA elongation reaction, the labeling probe 100 hybridizes with the labeling sequence 72 at the 5' end of the template strand via the complementary sequence 72a in the same way that the first probe 50 hybridizes with the template strand. As a result, in the DNA elongation reaction, the DNA polymerase reaction is inhibited or arrested at the 5' end of the template strand, or in other words at the site of hybridization of the labeling probe 100, and DNA elongation is inhibited or arrested.

Thus, as shown in **FIG.** 8, a partially double-stranded nucleic acid 92 is synthesized having the tag sequence 22 as a single strand at the 5' end of one strand, and the labeling sequence 72 as a single strand at the 5' end of the other strand. At the same time, the labeling probe 100 hybridizes with the labeling sequence 72 in the partially double-stranded nucleic acid 92. As a result, the amplification product obtained from the nucleic acid amplification reaction becomes composite 110 comprising labeling probe 100 hybridized with the partially double-stranded nucleic acid 92. The structure of this composite 110 as an amplification product differs from that of the partially double-stranded nucleic acid 10a, but retains the label 40 or label binding substance 42 at the other end of the single-stranded part having the tag sequence 22. It thus functions in the same way as the partially double-stranded nucleic acid 10a in subsequent hybridization.

The amplification product can be labeled efficiently if the labeling probe 100 is used in place of the second primer II 80. Detection sensitivity is improved as a result.

The DNA polymerase used in the amplification step shown in **FIG.** 8 is preferably one in which 3'-5' exonuclease activity is suppressed or lacking. This serves to avoid or inhibit decomposition of the labeling probe 100.

The amplification step is performed with these primers. As explained previously, various known methods may be applied as the nucleic acid amplification method, but typically PCR, multiplex PCR or another PCR method is used. The solution composition, temperature control and the like for performing the nucleic acid amplification step can be set appropriately by a person skilled in the art.

### (Hybridization step)

The hybridization step is a step of bringing the partially double-stranded nucleic acid 10 (including 10a) or its equivalent, the composite 110 (hereunder these are called the partially double-stranded nucleic acid and the like) into contact with the probe 220 on the solid phase body carrier 210, under conditions that allow hybridization by nucleic acid chromatography. The chromatography unit 200 used in the hybridization step, which is a solid phase body comprising the solid phase body carrier 210 with the probe 220 fixed on the solid phase body carrier 210, is explained first.

### (Chromatography unit)

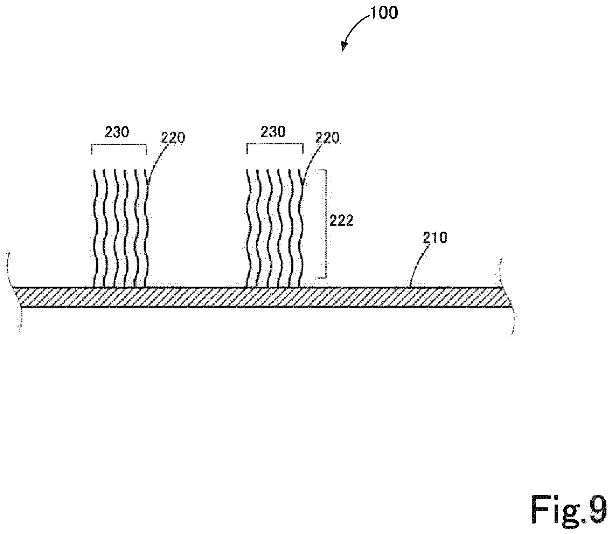

As shown in **FIG.** 9, chromatography unit 200 is provided with the solid phase body carrier 210 and one or two or more probes 220 fixed to the solid phase body carrier 210. The solid phase body carrier 210 is not particularly limited, and a known convention carrier on which a liquid can be moved by capillary action may be adopted. For example, a porous material consisting principally of a polymer such as polyethersulfone, nitrocellulose, nylon, vinylidene polyfluoride or the like can be used for the solid phase body carrier 210. A cellulose material such as filter paper may also be used by preference. The chromatography unit 200 does not necessarily have to be configured with a single solid phase body carrier 210. Multiple solid phase body carriers 210 can be linked as long as the whole unit can move the developing medium by capillary action. The overall form of the chromatography unit 200 is not particularly limited. It may be in the form of a sheet, thin bar, or other form that allows for developing and dispersal of the chromatography solution by capillary action. Preferably it has a long, thin form, one end of which in the lengthwise direction is brought into contact with the developing medium for chromatography.

Probe 220 includes a detection sequence 222 capable of hybridizing specifically with the tag sequence 22 of a partially double-stranded nucleic acid or the like associated with the target nucleic acid. The detection sequence 222 preferably has a base sequence that is complementary to, and preferably entirely complementary to, the tag sequence 22 of the partially double-stranded nucleic acid or the like.

Because the detection sequence 222 need only be capable of hybridizing specifically with the tag sequence 22 attached to the partially double-stranded nucleic acid or the like, it can be designed independently of the target nucleic acid.

The length of the detection sequence 222 is not particularly limited. For example, it may be about 20 to 50 bases long. This is because within this range, hybridization efficiency can generally be secured while ensuring the specificity of each detection sequence. For example, a detection sequence of this base length may be a 46-base sequence obtained by combining two 23-base sequences selected from the base sequences of SEQ ID NOS:1 to 100 below and their complementary sequences, or a base sequence obtained by addition, deletion or the like of suitable bases in these combined base sequences. More preferably, it is 20 to 25 bases long. For example, a detection sequence of this base length may be one of the 23-base sequences of SEQ ID NOS:1 to 100 below or a complementary sequence thereof, or a sequence obtained by addition, deletion or the like of suitable bases in these base sequences.

Because the tag sequence 22 in the first primer 50 and partially double-stranded nucleic acid or the like is paired with the detection sequence 122, the tag sequence 22 preferably has the same base length as the detection sequence.

One or two or more probes 220 are fixed to a single solid phase body carrier 210. The mode of fixing is not particularly limited. A known fixing method may be used. This be accomplished by Apart static interaction between the probe 220 and the surface of the solid phase body carrier 210 for example, or by covalent binding or the like between functional groups within the material of the solid phase body carrier (including pre-existing functional groups and functional groups added for fixing purposes) and functional groups within the probe 220.

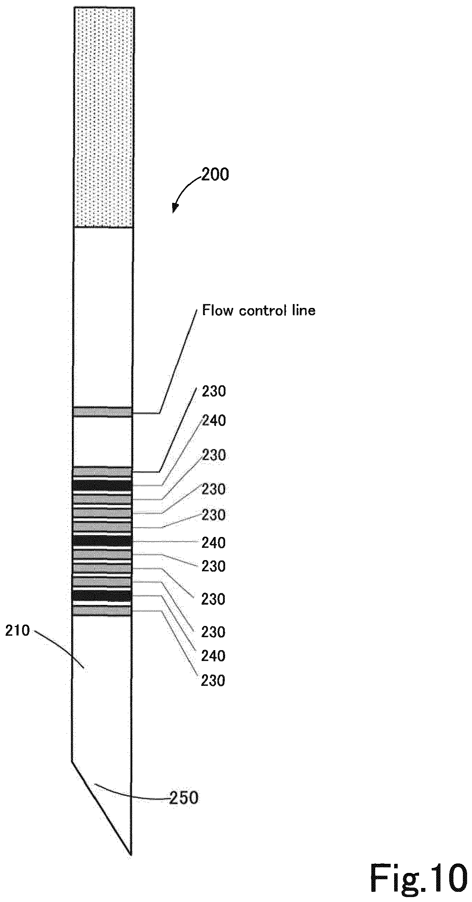

As shown in FIG. 10, the regions (probe regions) 230 on the solid phase body carrier 210 with the probes 220 fixed thereto are formed in an arbitrary pattern. The probe regions 230 may have any configuration, for example, these regions may have a dot shape or line shape or may be formed in other configurations. Typically, multiple probe regions 230 are arranged at suitable intervals in the developing direction, with each region formed as a line perpendicular to the developing direction of the chromatography developing medium. Preferably one probe region 230 corresponds to one kind of probe.

As shown in **FIG.** 10, when probe regions 230 are provided for 3 or more probes 220, the three or more probe regions 230 may also be formed as parallel lines. In this case, the intervals between the three or more probe regions 230 are determined appropriately. For example, if there are 7 probe regions, they can be divided into a group of 2 probe regions 230 furthest upstream in the developing direction, a group of 3 probe regions 230 downstream, and a group of 2 probe regions 230 further downstream. In this case, specific intervals can be set between individual probe regions 230 within each group of multiple regions 230 in each part of the solid phase body carrier 210. For example, the intervals can be set to be the same.

As shown in **FIG.** 10, the solid phase body carrier 210 can also be provided with one or two or more position marker regions 240. The position marker regions 240 are preferably designed as regions that can be detected visually in the hybridization and detection steps. Typically, they are composed of a pigment or dye that is insoluble in the developing medium. They are also preferably formed as lines perpendicular to the developing direction of the developing medium. Moreover, the position marker regions 240 may themselves be composed of a combination of one or two or more selected from the group consisting of letters, symbols, numbers and graphics. Also, the coloration of the position marker regions 240 is not particularly limited. Multiple position marker regions 240 may be of the same color, or may be assigned different colors. Preferably, they are of a hue different from the coloration of the probe regions 230.

The locations of the position marker regions 240 are determined appropriately, but preferably there are two or more. For example, as shown in **FIG.** 10, a suitable number of probe regions 230, such as 2, 3 or 4 or more, are preferably provided between two position marker regions 240. This makes it easier to identify multiple probe regions 230 without errors. 1, 2 or 3 or more probe regions 230 may also be provided upstream from the upstream position marker region 240, and 1, 2, or 3 or more probe regions 230 may also be provided downstream from the downstream position marker region 240. This makes it easier to effectively identify multiple probe regions 230 using two position marker regions 240.

When the total of the 1 or 2 or more probe regions 230 and position marker regions 240 arranged on the solid phase body carrier 210 is 3 or more, they intervals between them may be equal. Providing equal intervals makes it easier to specify the positions of the probe regions 230. It is particularly appropriate to have three probe regions 230 between two position markers 240. It is thus possible to determine at a glance whether a colored probe region 230 is closest to one or the other of two position marker regions 240, or whether it is located exactly between the two position marker regions. When there are 5 probe regions 230, and the two additional probe regions 230 are arranged outside the respective position markers, up to 5 locations can be easily distinguished by determining at a glance whether a probe region is outside the position markers 240 rather in the 3 locations. From this perspective, the number of easily distinguishable probe regions 230 can be increased by increasing the number of position marker regions 240.

As shown in **FIG.** 10, the chromatography unit 200 can be provided with a liquid contact part 250 for bringing one end of the unit into contact with a developing medium for nucleic acid chromatography. The chromatography unit 100 is preferably in the form of a long thin strip, and one end in the lengthwise direction is the liquid contact part 250. Such a liquid contact part 250 is particularly desirable when the developing medium is disposed at the bottom of the chromatography unit 200 and the developing medium is moved upward towards the top of the unit.

The form of the liquid contact part 250 is not particularly limited as long as it can be immersed in the developing medium. For example, it may have a form that tapers towards the tip. This makes it possible to immerse or bring the liquid contact part 250 of the chromatography unit 200 into contact with an developing medium supplied to a tube for testing purposes. Conversely, when the aim is to increase the developing speed, the liquid contact part 250 may be given a larger area or capacity than other areas. For example, when a bar shape is adopted for the chromatography unit 200, it may have a tapered liquid contact part 250 that is wider towards the bottom than the bar-shaped part.

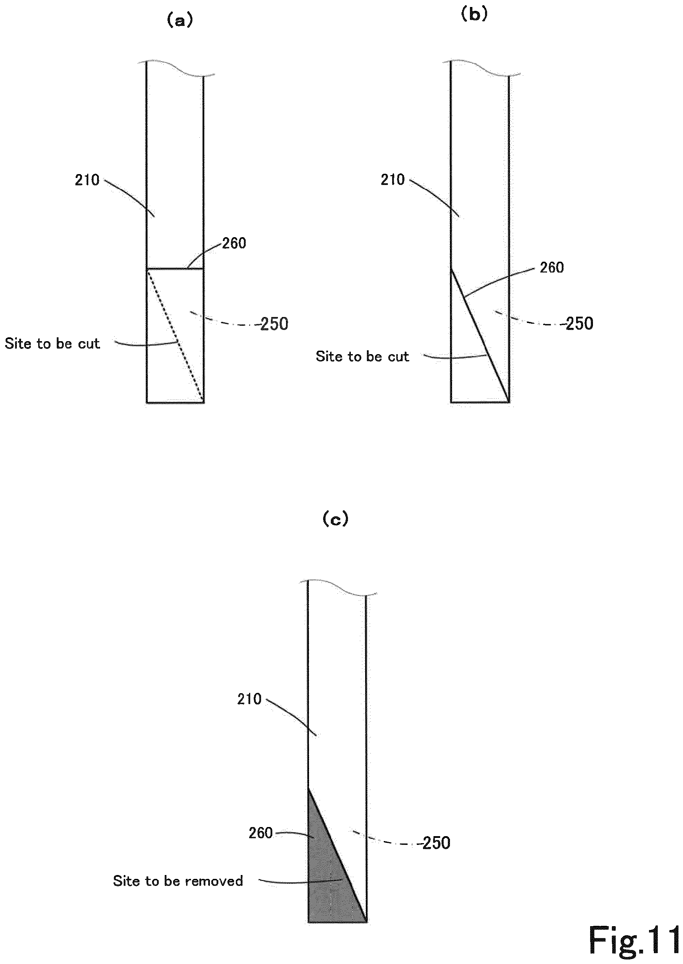

The liquid contact part 250 may have a specific shape in advance, but the liquid contact part 250 may also be formed by making the tip of the chromatography unit 200 into a specific shape at the time of contact with the developing medium. For example, as shown in **FIG.** 11A to **FIG.** 11C, a liquid contact part-forming marker 260 may be provided for forming the liquid contact part 250 with a specific shape. The liquid contact part-forming marker 260 may be a marker that shows a cutting site for cutting the chromatography unit 200 with scissors or the like to form a liquid contact part 250 with a specific shape. For example, as shown in **FIG.** 11A, a cutting start point and cutting end point may be shown with visible lines. Alternatively, as shown in **FIG** 11B, the cutting line itself may be shown with a visible line. Furthermore, as shown in **FIG** 11C, the part to be removed may also be indicated. It is also possible to not show the cut at all, or to show the part to be left.

The liquid contact part-forming marker 260 may also be weak so as to allow the chromatography unit 200 to be cut to form the liquid contact part 250. "Weak" here means weakness or fragility of the chromatography unit 200 sufficient to guide or promote cutting or removal with hands or fingers. Weakness may mean chemical weakness or the like, or physical weakness or the like. For example, the liquid contact part-forming marker 260 may be formed by providing the intended cutting site with perforations, or reducing the thickness of the intended cutting site to facilitate concentration of stress at the intended cutting site. Providing marker 260 is a way of allowing the chromatography unit 200 to be cut along the marker 260 so that the liquid contact part 250 can be formed easily with a specific shape.

The chromatography unit 200 may also be provided with a separate marker region or the like for indicating when the developing medium has expanded sufficiently. It may also be provided with a holding part for the label 40 for purposes of binding the label 40 to the label binding substance 42 when the partially double-stranded nucleic acid or the like has a label binding substance 42. Like the probe region and the like, these sites are all formed from porous materials that are themselves capable of moving the developing medium, and are configured so as not to inhibit continuous developing of the developing medium at each site. A water-absorbing part for absorbing the developing medium after completion of developing may also be provided downstream from the probe region.

When a partially double-stranded nucleic acid or the like is obtained as the amplification product of an amplification step prior to the hybridization step, a developing medium can be prepared that comprises an amplification reaction solution containing this amplification product. When the partially double-stranded nucleic acid or the like has a label 40, there is no need to add a separate label 40 to the developing medium.

The chromatography unit 200 explained above may also be provided as a kit together with a primer set matched to the probe 220 of the solid phase body carrier 210. A reagent for the label 40 may also be included in the kit. For example, a primer having the label 40 may be included in the primer set, or dNTP provided with the label 40 or label binding substance 42 used in the nucleic acid amplification reaction may also be included. A label 40 for binding to a label binding substance 42 may also be included.

### (Developing medium)

The developing medium is a liquid that is dispersed and moved by capillary action in the solid phase body carrier 110 of the chromatography unit 100, and is a medium for moving the partially double-stranded nucleic acid 10 on the solid phase body carrier 110. The developing medium is an aqueous medium. The aqueous medium is not particularly limited, and may be water, an organic solvent soluble in water, or a mixture of water and 1 or 2 or more such organic solvents. Organic solvents soluble in water are well known to those skilled in the art, and examples include lower alcohols with 1 to 4 carbon atoms, DMSO, DMF, methyl acetate, ethyl acetate and other esters, and acetones and the like. The developing medium preferably consists primarily of water.

The developing medium can include components for maintaining a constant pH. This is to ensure a pH range so as to maintain a desirable condition, stability and developing environment for the partially double-stranded nucleic acid 10. The buffer salts differ depending on the desired pH, which is normally 6.0 to 8.0, with 7.0 to 8.0 being more desirable. The components for obtaining such a pH may be for example acetic acid and sodium acetate (acetate buffer), citric acid and sodium citrate (citrate buffer), phosphoric acid and sodium phosphate (phosphate buffer) and the like. Another example is phosphate-buffered saline (PBS) or the like. The amplification reaction solution may also be used as is as the developing medium. The amplification reaction solution can also be used as the developing medium after its composition or concentration has been adjusted by addition of an additional solvent or other component such as a surfactant or suitable salts or the like.

When the partially double-stranded nucleic acid 10 is provided with the label binding substance 42, the label 40 may be added in advance in the amplification step before performing the amplification reaction. It is thus possible to obtain a partially double-stranded nucleic acid 10 provided with a label 40 as a composite of the label 40 bound to the label binding substance 42. A composite can similarly be obtained by adding the label 40 to the partially double-stranded nucleic acid 10 in the amplification reaction solution after completion of the reaction. A similar composite can also be obtained by adding the label 40 to the liquid for the developing medium (the added liquid is called the liquid for the developing medium when the amplification reaction solution is used as the developing medium), and then mixing the amplification reaction solution with the liquid for the developing medium.

When supplying an amplification reaction solution containing the partially double-stranded nucleic acid 10 to the hybridization step, preferably the developing medium is prepared in a container, tube or other cavity used in the amplification step, or in other words in a cavity holding the amplification reaction solution, and the hybridization step is performed by bringing the developing medium into contact with the chromatography unit inside the cavity. In this way highly reliable detection with a low risk of contamination can be achieved without the need to collect the amplification reaction solution with a pipette or the like and supply it to the hybridization step. Operational errors can also be reduced.

For example, the PCR or other amplification step is normally performed in a tube-shaped container. Hybridization can be performed by adding label 40 as necessary to the amplification reaction solution in this tube-shaped container, and then supplying the chromatography unit 100 to the tube-shaped container. For example, when the partially double-stranded nucleic acid 10 is provided with biotin as the label binding substance 42, colored latex particles or the like coated with streptavidin or the like can be used as the label 40.

The steps for performing hybridization between the partially double-stranded nucleic acid 10 and probe 120 using the developing medium and chromatography unit 100 are explained next.

The embodiment of the nucleic acid chromatography is not particularly limited when performing the hybridization step. The aim may be to achieve development in a roughly horizontal state. In this case, chromatography is typically performed for example by dripping a fixed amount of the developing medium onto a liquid contact 150. The form of chromatography may also be designed to achieve roughly perpendicular development. In this case, chromatography is typically performed by holding the chromatography unit 100 in a roughly perpendicular direction, and immersing the liquid contact part 50 at the end of the unit 100 in the developing medium.

Depending on the form of chromatography, the developing medium may disperse through the solid phase body carrier 210 and expand to the probe region 230 when the chromatography unit 200 is brought into contact with the developing medium.

When the developing medium contains a partially double-stranded nucleic acid 10 having a tag part 20 for hybridizing with a probe 220 in a probe region 230, the partially double-stranded nucleic acid 10 hybridizes with the probe 220, forming a hybridization product. A signal is thereby obtained corresponding to the label 40 of the partially double-stranded nucleic acid 10. When the label 40 exhibits luminescence or coloration that is detectable with the naked eye, the label pre-associated with the partially double-stranded nucleic acid 10 can be rapidly detected.

When there are multiple probe regions 230, and another partially double-stranded nucleic acid 10 for hybridizing with another probe 220 is present, a hybridization product is formed in the corresponding probe region 230.

There are no particular limitations on the conditions for the hybridization step, which may be performed in air at a temperature of 5°C to 40°C for example, or preferably 15°C to 35°C. Chromatography is initiated in the chromatography unit 100 for example by impregnating part of (the lower part or supply part) of a sheet-shaped chromatography unit with a width of 2.0 mm to 8.0 mm and a height (or length) of 20 mm to 100 mm with about 10 µl to 60 µl of developing medium. The time taken for the developing medium to completely pass through the probe regions 130 is about 2 to 50 minutes.

### (Detection step)

The detection step is a step of detecting the final hybridization product based on the label 40. More specifically, it is a step of confirming the coloring and position of a probe region 230 with a probe 220 fixed thereon. The method of detecting the signal from the label 40 can be selected appropriately according to the type of the label 40. When a coloring reaction using a specific binding reaction or enzyme is required, these operations are performed as necessary. In this chromatography method, the detection step is preferably performed as is without washing the solid phase body carrier.

When the label 40 is a label such as latex particles, gold colloid particles or silver colloid particles that exhibits coloration or luminescence detectable with the naked eye, the presence and amount (based on color concentration or the like) of the target nucleic acid can be detected directly with the naked eye. This allows for even more rapid detection.

### [Examples]

The present disclosure is explained in detail below with examples, but these examples do not limit the present disclosure. In the following examples, all percentages (%) represent mass percentages.

### [Example 1]

In the following examples, the target nucleic acid was detected by the following procedures in the detection method of the present disclosure. The procedures are explained below in order.
(1) Preparation of DNA microarray
(2) Preparation and amplification of target nucleic acid and primers
(3) Hybridization
(4) Detection using scanner

### (1) Preparation of DNA microarray

Aqueous solutions of dissolved synthetic oligo-DNA (Nihon Gene Research Laboratories, Inc.) modified with amino groups at the 3' ends were spotted with a NGK Insulators, Ltd. Geneshot® spotter as detection probes. For the synthetic oligo-DNA sequences, the following 33 sequences capable of rapid hybridization were selected from SEQ ID NOS:1 to 100.

**[Table 3]**

| Name | Seq(5→3') | SEQ.ID. |
|---|---|---|
| D1-001 | TGTTCTCTGACCAATGAATCTGC | 1 |
| D1-002 | TGGAACTGGGAACGCTTTAGATG | 2 |
| D1-003 | TTCGCTTCGTTGTAATTTCGGAC | 3 |
| D1-005 | TAGCCCAGTGATTTATGACATGC | 5 |
| D1-006 | CGCTCTGGTTACTATTGGACGTT | 6 |
| D1-010 | GAGTAGCAGGCAAATACCCTAGA | 10 |
| D1-012 | AGTCATACAGTGAGGACCAAATG | 12 |
| D1-014 | TGCTCACTTACATTACGTCCATG | 14 |
| D1-016 | AGGTCCGGTAGTAATTTAGGTGC | 16 |
| D1-020 | TATTCTACCAACGACATCACTGC | 20 |
| D1-023 | CATCTCCAAGAATTGACCCACCA | 23 |
| D1-025 | GAAGGATCGCTTTTATCTGGCAT | 25 |
| D1-026 | CATTTGTCAGGTACAGTCCACTT | 26 |
| D1-027 | GCCCACACTCTTACTTATCGACT | 27 |
| D1-030 | CCGTCTGGGTTAAAGATTGCTAG | 30 |
| D1-035 | ATGCCGTTGTCAAGAGTTATGGT | 35 |
| D1-038 | CGCGACATTTAGTCCAGGAGATG | 38 |
| D1-040 | AGACAATTAGAATCAGTGCCCCT | 40 |
| D1-041 | GCATTGAGGTATTGTTGCTCCCA | 41 |
| D1-044 | GAGTCCGCAAAAATATAGGAGGC | 44 |
| D1-045 | GCCTCACATAACTGGAGAAACCT | 45 |
| D1-050 | GGGATAGGTATTATGCTCCAGCC | 50 |
| D1-052 | GCCTATATGAACCAAGCCACTGC | 52 |
| D1-062 | CTAGCACAATTAATCAATCCGCC | 62 |
| D1-064 | GCCTATAGTGTCGATTGTCCTCG | 64 |
| D1-065 | CGATCACGGATTAATGTCACCCC | 65 |
| D1-077 | CGCAGTTTGCAAGAACGAACAAA | 77 |
| D1-084 | CCGTGTGTATGAGTATGACAGCA | 84 |
| D1-089 | GAGTCGAAGACCTCCTCCTACTC | 89 |
| D1-090 | ATGCCAATATGTACTCGTGACTC | 90 |
| D1-095 | TGCCGGTTATACCTTTAAGGACG | 95 |
| D1-097 | CGCGGTACTATTAGAAAGGGCTA | 97 |
| D1-100 | TGCAGTGTAAGCAACTATTGTCT | 100 |

After spotting, this was baked at 80°C for one hour. The synthetic oligo-DNA was then fixed by the procedures described below. That is, it was washed for 15 minutes with 2 x SSC/0.2% SDS, then washed for 5 minutes with 95°C 2 x SSC/0.2% SDS, and then washed (by shaking up and down 10 times) three times with sterile water. The water was then removed by centrifugation (1000 rpm x 3 min.).

### (2) Amplification of target nucleic acid

Using human DNA as the genome DNA for amplification, the primers P1-1 to P1-6 (Nihon Gene Research Laboratories, Inc.), P2-1 to P2-6 (Nihon Gene Research Laboratories, Inc.) and P3-1 to P3-6 (Nihon Gene Research Laboratories, Inc.) shown in the following **table** were prepared specific to 6 target nucleic acids ((1) to (6)) in the human genome. Each series was configured as follows (displayed from 5' to 3'). The propylene part of the P3 primers was synthesized in accordance with normal oligonucleotide synthesis methods using Spacer Phosphoramidite C3, a phosphoramidite from GlenResearch shown by the following formula. 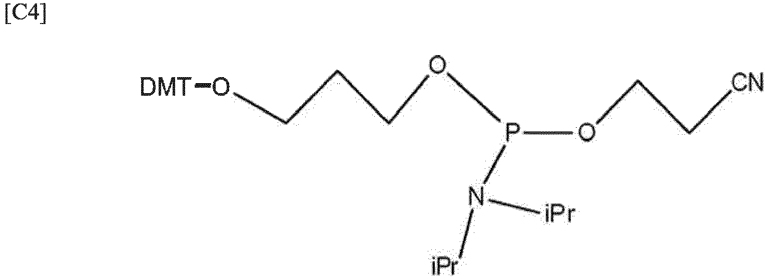
P1 primers: F,R: contain base sequences for specific target nucleic acids (1) to (6) in human DNA
P2 primers: F: binding sequences for labeling probes (tag sequences) + base sequences for target nucleic acids of P1 series
   R: tag sequences consisting of base sequences identical to base sequences of synthetic oligonucleotide probes + base sequences for target nucleic acids of P1 series (because the complement chains of base sequences complementary to these tag sequences are also amplified when using P2 primers, the complement chains hybridize with the probes, and can detect the amplified fragments).
P3 primers: F: binding sequences for labeling probes + linking sites X (propylene chains) + base sequences for target nucleic acids of P1 series
   R: tag sequences consisting of base sequences complementary to base sequences of synthetic oligonucleotide probes + linking sites X (propylene chains) + base sequences for target nucleic acids of P1 series

**[Table 4]**

| Name | | Seq(5' →3') |
|---|---|---|
| P1-1 | F | ACCAAAGAATATGGCTGAATTTAGTAGTGTTTTAAATAATTTTAA |
| | R | ACCTGCTAATGAGATGATCCCTTATTTTGAAAACAACTATTCCTA |
| P1-2 | F | AGCCTAGATTCATTATTCAAAGATATGAAATTTTAAAATGCATA |
| | R | CTAGAGATACTACAGAGCCTGTCCGTCCAAGGTCATA |
| P1-3 | F | CATTTTAATATTGGGTAGAAAAATCAAGAATGCATTGCTCATA |
| | R | TGTTAATCTTATTAAGGTTGCTCAGCTCTAAGATTCTATA |
| P1-4 | F | CATTCATATATTTATGCATTCCTCCATTCAAAAGATCTTATT |
| | R | GTTTAAGTAAAGGATACAGAGGTTTATAAAAGTTTGAAAAC |
| P1-5 | F | TCTGTGGCAATAAGATCCCTATGACTGAAGATGCC |
| | R | GGATAAACCTTAATATAGAAGGAATTAGAGCTGCCACAGC |
| P1-6 | F | GAGAACCTTTGAGGCATCCCTGCTGTTCTCGAGATA |
| | R | AAACATGTGAGGCGTTCACAGAAAGGGTTCAGGAA |

**[Table 5]**

| Name | | Seq(5' →3') |
|---|---|---|
| P2-1 | F | AGGTTTTTCTAGAGTGGACACGGACCAAAGAATATGGCTGAATTTAGTAGTGTTTTAAATAATTTTAA |
| | R | TGTTCTCTGACCAATGAATCTGCACCTGCTAATGAGATGATCCCTTATTTTGAAAACAACTATTCCTA |
| P2-2 | F | AGGTTTTTCTAGAGTGGACACGGAGCCTAGATTCATTATTCAAAGATATGAAATTTTAAAATGCATA |
| | R | TGGAACTGGGAACGCTTTAGATGCTAGAGATACTACAGAGCCTGTCCGTCCAAGGTCATA |
| P2-3 | F | AGGTTTTTCTAGAGTGGACACGGCATTTTAATATTGGGTAGAAAAATCAAGAATGCATTGCTCATA |
| | R | TTCGCTTCGTTGTAATTTCGGACTGTTAATCTTATTAAGGTTGCTCAGCTCTAAGATTCTATA |
| P2-4 | F | AGGTTTTTCTAGAGTGGACACGGCATTCATATATTTATGCATTCCTCCATTCAAAAGATCTTATT |
| | R | AGGCATCCTAAGAAATCGCTACTGTTTAAGTAAAGGATACAGAGGTTTATAAAAGTTTGAAAAC |
| P2-5 | F | AGGTTTTTCTAGAGTGGACACGGTCTGTGGCAATAAGATCCCTATGACTGAAGATGCC |
| | R | TAGCCCAGTGATTTATGACATGCGGATAAACCTTAATATAGAAGGAATTAGAGCTGCCACAGC |
| P2-6 | F | AGGTTTTTCTAGAGTGGACACGGGAGAACCTTTGAGGCATCCCTGCTGTTCTCGAGATA |
| | R | GGCTCTGGTTACTATTGGACGTTAAACATGTGAGGCGTTCACAGAAAGGGTTCAGGAA |

**[Table 6]**

| Name | | Seq(5'→3') |
|---|---|---|
| P3-1 | F | AGGTTTTTCTAGAGTGGACACGGXACCAAAGAATATGGCTGAATTTAGTAGTGTTTTAAATAATTTTAA |
| | R | GCAGATTCATTGGTCAGAGAACAXACCTGCTAATGAGATGATCCCTTATTTTGAAAACAACTATTCCTA |
| P3-2 | F | AGGTTTTTCTAGAGTGGACACGGXAGCCTAGATTCATTATTCAAAGATATGAAATTTTAAAATGCATA |
| | R | CATCTAAAGCGTTCCCAGTTCCAXCTAGAGATACTACAGAGCCTGTCCGTCCAAGGTCATA |
| P3-3 | F | AGGTTTTTCTAGAGTGGACACGGXCATTTTAATATTGGGTAGAAAAATCAAGAATGCATTGCTCATA |
| | R | GTCCGAAATTACAACGAAGCGAAXTGTTAATCTTATTAAGGTTGCTCAGCTCTAAGATTCTATA |
| P3-4 | F | AGGTTTTTCTAGAGTGGACACGGXCATTCATATATTTATGCATTCCTCCATTCAAAAGATCTTATT |
| | R | AGTAGCGATTTCTTAGGATGCCTXGTTTAAGTAAAGGATACAGAGGTTTATAAAAGTTTGAAAAC |
| P3-5 | F | AGGTTTTTCTAGAGTGGACACGGXTCTGTGGCAATAAGATCCCTATGACTGAAGATGCC |
| | R | GCATGTCATAAATCACTGGGCTAXGGATAAACCTTAATATAGAAGGAATTAGAGCTGCCACAGC |
| P3-6 | F | AGGTTTTTCTAGAGTGGACACGGXGAGAACCTTTGAGGCATCCCTGCTGTTCTCGAGATA |
| | R | AACGTCCAATAGTAACCAGAGCGXAAACATGTGAGGCGTTCACAGAAAGGGTTCAGGAA |

Next, genome DNA was amplified as follows using these primers. QIAGEN multiplex PCR master mix was used as the sample amplification reagent. An Applied Biosystems GeneAmp PCR System 9700 was used as the thermal cycler.

First, the following reagents were prepared for each individual sample.

**(Preparation of Reagent)**

| | |
|---|---|
| dH₂O | 4.0µl |
| 2×multiplex PCR master mix | 5.0µl |
| Primer mixture(500nM each) | 0.5µl |
| GenomeDNA(50ng/µl) | 0.5µl |
| Total | 10.0µl |

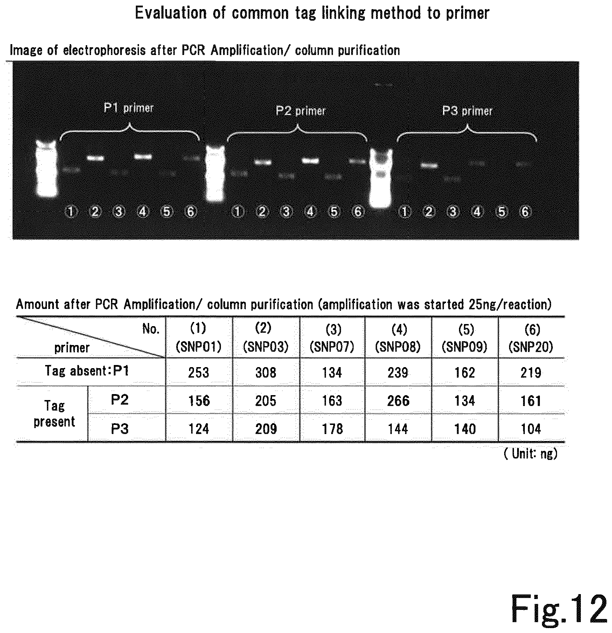

Next, the amplification reagents were transferred to thermal cycle plates, and a thermal cycle reaction was performed (15 minutes at 95°C; 40 cycles of 30 seconds at 95°C, 1 second at 80°C, 6 minutes at 64°C; temperature then lowered to 10°C). The amplified samples were then purified with a QIAGEN MinElute PCR Purification Kit, and amplification of the intended length was confirmed by agarose electrophoresis. The results are shown in **FIG.** 12. Electrophoresis results are shown in the top part of **FIG.** 12, and amplified amounts as calculated from fluorescent strength are shown in the bottom part.

### (3) Hybridization

To hybridize the amplified samples obtained in (2) with detection probes fixed on a microarray, the following Hybri control and Hybri solution were prepared, and hybridization reagents were prepared from these. The PrimerMix contains 25 µm of the labeling probe (bound to the F 5' side of the P2 and P3 primers, which are fluorescent modified oligonucleotides). The Alexa555-rD1_100 used for the Hybri control was a sequence complementary to a sequence corresponding to D1_100, labeled at the 5' end with Alexa555.

**(Hybri control)**

| | |
|---|---|
| Alexa555-rD_1_100(100nM) | 10µl |
| TE(pH8.0) | 390µl |
| Total | 400µl |

**(Hybri solution)**

| | |
|---|---|
| 20×SSC | 2.0ml |
| 10%SDS | 0.8ml |
| 100% Formamide | 12.0ml |
| 100mM EDTA | 0.8ml |
| milliQ | 24.4ml |
| 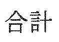 | 40.0ml |

**(Hybridization reagent)**

| | |
|---|---|
| Hybri control | 1.5µl |
| Primer mix | 1.0µl |
| Hybri solution | 9.0µl |
| Sub Total | 10.5µl |
| Amplified sample | 3.0µl |
| Total | 18.0µl |

9 µl of each of the prepared hybridization reagents (labeled sample solutions) was added to a spot area of the microarray without being heated for denaturing or the like, and a hybridization reaction was performed by still standing for 30 minutes at 37°C using a Comfort/plus thermoblock slide (Eppendorf) to prevent drying.

### (Washing)

After completion of the hybridization reaction, the microarray plate was immersed in a glass dye vat filled with a washing solution of the following composition, and shaken up and down for 5 minutes, after which the microarray plate was transferred to a glass dye vat filled with sterile water, shaken up and down for 1 minute, and centrifuged for 1 minute at 2000 rpm to remove the remaining moisture from the surface of the microarray plate.

**(Washing solution)**

| | |
|---|---|
| milliQ | 188.0ml |
| 20×SSC | 10.0ml |
| 10%SDS | 2.0ml |
| Total | 200.0ml |

### (4) Detection using scanner

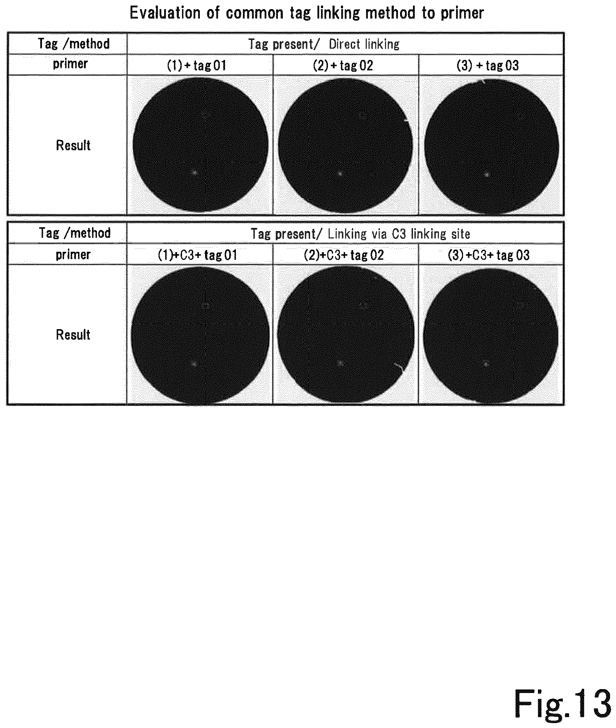

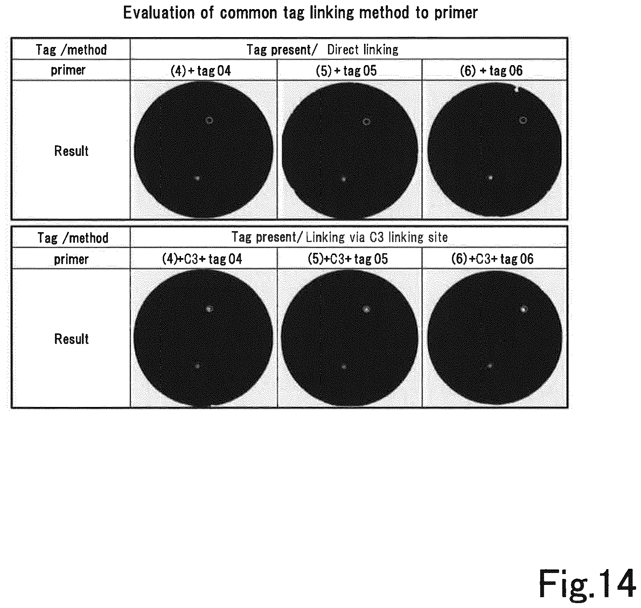

Fluorescent images were obtained using an Applied Precision Co. ArrayWoRx, with the exposure times adjusted appropriately. The results for the plastic plates are shown in **FIGS.** 13 and 14.

As shown in the top part of **FIG.** 12, the target nucleic acid in the genome DNA was amplified whether or not the tag sequence was present. As shown in the bottom part of **FIG.** 12, moreover, there was no great difference in the amount of amplification depending on whether the tag sequence was linked directly to the recognition sequence, or linked via a linking site containing a propylene group.

As shown in **FIGS.** 13 and 14, moreover, using the P2 primers (tag sequence + recognition sequence) and P3 primers (tag sequence + linking site + recognition sequence), a more or less uniformly strong fluorescence was clearly observed irrespective of the individual tag sequence in the results for hybridization with DNA fragments obtained by amplification using the P3 primers. By contrast, almost no fluorescence was observed in any case irrespective of the tag sequence in the hybridization results using the P2 primers.

Moreover, in the hybridization results obtained with the sample concentration diluted 10 times, fluorescence was still observed using the P3 primers. Results similar to those obtained above using plastic plates were also confirmed using glass plates.

These results show that detection sensitivity is improved by at least 10 times or more by using the P3 primers. In the examples above, the amplified samples were applied to the arrays without a denaturing step, and as shown in **FIG.** 12, the synthesized amounts of the amplified samples were equivalent to those obtained with the P2 primers. This shows that double-stranded fragments can be hybridized efficiently and with good labeling efficiency by using the P3 primers.

### [Example 2]

In this example, (1) preparation of the DNA microarray, (2) preparation and amplification of the target nucleic acid and primers, (3) hybridization and (4) detection using the scanner were performed as in Example 1 to detect the target nucleic acid, except that in preparing the DNA microarrays in (1) of Example 1, glass plates (Toyo Kohan Co. geneslide) were used in place of the plastic plates, the 33 sequences shown in the **table** below were selected as the base sequences of the detection probes, and a reagent of the following composition was used as the hybridization reagent in (3) hybridization. As in Example 1, the hybridization signals obtained using the P3 primers (tag sequence + linking site X + recognition sequence) were obviously at least 10 times stronger than those obtained using the P2 primers (tag sequence + recognition sequence), even without a denaturing step.

**(Hybridization reagent)**

| | |
|---|---|
| Hybri control | 1.5µl |
| Primer Mix | 3.5µl |
| Hybri solution | 9.0µl |
| Sub total | 14.0µl |
| Amplified sample | 4.0µl |
| Total | 18.0µl |

**[Table 7]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| 01-004 | AGGCATCCTAAGAAATCGCTACT |
| D1-005 | TAGCCCAGTGATTTATGACATGC |
| D1-006 | CGCTCTGGTTACTATTGGACGTT |
| D1-007 | TAGCCAACTCTAAATAACGGACG |
| D1-008 | TTCGGTTGTCGATATGAGGATCT |
| D1-009 | GGGGGGTACTTCATACAAGATGC |
| D1-010 | GAGTAGCAGGCAAATACCCTAGA |
| D1-011 | GCCTATTAAGGTCTACGTCATCG |
| D1-012 | AGTCATACAGTGAGGACCAAATG |
| D1-013 | CATTCGACATAAGCTGTTGATGC |
| D1-014 | TGCTCACTTACATTACGTCCATG |
| D1-015 | TACACCTATCAACTCGTAGAGCA |
| D1-016 | AGGTCCGGTAGTAATTTAGGTGC |
| D1-017 | TGCACTCTGATATATACAGGCCA |
| D1-018 | GCAGCCCTTATAGATAACGGGAC |
| D1-019 | GAAGCCATGATACTGTTCAGGGT |
| D1-020 | TATTCTACCAACGACATCACTGC |
| D1-021 | CCATCAGTTATTCGGAGGGACTC |
| D1-022 | CCATATCCGATTATTAGCGACGG |
| D1-023 | CATCTCCAAGAATTGACCCACCA |
| D1-024 | CCGTCGTGTTATTAAAGACCCCT |
| D1-025 | GAAGGATCGCTTTTATCTGGCAT |
| D1-026 | CATTTGTCAGGTACAGTCCACTT |
| D1-027 | GCCCACACTCTTACTTATCGACT |
| D1-028 | CGCTGTTACTGTAAGCGTACTAG |
| D1-029 | CGCGATTCCTATTGATTGATCCC |
| D1-030 | CCGTCTGGGTTAAAGATTGCTAG |
| D1-031 | AGTCAGTCCAAATCTCAGGATGG |
| D1-032 | CGCCTAAATGAAACTCACTCTGC |
| D1-100 | TGCAGTGTAAGCAACTATTGTCT |

### [Example 3]

In this example, target nucleic acids were detected by the following methods.

### (1) Preparation of membrane-type DNA microarrays

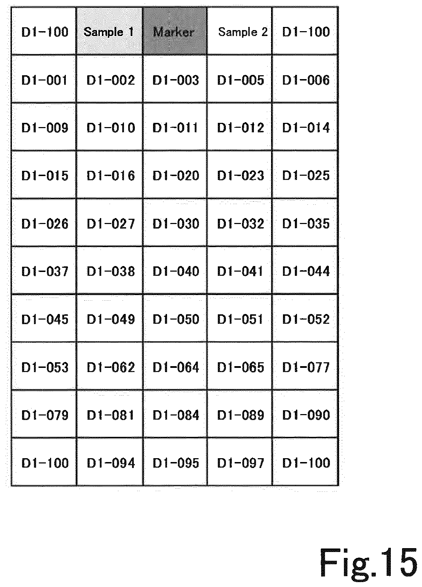

Capture DNA probe solutions comprising the base sequences shown in the following **table** were spotted on Merck Millipore Hi-Flow Plus membrane plates (60 mm x 600 mm), using a NGK Insulators, Ltd. Geneshot® spotter with the discharge unit (inkjet method) described in Japanese Patent Application Publication No. 2003-75305. For the synthetic oligo-DNA sequences, the 44 sequences shown in the following **table** out of the 100 sequences D1_1 to D1_100 described in the Supplementary **Table** 1 of the literature (Analytical Biochemistry 364 (2007) 78-85) were used as probes, and arrayed as shown in **FIG.** 15. The sequences were modified with amino groups at the 3' end of the oligonucleotides for use as probes.

**[Table 8]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| D1-005 | TAGCCCAGTGATTTATGACATGC |
| D1-006 | CGCTCTGGTTACTATTGGACGTT |
| D1-009 | GGGGGGTACTTCATACAAGATGC |
| D1-010 | GAGTAGCAGGCAAATACCCTAGA |
| D1-011 | GCCTATTAAGGTCTACGTCATCG |
| D1-012 | AGTCATACAGTGAGGACCAAATG |
| D1-014 | TGCTCACTTACATTACGTCCATG |
| D1-015 | TACACCTATCAACTCGTAGAGCA |
| D1-016 | AGGTCCGGTAGTAATTTAGGTGC |
| D1-020 | TATTCTACCAACGACATCACTGC |
| D1-023 | CATCTCCAAGAATTGACCCACCA |
| D1-025 | GAAGGATCGCTTTTATCTGGCAT |
| D1-026 | CATTTGTCAGGTACAGTCCACTT |
| D1-027 | GCCCACACTCTTACTTATCGACT |
| D1-030 | CCGTCTGGGTTAAAGATTGCTAG |
| D1-032 | CGCCTAAATGAAACTCACTCTGC |
| D1-035 | ATGCCGTTGTCAAGAGTTATGGT |
| D1-037 | GCACCTCATACCTTCATAGAGCA |
| D1-038 | CGCGACATTTAGTCCAGGAGATG |
| D1-040 | AGACAATTAGAATCAGTGCCCCT |
| D1-041 | GCATTGAGGTATTGTTGCTCCCA |
| D1-044 | GAGTCCGCAAAAATATAGGAGGC |
| D1-045 | GCCTCACATAACTGGAGAAACCT |
| D1-049 | CGCGTCGAATTACTTAATCACCA |
| D1-050 | GGGATAGGTATTATGCTCCAGCC |
| D1-051 | CGCCATTATACAACGGTTCATGC |
| D1-052 | GCCTATATGAACCAAGCCACTGC |
| D1-053 | CGCCGTCAGTACTTGTATAGATG |
| D1-062 | CTAGCACAATTAATCAATCCGCC |
| D1-064 | GCCTATAGTGTCGATTGTCCTCG |
| D1-065 | CGATCACGGATTAATGTCACCCC |
| D1-077 | CGCAGTTTGCAAGAACGAACAAA |
| D1-079 | GGGGTGTGAGAGCTTTTTAGACG |
| D1-081 | ACCACTATGATTGAGGAAACGCG |
| D1-084 | CCGTGTGTATGAGTATGACAGCA |
| D1-089 | GAGTCGAAGACCTCCTCCTACTC |
| D1-090 | ATGCCAATATGTACTCGTGACTC |
| D1-094 | CTAGGTACAACACCAACTGTCTC |
| D1-095 | TGCCGGTTATACCTTTAAGGACG |
| D1-097 | CGCGGTACTATTAGAAAGGGCTA |
| D1-100 | TGCAGTGTAAGCAACTATTGTCT |

After the probes were spotted, they were fixed by exposure to UV light at about 200 to 500 mJ/cm² using a Spectroline Co. UV irradiation device (XL-1 500 UV Crosslinker).

### (2) Amplification of sample genes

Using human DNA as the genomic DNA for amplification, amplification was performed using P2 primers with the sequences shown in **Table** 9 below and P3 primers with the sequences shown in **Table** 10 below. Each of the primers was configured as follows.
P2 primers: F: binding sequences for labeling probes + base sequences for target nucleic acids of P1 series
   R: tag sequences consisting of base sequences identical to base sequences of synthetic oligonucleotide probes + base sequences for target nucleic acids of P1 series (because the complement chains of base sequences complementary to these tag sequences are also amplified when using P2 primers, the complement chains hybridize with the probes, and can detect the amplified fragments).
P3 primers: F: binding sequences for labeling probes + linking sites X (propylene chains) + base sequences for target nucleic acids of P1 series
   R: tag sequences consisting of base sequences complementary to base sequences of synthetic oligonucleotide probes + linking sites X (propylene chains) + base sequences for target nucleic acids of P1 series

**[Table 9]**

| Name | | Seq(5' ⇒3') |
|---|---|---|
| P2-1 | F | AGGTTTTTCTAGAGTGGACACGGACCAAAGAATATGGCTGAATTTAGTAGTGTTTTAAATAATTTTAA |
| | R | TGTTCTCTGACCAATGAATCTGCACCTGCTAATGAGATGATCCCTTATTTTGAAAACAACTATTCCTA |
| P2-2 | F | AGGTTTTTCTAGAGTGGACACGGAGCCTAGATTCATTATTCAAAGATATGAAATTTTAAAATGCATA |
| | R | TGGAACTGGGAACGCTTTAGATGCTAGAGATACTACAGAGCCTGTCCGTCCAAGGTCATA |

**[Table 10]**

| Name | | Seq(5' ⇒3') |
|---|---|---|
| P3-1 | F | AGGTTTTTCTAGAGTGGACACGGXACCAAAGAATATGGCTGAATTTAGTAGTGTTTTAAATAATTTTAA |
| | R | GCAGATTCATTGGTCAGAGAACAXACCTGCTAATGAGATGATCCCTTATTTTGAAAACAACTATTCCTA |
| P3-2 | F | AGGTTTTTCTAGAGTGGACACGGXAGCCTAGATTCATTATTCAAAGATATGAAATTTTAAAATGCATA |
| | R | CATCTAAAGCGTTCCCAGTTCCAXCTAGAGATACTACAGAGCCTGTCCGTCCAAGGTCATA |

The linking sites were synthesized by ordinary oligonucleotide synthesis methods as in Example 1, using Glen Research Spacer Phosphoramidite C3.

Next, genome DNA was amplified as follows using these primers. A QIAGEN multiplex PCR master mix was used as the sample amplification reagent. An Applied Biosystems GeneAmp PCR System 9700 was used as the thermal cycler.

**(Preparation of Reagent)**

| | |
|---|---|
| dH₂O | 4.0µl |
| 2×multiplex PCR master mix | 5.0µl |
| Primer mixture(500nM each) | 0.5µl |
| GenomeDNA(50ng/µl) | 0.5µl |
| Total | 10.0µl |

Next, the amplification reagents were transferred to thermal cycle plates, and a thermal cycle reaction was performed (15 minutes at 95°C; 40 cycles of 30 seconds at 95°C, 1 second at 80°C, 6 minutes at 64°C; temperature then lowered to 10°C). The amplified samples were then purified with a QIAGEN MinElute PCR Purification Kit, and amplification of the intended length was confirmed by agarose electrophoresis.

### (3) Detection using membrane-type DNA microarray

The reaction on the membrane-type DNA microarray using the samples amplified in (2) and the detection procedures were as follows.

**(Hybiridization sample)**

| | |
|---|---|
| Hybri Solution* (0.5%Tween20-1%BSA-PBS) | 200.0µl |
| Biotin labeled oligoDNA complementary to label biding sequence of F' primer (25µM) | 4.0µl |
| Sample | 4.0µl |
| Total | 208.0µl |

### (Hybridization and coloring reaction)

Membrane-type DNA microarrays were cut to a size for insertion into 0.2 ml tubes and set in the tubes, 200 µl of each prepared hybridized sample was added without being heated for purposes of denaturing or the like, and a hybridization reaction was performed for 30 minutes at a heat block temperature of 37°C.

After completion of the hybridization reaction, each membrane-type DNA microarray was transferred to a 0.2 ml tube filled with washing liquid (0.1% Tween 20-1 mM EDTA-TBS), and washed in a heat block at 37°C (37°C x 1 min, 37°C x 10 min, 37°C x 1 min).

Each washed membrane-type DNA microarrays was transferred to an 0.2 ml tube filled with a mixture of biotin-HRP and streptavidin, and reacted for 20 minutes at room temperature.

After completion of the reaction, each membrane-type DNA microarray was transferred to an 0.2 ml tube filled with washing liquid (0.1% Tween 20-1 mM EDTA-TBS), and washed (room temp. x 1 min, room temp. x 10 min, room temp. x 1 min).

The washed membrane-type DNA microarray was subjected to a coloring reaction for about 5 minutes at room temperature using a Vector Laboratories TMB Peroxidase Substrate Kit, 3,3',5,5'-tetramethylbenzidine.

### (Detection assessment)

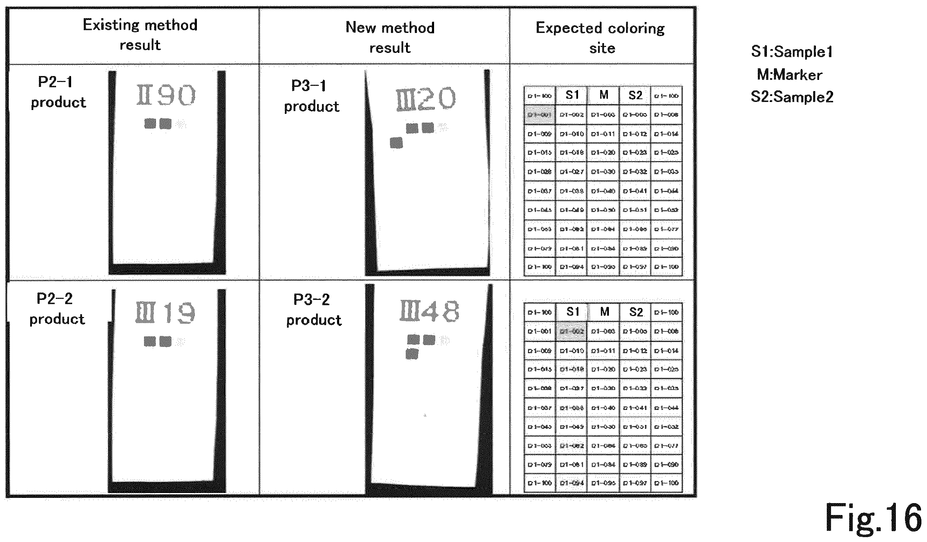

Coloration of the array after drying was confirmed visually. The results are shown in **FIG.** 16. As shown in **FIG.** 16, while only a slight coloration was barely detected by conventional methods using the P2 primers in both samples, dark coloration was observed with the method of the disclosure using the P3 primers. This shows that detection sensitivity is improved by using the P3 primers. It also shows that hybridization is possible without heat denaturing.

### [Example 4]

In this example, target nucleic acids were detected by the following methods.

### (1) Preparation of membrane-type DNA microarrays

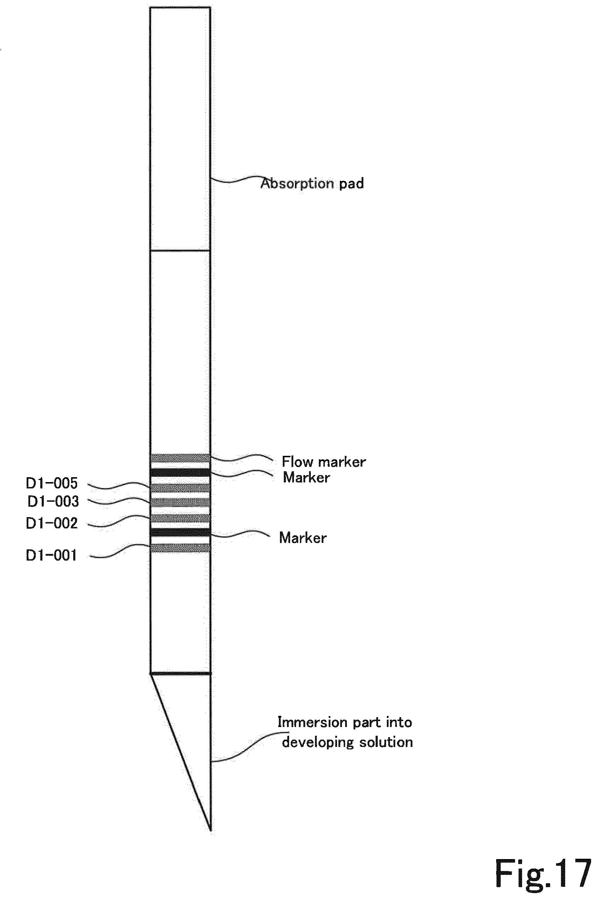

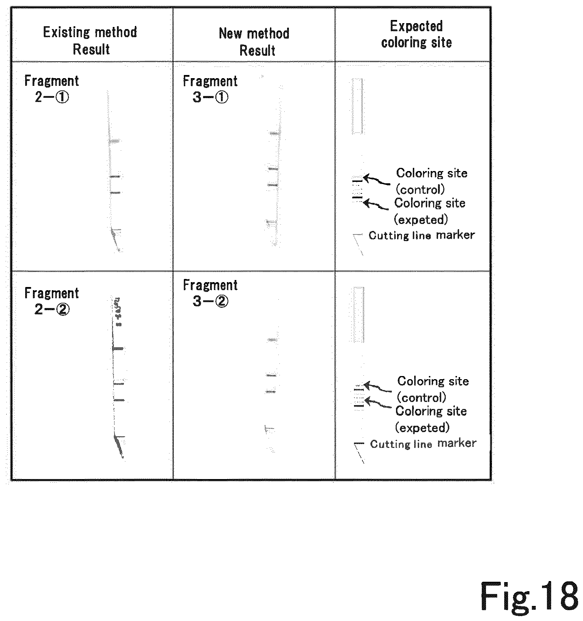

Capture DNA probe solutions comprising the base sequences shown in the following **Table** 11 were spotted on Merck Millipore Hi-Flow Plus membrane plates (60 mm x 600 mm), using a NGK Insulators, Ltd. Geneshot® spotter with the discharge unit (inkjet method) described in Japanese Patent Application Publication No. 2003-75305. For the synthetic oligo-DNA sequences, the 4 sequences shown in the following **table** out of the 100 sequences D1_1 to D1_100 described in the Supplementary **Table** 1 of the literature (Analytical Biochemistry 364 (2007) 78-85) were used as probes. The sequences were modified with amino groups at the 3' end of the oligonucleotides for use as probes. These DNA were arrayed in stream like lines as shown in **FIG.** 17. In the array shown in **FIG.** 17, the probe fixing solution was colored with a dye and spotted in streams to clearly show the probe fixing regions. In the arrays shown in **FIG.** 18, a liquid containing a pigment was spotted in streams (bands) next to the probe fixing regions to make it easier to find the probe-fixing regions where hybridization products are detected.

**[Table 11]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| D1-005 | TAGCCCAGTGATTTATGACATGC |

After the probes were spotted, they were fixed by exposure to UV light at about 200 to 500 mJ/cm² using a Spectroline Co. UV irradiation device (XL-1 500 UV Crosslinker).

### (2) Amplification of sample gene

Using human DNA as the genomic DNA for amplification, amplification was performed using P2 primers with the sequences shown in **Table** 9 of Example 3 and P3 primers with the sequences shown in **Table** 10 of Example 3. As in Example 3, an amplification reaction was performed on genome DNA using these primers. The amplified samples were then purified with a QIAGEN MinElute PCR Purification Kit, and amplification of the intended length was confirmed by agarose electrophoresis.

### (3) Detection using membrane-type DNA microarray

The reaction and detection procedures on the membrane-type DNA microarray using the samples amplified in (2) were as follows. The developing solution and latex solution were from AMR Inc. The latex solution consisted of oligo-DNA with a sequence complementary to the labeling probe binding sequence of each F primer, fixed to polystyrene latex beads containing a blue colorant, and diluted to a concentration of 100 nM with developing solution. The oligo-DNA was fixed to the beads by forming covalent bonds between oligo-DNA modified at the 5' end with amino groups and carboxyl groups on the surface of the latex. Phosphate buffered saline was used as the developing solution.

**(Hybridization sample)**

| | |
|---|---|
| Developing solution * | 35.0µl |
| Latex solution* | 5.0µl |
| Sample | 10.0µl |
| Total | 50.0µl |

### (Hybridization)

50 µl of each of the hybridization samples was added to a 0.2 ml tube without being heated for purposes of denaturing or the like, and hybridization by chromatography was initiated by inserting the membrane-type DNA microarray. In about 20 minutes, all the sample liquid had been absorbed and the reaction was complete. After completion of the reaction, the membrane-type DNA microarray was air dried.

### (Detection evaluation)

The presence or absence of coloration in the membrane-type DNA arrays after drying was evaluated visually. The results are shown in **FIG.** 18. As shown in **FIG.** 18, no coloration was observed with the conventional method using the P2 primers, but dark coloration was observed with the method using the P3 primers. This shows that hybridization detection sensitivity is improved by using the P3 primers regardless of the mode of hybridization. It also shows that hybridization is possible with the P3 primers even without heat denaturing.

### [Example 5]

### (1) Preparation of membrane-type DNA chromatography

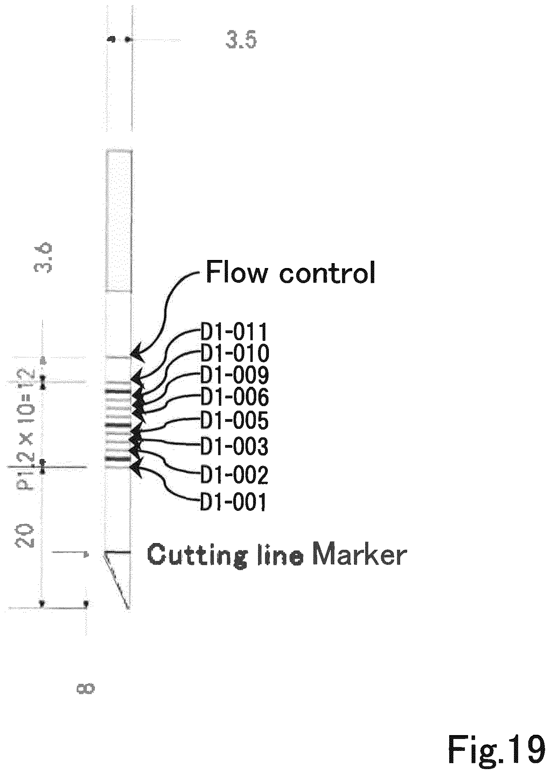

DNA probe solutions comprising the base sequences shown in the following **table** were spotted on Merck Millipore Hi-Flow Plus membrane plates (60 mm x 600 mm) and arrayed as shown in **FIG.** 19, using a NGK Insulators, Ltd. Geneshot® spotter with the discharge unit (inkjet method) described in Japanese Patent Application Publication No. 2003-75305. For the synthetic oligo-DNA sequences, the 8 sequences shown in the following **table** out of the 100 sequences D1_1 to D1_100 described in the Supplementary **Table** 1 of the literature (Analytical Biochemistry 364 (2007) 78-85) were used as probes. The sequences were modified with amino groups at the 3' end of the oligonucleotides for use as probes.

Further, three position marker regions were formed using red pigment as shown **FIG.** 19.

**[Table 12]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| D1-005 | TAGCCCAGTGATTTATGACATGC |
| D1-006 | CGCTCTGGTTACTATTGGACGTT |
| D1-009 | GGGGGGTACTTCATACAAGATGC |
| D1-010 | GAGTAGCAGGCAAATACCCTAGA |
| D1-011 | GCCTATTAAGGTCTACGTCATCG |

After the probes were spotted, they were fixed by exposure to UV light at about 300 mJ/cm² using a Spectroline Co. UV irradiation device (XL-1 500 UV Crosslinker) and chromatography units were obtained comprising the probe regions of 8 different probes together with 3 different position marker regions.

### (2) Amplification of target nucleic acid

Human DNA (commercial product from Cosmo Bio) was used as the genome DNA for amplification. Primer sets consisting of the base sequences shown in **Table** 13 below were used as the primers. That is, each R primer was provided with a tag sequence complementary to a probe (D1-001, 002, 003 and 005), a linking site X and a first recognition sequence, and each F primer was provided with biotin, a linking site X and a second recognition sequence, and amplification was performed using these. With these primer sets, it was possible to obtain partially double-stranded DNA (single-stranded single type) having a tag sequence as a single strand. As a comparative example, a similar nucleic acid amplification reaction was performed using primer sets consisting of the base sequences shown in **Table** 14. With these primer sets, it is possible to obtain partially double-stranded DNA (single-stranded double type) having both a tag sequence and a labeling sequence capable of binding to a labeling probe as single strands.

**[Table 13]**

| Name | | Seq(5'⇒3') |
|---|---|---|
| single-stranded single 1 | F | GCAGATTCATTGGTCAGAGAACAXCCTGACTAGCATATAAGAAGCTTTCAGCAAGTGCAGACTA |
| | R | [biotin]-ATTTTTGCATCGTAAGCAAAAATGATTGGTTGAACATGAA |
| single-stranded single 2 | F | CATCTAAAGCGTTCCCAGTTCCAXGCTTGCCCCTGGGCTTTTATAAGTCGTCACGGAGA |
| | R | [biotin]-CAAATTTGAGACGGCTCCAACTCAGTAATCTTTTTCCAAA |
| single-stranded single 3 | F | GTCCGAAATTACAACGAAGCGAAXGAAATGGGTTCCCTGGTTGTCAGCCTCTGCGAAGTA |
| | R | [biotin]-AACCGGCCCAGCTTCGACGGTATCCTCTACTACTA |
| single-stranded single 4 | F | GCATGTCATAAATCACTGGGCTAXCATAGCAAGTTGTTCATTGTTGTAACCCTGGTACCTG |
| | R | [biotin]-TTTAAGTGCAAATTTATTTCGCCTCCAAAGGGACCTCCCA |

The primers shown in **Table** 13 all have tag sequences complementary to the respective probes (D1-001, 002, 003 and 005) together with linking sites X and first and second recognition sequences. These primers were all from Nihon Gene Research Laboratories, Inc.

**[Table 14]**

| Name | | Seq(5'⇒3') |
|---|---|---|
| single-stranded double 1 | F | GCAGATTCATTGGTCAGAGAACAXCCTGACTAGCATATAAGAAGCTTTCAGCAAGTGCAGACTA |
| | R | AGGTTTTTCTAGAGTGGACACGGXATTTTTGCATCGTAAGCAAAAATGATTGGTTGAACATGAA |
| single-stranded double 2 | F | CATCTAAAGCGTTCCCAGTTCCAXGCTTGCCCCTGGGCTTTTATAAGTCGTCACGGAGA |
| | R | AGGTTTTTCTAGAGTGGACACGGXCAAATTTGAGACGGCTCCAACTCAGTAATCTTTTTCCAAA |
| single-stranded double 3 | F | GTCCGAAATTACAACGAAGCGAAXGAAATGGGTTCCCTGGTTGTCAGCCTCTGCGAAGTA |
| | R | AGGTTTTTCTAGAGTGGACACGGXAACCGGCCCAGCTTCGACGGTATCCTCTACTACTA |
| single-stranded double 4 | F | GCATGTCATAAATCACTGGGCTAXCATAGCAAGTTGTTCATTGTTGTAACCCTGGTACCTG |
| | R | AGGTTTTTCTAGAGTGGACACGGXTTTAAGTGCAAATTTATTTCGCCTCCAAAGGGACCTCCCA |

The linking sites X are propyleneoxy chains, introduced with phosphoramidite (spacer phosphoramidite C3, GlenResearch).

The composition in the amplification reaction was as follows, and the thermal cycle conditions were 15 minutes at 95°C followed by 40 cycles, each of which comprises 30 seconds at 95°C, 1 second at 80°C and 6 minutes at 64°C, followed by cooling to 10°C.

**(Composition)**

| | |
|---|---|
| 2×Qiagen multiplex PCR master mix | 5.0µl |
| Primer mix(500nM each) | 0.5µl |
| dH₂O | 4.0µl |
| Genome DNA | 0.5µl |
| Total | 10.0µl |

Mixtures of the primer sets for each of the amplification product singles 1 to 4 shown in **Table** 13 and a mixture of all of the primer sets for the singles 1 to 4 were prepared, for a total of 5 mixes. Similarly, a total of 5 primer mixes were prepared for the amplification product doubles 1 to 4 shown in **Table** 14.

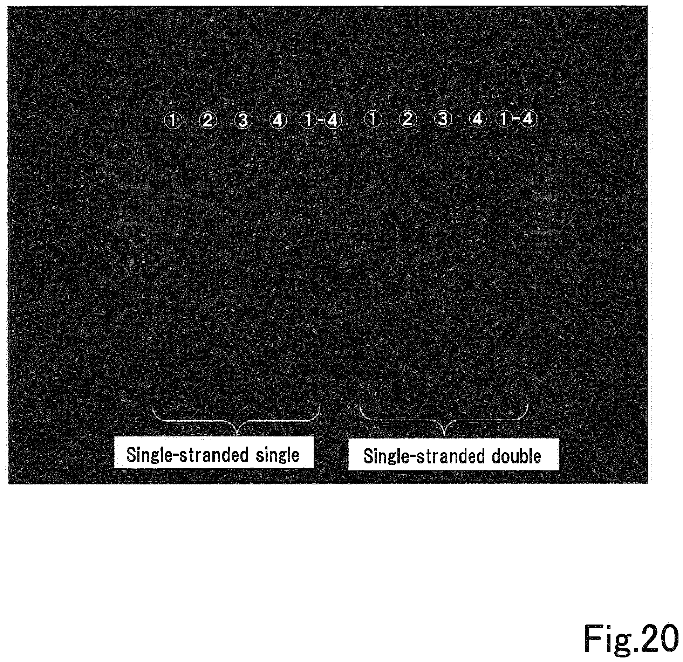

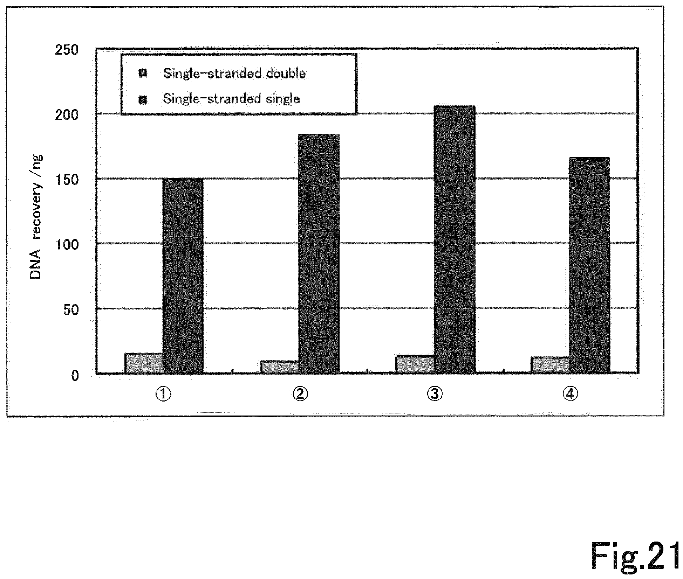

The amplification products obtained by amplification were purified with a QIAGEN MinElute PCR Purification Kit, and amplification of fragments of the desired length was confirmed by agarose electrophoresis. The yield of each amplification product after purification was also confirmed. The results are shown in **FIG.** 20 and **FIG.** 21. In **FIG.** 20 and **FIG.** 21, each of the circled numbers represents the number of a primer set for obtaining a single-stranded single or single-stranded double partially double-stranded nucleic acid.

As shown in **FIG.** 20 and **FIG.** 21,the single-stranded single amplification products had greater amplified amounts and greater yields after purification than the comparative examples. In other words, with a partially double-stranded nucleic acid having a single strand on only one chain, better amplification efficiency can be obtained than with a partially double-stranded nucleic acid having single strands on both chains.

### (3) Detection using chromatography unit

A hybridization reaction and detection were carried out by nucleic acid chromatography using the partially double-stranded DNA amplified in (2). The operations were as follows for the single-stranded singles and single-stranded doubles, respectively.

**(Developing solution for single-stranded singles)**

| | |
|---|---|
| PBS | 30.0µl |
| Latex solution | 5.0µl |
| Amplification reaction solution | 10.0µl |
| Milipore water | 5.0µl |
| Total | 50µl |

The developing solution was prepared by mixing PBS (phosphate-buffered saline) with a latex solution and amplification reaction solution (5 kinds). For the latex storage solution, polystyrene latex beads containing a blue colorant were coated within avidin (streptavidin), and prepared with PBS to a specific concentration.

**(Developing solution for single-stranded doubles)**

| | |
|---|---|
| PBS | 30.0µl |
| Latex solution | 5.0µl |
| Amplification reaction solution | 10.0µl |
| Milipore water | 5.0µl |
| Total | 50µl |

The developing solution was prepared by mixing PBS (phosphate-buffered saline) with a latex solution and the amplification reaction solution (5 kinds), and then mixing in millipore water. For the latex storage solution used in the developing solutions of the single-stranded doubles, linking DNA with a sequence complementary to the labeling sequence of the R primer was fixed to polystyrene latex beads containing a blue colorant, and prepared with PBS to a similar concentration as the single-stranded singles. The linking DNA was modified with an amino group at the 5' end, and fixed by forming covalent bonds between the amino groups and the carboxyl groups on the latex surface.

### (Hybridization step)

50 µl of each of the developing solutions above was added to a 0.2 ml tube, and the bottom ends of each of the chromatography units (8-type and 4-type) were inserted to initiate a hybridization reaction by chromatography. All of the developing solution was absorbed in about 20 minutes, and the hybridization reaction by chromatography was completed. After completion of the reaction, the chromatography units were air dried, and the reaction sites were confirmed with the naked eye and photographed.

### (Detection step)

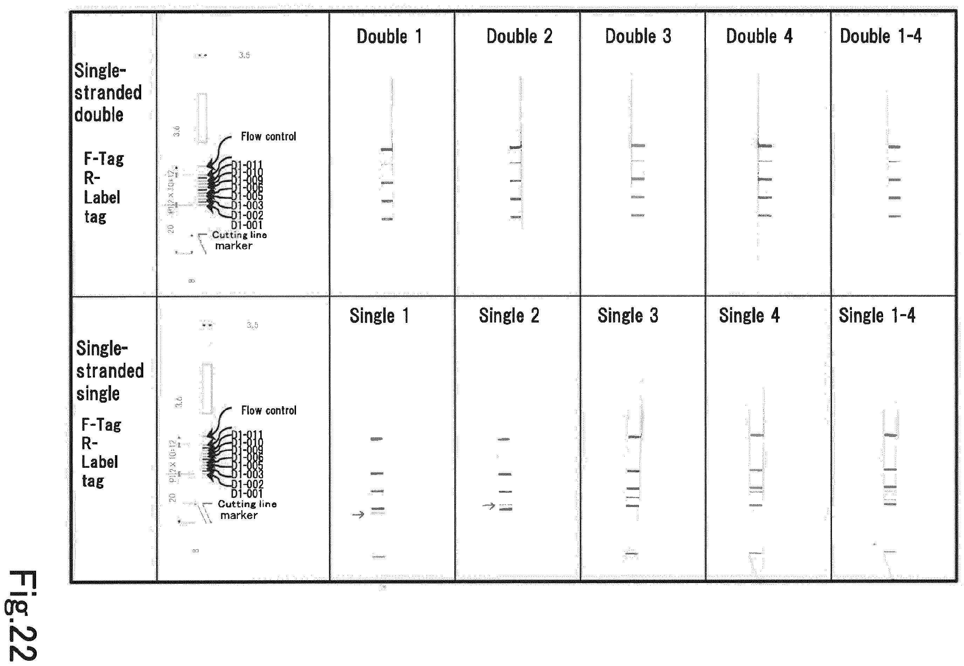

The presence or absence of coloration in the array after drying was confirmed. The results are shown in **FIG.** 22. As shown in **FIG.** 22, although coloration was also confirmed in the amplification products of the single-stranded double type, a darker coloration was observed in the amplification products of the single-stranded single type, confirming the effectiveness of the present method using a partially double-stranded nucleic acid of the single-stranded single type in nucleic acid chromatography instead of a partially double-stranded nucleic acid of the single-stranded double type.

### [Example 6]

### (1) Preparation of membrane-type DNA chromatography

DNA probe solutions comprising the base sequences shown in the following **table** were spotted on Merck Millipore Hi-Flow Plus membrane plates (60 mm x 600 mm) and arrayed as shown in **FIG.** 19, using a NGK Insulators, Ltd. Geneshot® spotter with the discharge unit (inkjet method) described in Japanese Patent Application Publication No. 2003-75305. For the synthetic oligo-DNA sequences, the 8 sequences shown in the following **table** out of the 100 sequences D1_1 to D1_100 described in the Supplementary **Table** 1 of the literature (Analytical Biochemistry 364 (2007) 78-85) were used as probes. The sequences were modified with amino groups at the 3' end of the oligonucleotides for use as probes. Further, three position marker regions were formed using red pigment as shown **FIG.** 19

**[Table 15]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| D1-005 | TAGCCCAGTGATTTATGACATGC |
| D1-006 | CGCTCTGGTTACTATTGGACGTT |
| D1-009 | GGGGGGTACTTCATACAAGATGC |
| D1-010 | GAGTAGCAGGCAAATACCCTAGA |
| D1-011 | GCCTATTAAGGTCTACGTCATCG |

After the probes were spotted, they were fixed by exposure to UV light at about 300 mJ/cm² using a Spectroline Co. UV irradiation device (XL-1 500 UV Crosslinker) and chromatography units were obtained comprising the probe regions of 8 different probes together with 3 different position marker regions.

### (2) Amplification of target nucleic acid

Human DNA (commercial product from Cosmo Bio) was used as the genome DNA for amplification. Primer sets consisting of the base sequences shown in **Table** 16 below were used as the primers. That is, each R primer was provided with a tag sequence complementary to a probe (D1-001, 002, 003 and 005), a linking site X and a first recognition sequence, and each F primer was provided with a second recognition sequence, and amplification was performed using these. During amplification, biotin-16-dUTP (Roche Applied Science) was added in addition to the raw dNTP so that biotin was incorporated into the double-stranded DNA after amplification, resulting in partially double-stranded DNA (single-stranded single type) having a tag sequence as a single strand. As a comparative example, a similar nucleic acid amplification reaction was performed using primer sets consisting of the base sequences shown in **Table** 17. As above, biotin-16-dUTP (Roche Applied Science) was added in addition to the raw dNTP to obtain partially double-stranded DNA (single-stranded double type) having labeling sequences capable of binding to the tag sequence and labeling probe, respectively, as single strands.

**[Table 16]**

| Name | | Seq(5'⇒3') |
|---|---|---|
| single-stranded single① | F | GCAGATTCATTGGTCAGAGAACAXCCTGACTAGCATATAAGAAGCTTTCAGCAAGTGCAGACTA |
| | R | ATTTTTGCATCGTAAGCAAAAATGATTGGTTGAACATGAA |
| single-stranded single② | F | CATCTAAAGCGTTCCCAGTTCCAXGCTTGCCCCTGGGCTTTTATAAGTCGTCACGGAGA |
| | R | CAAATTTGAGACGGCTCCAACTCAGTAATCTTTTTCCAAA |
| single-stranded single③ | F | GTCCGAAATTACAACGAAGCGAAXGAAATGGGTTCCCTGGTTGTCAGCCTCTGCGAAGTA |
| | R | AACCGGCCCAGCTTCGACGGTATCCTCTACTACTA |
| single-stranded single④ | F | GCATGTCATAAATCACTGGGCTAXCATAGCAAGTTGTTCATTGTTGTAACCCTGGTACCTG |
| | R | TTTAAGTGCAAATTTATTTCGCCTCCAAAGGGACCTCCCA |

The primers shown in **Table** 16 all have tag sequences complementary to the respective probes (D1-001, 002, 003 and 005) together with linking sites X and first recognition sequences. These primers were all from Nihon Gene Research Laboratories, Inc.

**[Table 17]**

| Name | | Sea(5' ⇒3') |
|---|---|---|
| single-stranded double① | F | GCAGATTCATTGGTCAGAGAACACCTGACTAGCATATAAGAAGCTTTCAGCAAGTGCAGACTA |
| | R | ATTTTTGCATCGTAAGCAAAAATGATTGGTTGAACATGAA |
| single-stranded double② | F | CATCTAAAGCGTTCCCAGTTCCAGCTTGCCCCTGGGCTTTTATAAGTCGTCACGGAGA |
| | R | CAAATTTGAGACGGCTCCAACTCAGTAATCTTTTTCCAAA |
| single-stranded double③ | F | GTCCGAAATTACAACGAAGCGAAGAAATGGGTTCCCTGGTTGTCAGCCTCTGCGAAGTA |
| | R | AACCGGCCCAGCTTCGACGGTATCCTCTACTACTA |
| single-stranded double④ | F | GCATGTCATAAATCACTGGGCTACATAGCAAGTTGTTCATTGTTGTAACCCTGGTACCTG |
| | R | TTTAAGTGCAAATTTATTTCGCCTCCAAAGGGACCTCCCA |

The linking sites X are propyleneoxy chains, introduced with phosphoramidite (spacer phosphoramidite C3, GlenResearch).

The composition in the amplification reaction was as follows, and the thermal cycle conditions were 15 minutes at 95°C followed by 40 cycles, each of which comprises 30 seconds at 95°C, 1 second at 80°C and 6 minutes at 64°C, followed by cooling to 10°C.

**(Composition)**

| | |
|---|---|
| 2×Qiagen multiplex PCR master mix | 5.0µl |
| Primer mix(500nM each) | 0.5µl |
| Biotin-16-dUTP(1mM) | 0.5µl |
| dH₂O | 3.5µl |
| Genome DNA | 0.5µl |
| Total | 10.0µl |

Mixtures of the primer sets for each of the amplification product singles 1 to 4 shown in **Table** 16 and a mixture of all of the primer sets for the singles 1 to 4 were prepared, for a total of 5 mixes. Similarly, a total of 5 primer mixes were prepared for the amplification product doubles 1 to 4 shown in **Table** 17.

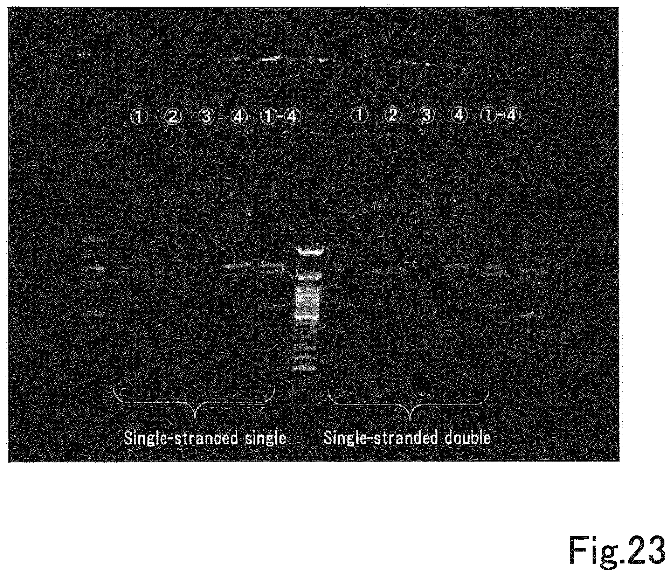

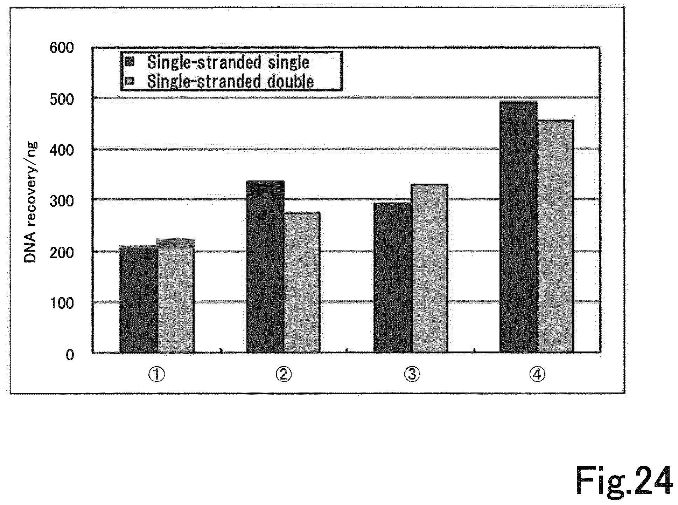

The amplification products obtained by amplification were purified with a QIAGEN MinElute PCR Purification Kit, and amplification of fragments of the desired length was confirmed by agarose electrophoresis. The yield of each amplification product after purification was also confirmed. The results are shown in **FIG.** 23 and **FIG.** 24.

As shown in **FIG.** 23 and **FIG.** 24, there was no great difference between the single-stranded single type and the double-stranded single type in terms of amplified amount of the amplification product or yield after purification.

### (3) Detection using chromatography unit

A hybridization reaction and detection were carried out by nucleic acid chromatography using the partially double-stranded DNA amplified in (2). The operations were as follows for the single-stranded singles and double-stranded singles, respectively.

**(Developing solution for single-stranded singles)**

| | |
|---|---|
| PBS | 30.0µl |
| Latex solution | 5.0µl |
| Amplification reaction solution | 10.0µl |
| Milipore water | 5.0µl |
| Total | 50µl |

The developing solution was prepared by mixing PBS (phosphate-buffered saline) with a latex solution and amplification reaction solution (5 kinds). For the latex storage solution, polystyrene latex beads containing a blue colorant were coated within avidin (streptavidin), and prepared with PBS to a specific concentration.

**(Developing solution for double-stranded singles)**

| | |
|---|---|
| PBS | 30.0µl |
| Latex solution | 5.0µl |
| Amplification reaction solution | 10.0µl |
| Milipore water | 5.0µl |
| Total | 50µl |

The developing solution was prepared by mixing PBS (phosphate-buffered saline) with a latex solution and the amplification reaction solution (5 kinds), and then mixing biotin solution. For the latex storage solution used in the developing solutions of the single-stranded doubles, linking DNA with a sequence complementary to the labeling sequence of the R primer was fixed to polystyrene latex beads containing a blue colorant, and prepared with PBS to a similar concentration as the single-stranded singles.

### (Hybridization step)

50 µl of each of the developing solutions above was added to a 0.2 ml tube, and the bottom ends of each of the chromatography units (8-type and 4-type) were inserted to initiate a hybridization reaction by chromatography. All of the developing solution was absorbed in about 20 minutes, and the hybridization reaction by chromatography was completed. After completion of the reaction, the chromatography units were air dried, and the reaction sites were confirmed with the naked eye and photographed.

### (Detection step)

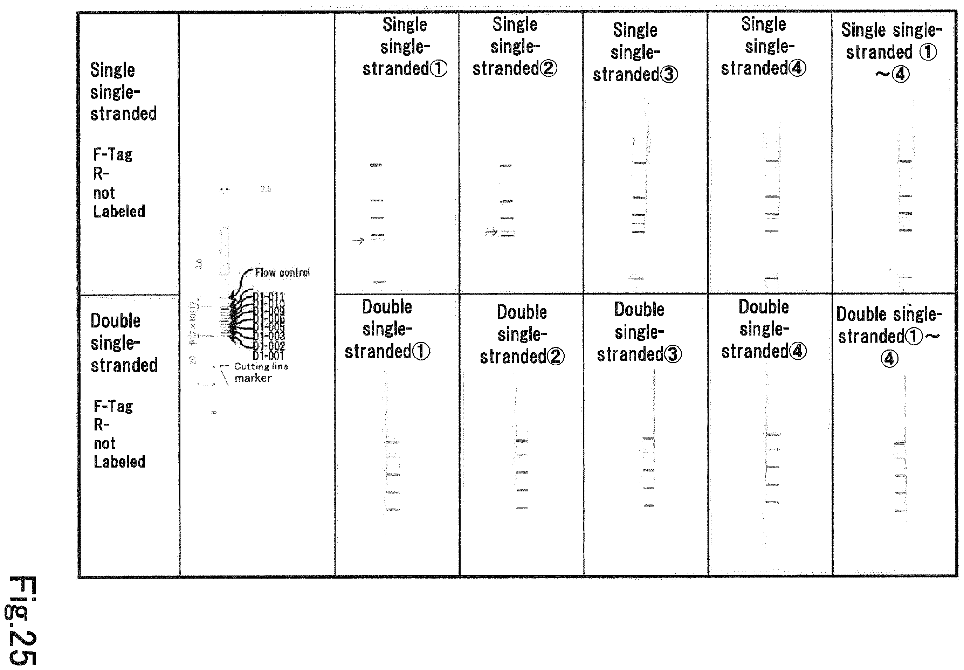

The presence or absence of coloration in the array after drying was confirmed visually. The results are shown in **FIG.** 25. As shown in **FIG.** 25, although coloration was not confirmed in the amplification products of the double-stranded single type, a dark coloration was observed in the amplification products of the single-stranded single type, confirming the effectiveness of the present method using a partially double-stranded nucleic acid of the single-stranded single type.

### [Example 7]

### (1) Preparation of membrane-type DNA chromatography

DNA probe solutions comprising the base sequences shown in the following **table** were spotted on Merck Millipore Hi-Flow Plus membrane plates (60 mm x 600 mm) and arrayed as shown in **FIG.** 19, using a NGK Insulators, Ltd. Geneshot® spotter with the discharge unit (inkjet method) described in Japanese Patent Application Publication No. 2003-75305. For the synthetic oligo-DNA sequences, the 8 sequences shown in the following **table** out of the 100 sequences D1_1 to D1_100 described in the Supplementary **Table** 1 of the literature (Analytical Biochemistry 364 (2007) 78-85) were used as probes. The sequences were modified with amino groups at the 3' end of the oligonucleotides for use as probes. Further, three position marker regions were formed using red pigment as shown **FIG.** 19.

**[Table 18]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| D1-005 | TAGCCCAGTGATTTATGACATGC |
| D1-006 | CGCTCTGGTTACTATTGGACGTT |
| D1-009 | GGGGGGTACTTCATACAAGATGC |
| D1-010 | GAGTAGCAGGCAAATACCCTAGA |
| D1-011 | GCCTATTAAGGTCTACGTCATCG |

After the probes were spotted, they were fixed by exposure to UV light at about 300 mJ/cm² using a Spectroline Co. UV irradiation device (XL-1 500 UV Crosslinker) and chromatography units were obtained comprising the probe regions of 8 different probes together with 3 different position marker regions.

### (2) Amplification of target nucleic acid

Human DNA (commercial product from Cosmo Bio) was used as the genome DNA for amplification. Primer sets consisting of the base sequences shown in **Table** 19 below were used as the primers. That is, each R primer was provided with a tag sequence complementary to a probe (D1-001, 002, 003 and 005), a linking site X and a first recognition sequence, and each F primer was provided with a tag sequence and a second recognition sequence, and amplification was performed using these. During amplification, a primer (R' primer; **Table** 20) modified with biotin at the 5' end of the tag sequence in the R primer was added in addition to the aforementioned two kinds of primers so that biotin would be incorporated into the double-stranded DNA ends after amplification, resulting in partially double-stranded DNA having a tag sequence as a single strand.

**[Table 19]**

| Name | | Seq(5'⇒3') |
|---|---|---|
| Fragment-1 | F | GCAGATTCATTGGTCAGAGAACAXCCTGACTAGCATATAAGAAGCTTTCAGCAAGTGCAGACTA |
| | R | AGGTTTTTCTAGAGTGGACACGGATTTTTGCATCGTAAGCAAAAATGATTGGTTGAACATGAA |
| Fragment-2 | F | CATCTAAAGCGTTCCCAGTTCCAXGCTTGCCCCTGGGCTTTTATAAGTCGTCACGGAGA |
| | R | AGGTTTTTCTAGAGTGGACACGGCAAATTTGAGACGGCTCCAACTCAGTAATCTTTTTCCAAA |
| Fragment-3 | F | GTCCGAAATTACAACGAAGCGAAXGAAATGGGTTCCCTGGTTGTCAGCCTCTGCGAAGTA |
| | R | AGGTTTTTCTAGAGTGGACACGGAACCGGCCCAGCTTCGACGGTATCCTCTACTACTA |
| Fragment-4 | F | GCATGTCATAAATCACTGGGCTAXCATAGCAAGTTGTTCATTGTTGTAACCCTGGTACCTG |
| | R | AGGTTTTTCTAGAGTGGACACGGTTTAAGTGCAAATTTATTTCGCCTCCAAAGGGACCTCCCA |

**[Table 20]**

| Name | Seq(5'⇒3') |
|---|---|
| R' primer | [biotin]-AGGTTTTTCTAGAGTGGACACGG |

The linking sites X are propyleneoxy chains, introduced with phosphoramidite (spacer phosphoramidite C3, GlenResearch).

The composition in the amplification reaction was as follows, and the thermal cycle conditions were 15 minutes at 95°C followed by 40 cycles, each of which comprises 30 seconds at 95°C, 1 second at 80°C and 6 minutes at 64°C, followed by cooling to 10°C.

**(Composition)**

| | |
|---|---|
| 2×Qiagen multiplex PCR master mix | 5.0µl |
| Primer mix (F,R, 500nM each) | 0.5µl |
| R'primer(2µM) | 0.5µl |
| dH₂O | 3.0µl |
| Genome DNA | 0.5µl |
| Total | 10.0µl |

Mixtures of the primer sets for each of the amplification product singles 1 to 4 shown in **Table** 19 and a mixture of all of the primer sets for the singles 1 to 4 were prepared, for a total of 5 mixes.

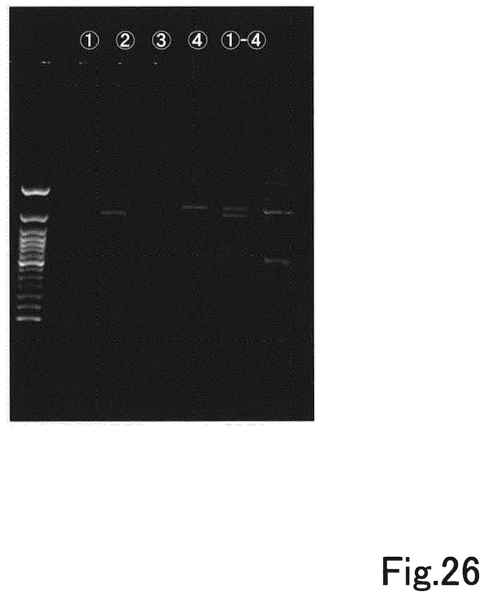

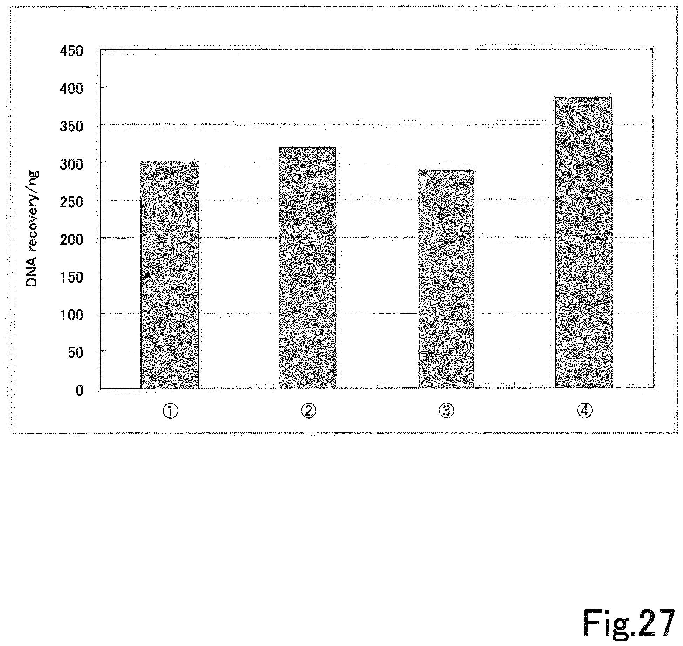

The amplification products obtained by amplification were purified with a QIAGEN MinElute PCR Purification Kit, and amplification of fragments of the desired length was confirmed by agarose electrophoresis. The yield of each amplification product after purification was also confirmed. The results are shown in **FIG.** 26 and **FIG.** 27.

As shown in **FIG.** 26 and **FIG.** 27, there was no great difference between the single-stranded single type and the double-stranded single type in terms of amplified amount of the amplification product or yield after purification.

### (3) Detection using chromatography unit

A hybridization reaction and detection were carried out by nucleic acid chromatography using the partially double-stranded DNA amplified in (2). The operations were as follows for the single-stranded singles and double-stranded singles, respectively.

**(Developing solution for single-stranded singles)**

| | |
|---|---|
| PBS | 30.0µl |
| Latex solution | 5.0µl |
| Amplification reaction solution | 10.0µl |
| Millipore water | 5.0µl |
| Total | 50µl |

The developing solution was prepared by mixing PBS (phosphate-buffered saline) with a latex solution and amplification reaction solution (5 kinds). For the latex storage solution, polystyrene latex beads containing a blue colorant were coated within avidin (streptavidin), and prepared with PBS to a specific concentration.

### (Hybridization step)

50 µl of each of the developing solutions above was added to a 0.2 ml tube, and the bottom ends of each of the chromatography units (8-type and 4-type) were inserted to initiate a hybridization reaction by chromatography. All of the developing solution was absorbed in about 20 minutes, and the hybridization reaction by chromatography was completed. After completion of the reaction, the chromatography units were air dried, and the reaction sites were confirmed with the naked eye and photographed.

### (Detection step)

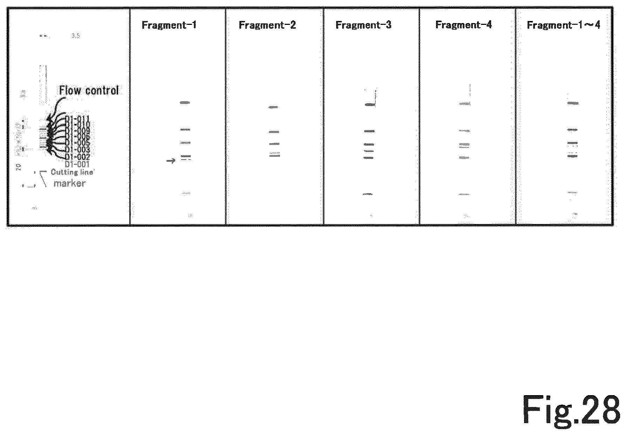

The presence or absence of coloration in the array after drying was confirmed visually. The results are shown in **FIG.** 28. As shown in **FIG.** 28, dark coloration was observed at the intended site for each amplification product, confirming the effects of this method.

### [Example 8]

### (1) Preparation of membrane-type DNA chromatography

DNA probe solutions comprising the base sequences shown in the following **table** were spotted on Merck Millipore Hi-Flow Plus membrane plates (60 mm x 600 mm) and arrayed as shown in **FIG.** 19, using a NGK Insulators, Ltd. Geneshot® spotter with the discharge unit (inkjet method) described in Japanese Patent Application Publication No. 2003-75305. For the synthetic oligo-DNA sequences, the 8 sequences shown in the following **table** out of the 100 sequences D1_1 to D1_100 described in the Supplementary **Table** 1 of the literature (Analytical Biochemistry 364 (2007) 78-85) were used as probes. The sequences were modified with amino groups at the 3' end of the oligonucleotides for use as probes. Further, three position marker regions were formed using red pigment as shown **FIG.** 19.

**[Table 21]**

| Name | Seq(5'⇒3') |
|---|---|
| D1-001 | TGTTCTCTGACCAATGAATCTGC |
| D1-002 | TGGAACTGGGAACGCTTTAGATG |
| D1-003 | TTCGCTTCGTTGTAATTTCGGAC |
| D1-005 | TAGCCCAGTGATTTATGACATGC |
| D1-006 | CGCTCTGGTTACTATTGGACGTT |
| D1-009 | GGGGGGTACTTCATACAAGATGC |
| D1-010 | GAGTAGCAGGCAAATACCCTAGA |
| D1-011 | GCCTATTAAGGTCTACGTCATCG |

After the probes were spotted, they were fixed by exposure to UV light at about 300 mJ/cm² using a Spectroline Co. UV irradiation device (XL-1 500 UV Crosslinker) and chromatography units were obtained comprising the probe regions of 8 different probes together with 3 different position marker regions.

### (2) Amplification of target nucleic acid

Human DNA (commercial product from Cosmo Bio) was used as the genome DNA for amplification. Primer sets consisting of the base sequences shown in **Table** 22 below were used as the primers. That is, each R primer was provided with a tag sequence complementary to a probe (D1-001, 002, 003 and 005), a linking site X and a first recognition sequence, and each F primer was provided with a tag sequence and a second recognition sequence, and amplification was performed using these. During amplification, a primer (R' primer; **Table** 23) modified with biotin at the 3' end of the tag complementary sequence in the R primer was added in addition to the aforementioned two kinds of primers so that biotin would be incorporated into the double-stranded DNA ends after amplification, resulting in partially double-stranded DNA having a tag sequence as a single strand.

**[Table 22]**

| Name | | Seq(5⇒3') |
|---|---|---|
| Fragment-1 | F | GCAGATTCATTGGTCAGAGAACAXCCTGACTAGCATATAAGAAGCTTTCAGCAAGTGCAGACTA |
| | R | AGGTTTTTCTAGAGTGGACACGGATTTTTGCATCGTAAGCAAAAATGATTGGTTGAACATGAA |
| Fragment-2 | F | CATCTAAAGCGTTCCCAGTTCCAXGCTTGCCCCTGGGCTTTTATAAGTCGTCACGGAGA |
| | R | AGGTTTTTCTAGAGTGGACACGGCAAATTTGAGACGGCTCCAACTCAGTAATCTTTTTCCAAA |
| Fragment-3 | F | GTCCGAAATTACAACGAAGCGAAXGAAATGGGTTCCCTGGTTGTCAGCCTCTGCGAAGTA |
| | R | AGGTTTTTCTAGAGTGGACACGGAACCGGCCCAGCTTCGACGGTATCCTCTACTACTA AGGTTTTTCTAGAGTGGACACGGAACCGGCCCAGCTTCGACGGTATCCTCTACTACTA |
| Fragment-4 | F | GCATGTCATAAATCACTGGGCTAXCATAGCAAGTTGTTCATTGTTGTAACCCTGGTACCTG |
| | R | AGGTTTTTCTAGAGTGGACACGGTTTAAGTGCAAATTTATTTCGCCTCCAAAGGGACCTCCCA |

**[Table 23]**

| Name | Seq(5⇒3') |
|---|---|
| R' primer | CCGTGTCCACTCTAGAAAAACCT-[biotin] |

The linking sites X are propyleneoxy chains, introduced with phosphoramidite (spacer phosphoramidite C3, GlenResearch).

The composition in the amplification reaction was as follows, and the thermal cycle conditions were 15 minutes at 95°C followed by 40 cycles, each of which comprises 30 seconds at 95°C, 1 second at 80°C and 6 minutes at 64°C, followed by cooling to 10°C.

**(Composition)**

| | |
|---|---|
| 10×TITANIUM Taq DNA Polymerase | 0.2µl |
| 50×dNTP Mix | 0.2µl |
| 10×TITANIUM Taq PCR buffer | 1.0µl |
| Primer mix(F,R, 500nM each) | 0.5µl |
| R'primer -(2µM) | 0.5µl |
| dH₂O | 7.1µl |
| Genomic DNA | 0.5µl |
| Total | 10.0µl |

Mixtures of the primer sets for each of the amplification product singles 1 to 4 shown in **Table** 22 and a mixture of all of the primer sets for the singles 1 to 4 were prepared, for a total of 5 mixes.

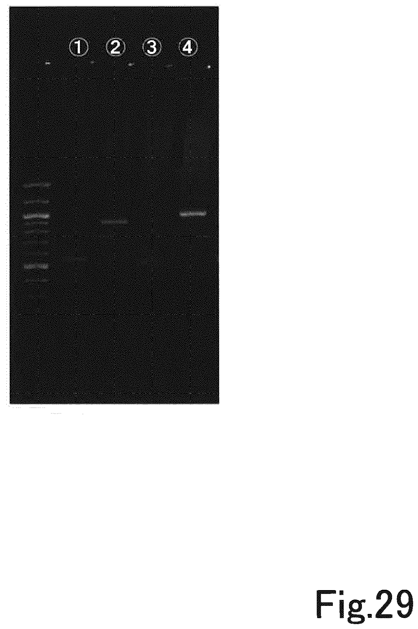

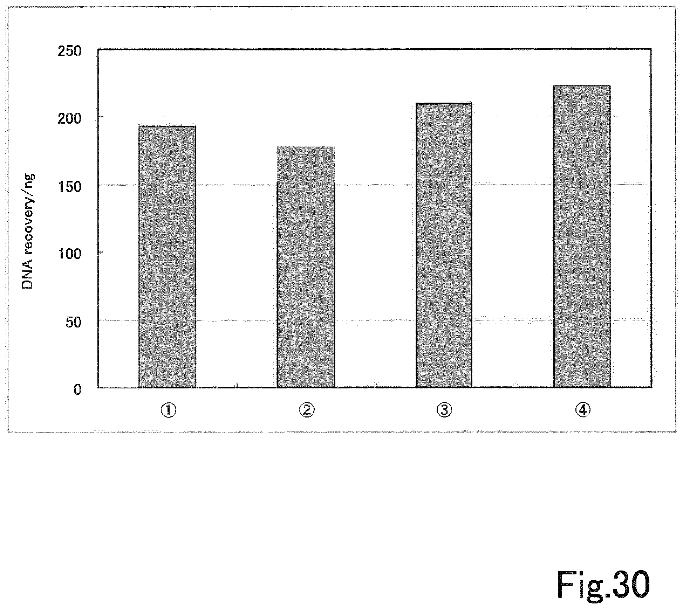

The amplification products obtained by amplification were purified with a QIAGEN MinElute PCR Purification Kit, and amplification of fragments of the desired length was confirmed by agarose electrophoresis. The yield of each amplification product after purification was also confirmed. The results are shown in **FIG.** 29 and **FIG.**30.

As shown in **FIG.** 29 and **FIG.** 30, there was no great difference between the single-stranded single type and the double-stranded single type in terms of amplified amount of the amplification product or yield after purification.

### (3) Detection using chromatography unit

A hybridization reaction and detection were carried out by nucleic acid chromatography using the partially double-stranded DNA amplified in (2). The operations were as follows for the single-stranded singles and double-stranded singles, respectively.

**(Developing solution for single-stranded singles)**

| | |
|---|---|
| PBS | 30.0µl |
| Latex solution | 5.0µl |
| Amplification reaction solution | 10.0µl |
| Millipore water | 5.0µl |
| Total | 50µl |

The developing solution was prepared by mixing PBS (phosphate-buffered saline) with a latex solution and amplification reaction solution (5 kinds). For the latex storage solution, polystyrene latex beads containing a blue colorant were coated within avidin (streptavidin), and prepared with PBS to a specific concentration.

### (Hybridization step)

50 µl of each of the developing solutions above was added to a 0.2 ml tube, and the bottom ends of each of the chromatography units (8-type and 4-type) were inserted to initiate a hybridization reaction by chromatography. All of the developing solution was absorbed in about 20 minutes, and the hybridization reaction by chromatography was completed. After completion of the reaction, the chromatography units were air dried, and the reaction sites were confirmed with the naked eye and photographed.

### (Detection step)

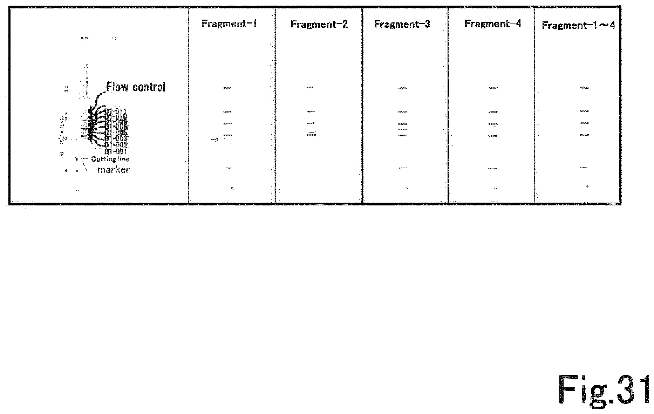

The presence or absence of coloration in the array after drying was confirmed visually. The results are shown in **FIG.** 31. As shown in **FIG.** 31, dark coloration was observed at the intended site for each amplification product, confirming the effects of this method.

### [Example 9]

In the following examples, base sequence analysis (one-pass sequencing) is performed on 18 amplified products obtained Example 1. The base sequence analysis was performed as explained below.

Both strands of each amplified sample were subjected to base sequence analysis (one-pass sequencing) at Takara Bio, and base sequence results were obtained including the primer sequences. The results are shown in **Table**s 24 to 26 below (one strand only).

**[Table 24]**

| Amplified product | Results (Caps:identical to primer sequence or complementary thereto) | length(bp) |
|---|---|---|
| P1-1 | | 523 |
| P1-2 | | 867 |
| P1-3 | | 504 |
| P1-4 | | 953 |
| P1-5 | | 502 |
| P1-6 | | 940 |

**[TABLE 25]**

| Amplified product | Results (Caps:identical to primer sequence or complementary thereto) | length(bp) |
|---|---|---|
| P2-1 | | 569 |
| P2-2 | | 913 |
| P2-3 | | 550 |
| P2-4 | | 999 |
| P2-5 | | 548 |
| P2-6 | | 986 |

**[TABLE 26]**

| Amplified product | Results (Caps:identical to primer sequence or complementary thereto) | length(bp) |
|---|---|---|
| P3-1 | | 523 |
| P3-2 | | 867 |
| P3-3 | | 504 |
| P3-4 | | 953 |
| P3-5 | | 502 |
| P3-6 | | 940 |

It was confirmed that the complementary strands of the tag sequences were amplified using the P2 primers. On the other hand, the complement strands of the tag sequences were not amplified with the P3 primers, and instead the same sequences as those obtained with the P1 primers were amplified as double strands. This confirms that when using P3 primers, the tag sequence part is retained as a single strand, potentially contributing to more efficient hybridization.

### [Sequence table free text]

SEQ ID NOS:1∼100: Probes
[Sequence **Table**s]

## Claims

1. A method for detecting a target nucleic acid,
the method comprising:
a hybridization step in which one or two or more partially double-stranded nucleic acids associated with one or two or more target nucleic acids are brought into contact with one or two or more probes that are on a solid phase body carrier and are associated with the one or two or more target nucleic acids, under conditions that allow hybridization, wherein the hybridization step is performed by nucleic acid chromatography; and
a detection step in which the hybridization product produced in the hybridization step is detected, wherein
each of the one or two or more partially double-stranded nucleic acids has a single-stranded tag part at only the 5' end of a first strand, which consists solely of natural nucleic acids and which is a tag sequence capable of hybridizing specifically with one of the probes and has a label or label binding substance at the 5' end of a second strand,
before the hybridization step an amplification step in which the partially double-stranded nucleic acid is obtained as a product of an amplification reaction performed on a target nucleic acid using a first primer having the tag sequence and a first recognition sequence that recognizes a first base sequence in the target nucleic acid and having a linking site capable of inhibiting or arresting a DNA polymerase reaction disposed between the tag sequence and the first recognition sequence, and a second primer having a second recognition sequence that recognizes a second base sequence in the target nucleic acid and the label or label binding substance;
wherein the linking site comprises an optionally substituted alkylene chain with an element number of 2 to 40, adjoining a nucleotide in the primer via a phosphate diester bond; and
wherein the linking site is represented by the following formula:
5'-O-CₘH₂ₘ-O-3' Formula (1)
(where 5' represents the oxygen atom of a phosphate diester bond at the 5' end, 3' represents the phosphorus atom of a phosphate diester bond at the 3' end, and m is an integer from 3 to 12).

2. The method according to Claim 1, wherein the hybridization step is performed by preparing a hybridization solution containing the one or two or more partially double-stranded nucleic acids together with a label for binding to the label binding substance, and bringing this hybridization solution into contact with at least a part of the solid phase body carrier.

3. The method according to Claim 1, wherein the hybridization step is performed by bringing a hybridization solution comprising an amplification reaction solution containing the amplification product of the amplification step into contact with a part of the solid phase body carrier.

4. The method according to Claim 1, wherein the hybridization step is performed by bringing the hybridization solution comprising the amplification reaction solution containing the one or two or more partially double-stranded nucleic acids as the amplification products carrying the label bound in advance to the label binding substance into contact with the part of the solid phase body carrier.

5. The method according to any one of Claims 1, 3 and 4, wherein the hybridization step is performed by preparing a developing medium comprising an amplification reaction solution containing the amplification product of the amplification step together with a label for binding to the label binding substance, and bringing this developing medium into contact with at least a part of the solid phase body carrier.

6. The method according to Claim 5, wherein the amplification step is performed in a cavity, the developing medium is prepared by supplying at least the label to the cavity holding the amplification reaction solution, and the developing medium is brought into contact with part of the solid phase body carrier in the cavity.

7. The method according to any one of Claims 1 to 6, wherein
the label binding substance is one or two or more selected from the group consisting of the antibodies in antigen-antibody reactions and biotin, digoxigenin, and FITC and other haptens, and
the label is provided with a site capable of binding with the label binding substance, and is a label that uses one or two or more selected from fluorescence, radioactivity, enzymes, phosphorescence, chemical luminescence and coloration.

8. The method according to any one of claims 1 to 7, wherein the second primer has a linking site disposed between the label-binding region and the second recognition sequence.

9. The method according to any one of claims 1 to 8, wherein the linking site does not contain natural bases or natural base derivatives that pair with natural bases.

10. The method according to any one of claims 1 to 9, wherein
the amplification step is a step of performing nucleic acid amplification using multiple primer sets each formed of the first primer and the second primer, so as to allow detection by a plurality of the detection probes pre-associated with a plurality of the target nucleic acids,
the hybridization step is a step of bringing a plurality of the amplification fragment obtained in the amplification step into contact with the plurality of detection probes so as to allow hybridization, and
the detection step is a step of detecting products of hybridization between the plurality of amplification fragments and the plurality of detection probes on the solid phase body carrier.

11. The method according to any one of claims 1 to 10, wherein the specific sequence of the detection probe is selected from the base sequences represented by SEQ ID NOS:1 to 100 and complementary sequences thereof.

12. The method according to any one of claims 1 to 11, wherein the specific sequence of the detection probe is selected from the base sequences represented by the SEQ ID NOS in the following **table** and complementary sequences thereof.
**[Table 27]**
| Name | Seq(5→3') | SEQ.ID. |
|---|---|---|
| D1-001 | TGTTCTCTGACCAATGAATCTGC | 1 |
| D1-002 | TGGAACTGGGAACGCTTTAGATG | 2 |
| D1-003 | TTCGCTTCGTTGTAATTTCGGAC | 3 |
| D1-005 | TAGCCCAGTGATTTATGACATGC | 5 |
| D1-006 | CGCTCTGGTTACTATTGGACGTT | 6 |
| D1-010 | GAGTAGCAGGCAAATACCCTAGA | 10 |
| D1-012 | AGTCATACAGTGAGGACCAAATG | 12 |
| D1-014 | TGCTCACTTACATTACGTCCATG | 14 |
| D1-016 | AGGTCCGGTAGTAATTTAGGTGC | 16 |
| D1-020 | TATTCTACCAACGACATCACTGC | 20 |
| D1-023 | CATCTCCAAGAATTGACCCACCA | 23 |
| D1-025 | GAAGGATCGCTTTTATCTGGCAT | 25 |
| D1-026 | CATTTGTCAGGTACAGTCCACTT | 26 |
| D1-027 | GCCCACACTCTTACTTATCGACT | 27 |
| D1-030 | CCGTCTGGGTTAAAGATTGCTAG | 30 |
| D1-035 | ATGCCGTTGTCAAGAGTTATGGT | 35 |
| D1-038 | CGCGACATTTAGTCCAGGAGATG | 38 |
| D1-040 | AGACAATTAGAATCAGTGCCCCT | 40 |
| D1-041 | GCATTGAGGTATTGTTGCTCCCA | 41 |
| D1-044 | GAGTCCGCAAAAATATAGGAGGC | 44 |
| D1-045 | GCCTCACATAACTGGAGAAACCT | 45 |
| D1-050 | GGGATAGGTATTATGCTCCAGCC | 50 |
| D1-052 | GCCTATATGAACCAAGCCACTGC | 52 |
| D1-062 | CTAGCACAATTAATCAATCCGCC | 62 |
| D1-064 | GCCTATAGTGTCGATTGTCCTCG | 64 |
| D1-065 | CGATCACGGATTAATGTCACCCC | 65 |
| D1-077 | CGCAGTTTGCAAGAACGAACAAA | 77 |
| D1-084 | CCGTGTGTATGAGTATGACAGCA | 84 |
| D1-089 | GAGTCGAAGACCTCCTCCTACTC | 89 |
| D1-090 | ATGCCAATATGTACTCGTGACTC | 90 |
| D1-095 | TGCCGGTTATACCTTTAAGGACG | 95 |
| D1-097 | CGCGGTACTATTAGAAAGGGCTA | 97 |
| D1-100 | TGCAGTGTAAGCAACTATTGTCT | 100 |

13. A method according to any one of claims 1 to 12, wherein the hybridization step is a step of supplying a liquid containing the amplified fragment as a mobile phase to a solid phase body containing a plurality of the detection probe, and expanding the mobile phase in the solid phase body.

14. A nucleic acid amplification kit, containing a first nucleic acid amplification primer having a single stranded tag sequence at only the 5' end which consists solely of natural nucleic acids, a first recognition sequence that recognizes a first base sequence in a target nucleic acid, and a linking site capable of inhibiting or arresting a DNA polymerase reaction disposed between the tag sequence and the first recognition sequence, wherein the linking site comprises an optionally substituted alkylene chain with an element number of 2 to 40, adjoining a nucleotide in the primer via a phosphate diester bond, and wherein the linking site is represented by the following formula:
5'-O-CₘH₂ₘ-O-3' Formula (1)
(where 5' represents the oxygen atom of a phosphate diester bond at the 5' end, 3' represents the phosphorus atom of a phosphate diester bond at the 3' end, and m is an integer from 3 to 12); and
a second nucleic acid amplification primer having a second recognition sequence that recognizes a second base sequence in a target nucleic acid and a label or label binding substance at the 5' end.

15. A composition for probe hybridization, comprising a double-stranded DNA fragment having
a single-stranded tag part on the 5' side of a first strand, which consists solely of natural nucleic acids and which is a tag sequence capable of hybridizing specifically with the probe,
a double-stranded part formed by base pairing, and
a label or label binding substance at the 5' end of the second strand,
the first strand having a linking site capable of inhibiting or arresting a DNA polymerase reaction disposed between the tag sequence and the double-stranded part;
wherein the linking site comprises an optionally substituted alkylene chain with an element number of 2 to 40, adjoining a nucleotide in the primer via a phosphate diester bond; and
wherein the linking site is represented by the following formula:
5'-O-CₘH₂ₘ-O-3' Formula (1)
(where 5' represents the oxygen atom of a phosphate diester bond at the 5' end, 3' represents the phosphorus atom of a phosphate diester bond at the 3' end, and m is an integer from 3 to 12).

16. The method according to any one of claims 1 to 13, wherein m is 3 to 6.

17. The method according to claim **16** wherein m is 3.

## Patentansprüche

1. Verfahren zum Detektieren einer Zielnucleinsäure,
wobei das Verfahren Folgendes umfasst:
einen Hybridisierungsschritt, in dem eine oder zwei oder mehr teilweise doppelsträngige Nucleinsäuren, die mit einer oder zwei oder mehr Zielnucleinsäuren assoziiert sind, mit einer oder zwei oder mehr Sonden, die auf einem Festphasenkörperträger vorliegen und mit der einen oder den zwei oder mehr Zielnucleinsäuren assoziiert sind, unter Bedingungen in Kontakt gebracht werden, die Hybridisierung erlauben, wobei der Hybridisierungsschritt mittels Nucleinsäurechromatographie durchgeführt wird; und
einen Detektionsschritt, in dem das im Hybridisierungsschritt erzeugte Hybridisierungsprodukt detektiert wird, wobei
jede der einen oder zwei oder mehr teilweise doppelsträngigen Nucleinsäuren einen einzelsträngigen Markierungsteil nur am 5'-Ende eines ersten Strangs aufweist, der ausschließlich aus natürlichen Nucleinsäuren besteht und eine Markierungssequenz ist, die zur spezifischen Hybridisierung mit einer der Sonden in der Lage ist, und eine Markierung oder markierungsbindende Substanz am 5'-Ende eines zweiten Strangs aufweist,
vor dem Hybridisierungsschritt einen Amplifizierungsschritt, in dem die teilweise doppelsträngige Nucleinsäure als Produkt einer Amplifizierungsreaktion erhalten wird, die unter Verwendung eines ersten Primers mit der Markierungssequenz und einer ersten Erkennungssequenz, die eine erste Basensequenz in der Zielnucleinsäure erkennt, und einer Linkerstelle, die zu Hemmung oder Anhalten einer DNA-Polymerasereaktion in der Lage ist und zwischen der Markierungssequenz und der ersten Erkennungssequenz angeordnet ist, und eines zweiten Primers mit einer zweiten Erkennungssequenz, die eine zweite Basensequenz in der Zielnucleinsäure und die Markierung oder markierungsbindenden Substanz erkennt, auf einer Zielnucleinsäure durchgeführt wird;
wobei die Linkerstelle eine gegebenenfalls substituierte Alkylenkette mit einer Elementzahl von 2 bis 40 umfasst, die über eine Phosphatdiesterbindung an ein Nucleotid in dem Primer angrenzt; und
wobei die Linkerstelle durch die folgende Formel dargestellt ist:
5'-O-CₘH₂ₘ-O-3' Formel (1)
(wobei 5' für das Sauerstoffatom einer Phosphatdiesterbindung am 5'-Ende steht, 3' für das Phosphoratom einer Phosphatdiesterbindung am 3'-Ende steht und m eine ganze Zahl von 3 bis 12 ist).

2. Verfahren nach Anspruch 1, wobei der Hybridisierungsschritt durch Herstellen einer Hybridisierungslösung, die die eine oder zwei oder mehr teilweise doppelsträngigen Nucleinsäuren zusammen mit einer Markierung zur Bindung an die markierungsbindende Substanz enthält, und In-Kontakt-Bringen dieser Hybridisierungslösung mit zumindest einem Teil des Festphasenkörperträgers durchgeführt wird.

3. Verfahren nach Anspruch 1, wobei der Hybridisierungsschritt durch In-Kontakt-Bringen einer Hybridisierungslösung, umfassend eine Amplifizierungsreaktionslösung, die das Amplifizierungsprodukt des Amplifizierungsschritts enthält, mit einem Teil des Festphasenkörperträgers durchgeführt wird.

4. Verfahren nach Anspruch 1, wobei der Hybridisierungsschritt durch In-Kontakt-Bringen der Hybridisierungslösung, umfassend die Amplifizierungsreaktionslösung, die die eine oder zwei oder mehr teilweise doppelsträngigen Nucleinsäuren als Amplifizierungsprodukte enthält, die die zuvor an die markierungsbindende Substanz gebundene Markierung tragen, mit dem Teil des Festphasenkörperträgers durchgeführt wird.

5. Verfahren nach einem der Ansprüche 1, 3 und 4, wobei der Hybridisierungsschritt durch Herstellen eines Entwicklungsmediums, umfassend eine Amplifizierungsreaktionslösung, die das Amplifizierungsprodukt des Amplifizierungsschritts zusammen mit einer Markierung zum Binden an die markierungsbindende Substanz enthält, und In-Kontakt-Bringen des Entwicklungsmediums mit zumindest einem Teil des Festphasenkörperträgers durchgeführt wird.

6. Verfahren nach Anspruch 5, wobei der Amplifizierungsschritt in einer Vertiefung durchgeführt wird, das Entwicklungsmedium durch Zuführen zumindest der Markierung an die Vertiefung, die die Amplifizierungsreaktionslösung hält, hergestellt wird und das Entwicklungsmedium mit einem Teil des Festphasenkörperträgers in der Vertiefung in Kontakt gebracht wird.

7. Verfahren nach einem der Ansprüche 1 bis 6, wobei
die markierungsbindende Substanz ein oder zwei oder mehr aus der aus den Antikörpern in Antigen-Antikörper-Reaktionen und Biotin, Digoxigenin und FITC und anderen Haptenen bestehenden Gruppe ausgewählte ist und
die Markierung mit einer Stelle, die zur Bindung an die markierungsbindende Substanz in der Lage ist, bereitgestellt wird und eine Markierung ist, die eine oder zwei oder mehr aus Fluoreszenz, Radioaktivität, Enzymen, Phosphoreszenz, chemischer Lumineszenz und Färbung ausgewählte verwendet.

8. Verfahren nach einem der Ansprüche 1 bis 7, wobei der zweite Primer eine Linkerstelle aufweist, die zwischen der markierungsbindenden Region und der zweiten Erkennungssequenz angeordnet ist.

9. Verfahren nach einem der Ansprüche 1 bis 8, wobei die Linkerstelle keine natürlichen Basen oder natürlichen Basenderivate, die sich mit natürlichen Basen paaren, enthält.

10. Verfahren nach einem der Ansprüche 1 bis 9, wobei
der Amplifizierungsschritt ein Schritt des Durchführens von Nucleinsäureamplifizierung unter Verwendung mehrerer Primersätze ist, die jeweils aus dem ersten Primer und dem zweiten Primer gebildet sind, um Detektion durch eine Vielzahl der Detektionssonden zu erlauben, die mit einer Vielzahl der Zielnucleinsäuren vorassoziiert sind,
der Hybridisierungsschritt ein Schritt des In-Kontakt-Bringens einer Vielzahl der im Amplifizierungsschritt erhaltenen Amplifizierungsfragmente mit der Vielzahl von Detektionssonden ist, um Hybridisierung zu erlauben, und
der Detektionsschritt ein Schritt des Detektierens von Hybridisierungsprodukten zwischen der Vielzahl von Amplifizierungsfragmenten und der Vielzahl von Detektionssonden auf dem Festphasenkörperträger ist.

11. Verfahren nach einem der Ansprüche 1 bis 10, wobei die spezifische Sequenz der Detektionssonde aus den Basensequenzen, die durch die Seq.-IDs Nr. 1 bis 100 dargestellt sind, und komplementären Sequenzen davon ausgewählt ist.

12. Verfahren nach einem der Ansprüche 1 bis 11, wobei die spezifische Sequenz der Detektionssonde aus den Basensequenzen, die durch die Seq.-ID-Nr. in der nachstehenden Tabelle dargestellt sind, und komplementären Sequenzen davon ausgewählt ist:
**Tabelle 27**
| Bezeichn. | Se(5→3') | SEQ.ID. |
|---|---|---|
| D1-001 | TGTTCTCTGACCAATGAATCTGC | 1 |
| D1-002 | TGGAACTGGGAACGCTTTAGATG | 2 |
| D1-003 | TTCGCTTCGTTGTAATTTCGGAC | 3 |
| D1-005 | TAGCCCAGTGATTTATGACATGC | 5 |
| D1-006 | CGCTCTGGTTACTATTGGACGTT | 6 |
| D1-010 | GAGTAGCAGGCAAATACCCTAGA | 10 |
| D1-012 | AGTCATACAGTGAGGACCAAATG | 12 |
| D1-014 | TGCTCACTTACATTACGTCCATG | 14 |
| D1-016 | AGGTCCGGTAGTAATTTAGGTGC | 16 |
| D1-020 | TATTCTACCAACGACATCACTGC | 20 |
| D1-023 | CATCTCCAAGAATTGACCCACCA | 23 |
| D1-025 | GAAGGATCGCTTTTATCTGGCAT | 25 |
| D1-026 | CATTTGTCAGGTACAGTCCACTT | 26 |
| D1-027 | GCCCACACTCTTACTTATCGACT | 27 |
| D1-030 | CCGTCTGGGTTAAAGATTGCTAG | 30 |
| D1-035 | ATGCCGTTGTCAAGAGTTATGGT | 35 |
| D1-038 | CGCGACATTTAGTCCAGGAGATG | 38 |
| D1-040 | AGACAATTAGAATCAGTGCCCCT | 40 |
| D1-041 | GCATTGAGGTATTGTTGCTCCCA | 41 |
| D1-044 | GAGTCCGCAAAAATATAGGAGGC | 44 |
| D1-045 | GCCTCACATAACTGGAGAAACCT | 45 |
| D1-050 | GGGATAGGTATTATGCTCCAGCC | 50 |
| D1-052 | GCCTATATGAACCAAGCCACTGC | 52 |
| D1-062 | CTAGCACAATTAATCAATCCGCC | 62 |
| D1-064 | GCCTATAGTGTCGATTGTCCTCG | 64 |
| D1-065 | CGATCACGGATTAATGTCACCCC | 65 |
| D1-077 | CGCAGTTTGCAAGAACGAACAAA | 77 |
| D1-084 | CCGTGTGTATGAGTATGACAGCA | 84 |
| D1-089 | GAGTCGAAGACCTCCTCCTACTC | 89 |
| D1-090 | ATGCCAATATGTACTCGTGACTC | 90 |
| D1-095 | TGCCGGTTATACCTTTAAGGACG | 95 |
| D1-097 | CGCGGTACTATTAGAAAGGGCTA | 97 |
| D1-100 | TGCAGTGTAAGCAACTATTGTCT | 100 |

13. Verfahren nach einem der Ansprüche 1 bis 12, wobei der Hybridisierungsschritt ein Schritt des Zuführens einer Flüssigkeit, die das amplifizierte Fragment als mobile Phase enthält, zu einem Festphasenkörper, der eine Vielzahl der Detektionssonden enthält, und Expandierens der mobilen Phase in dem Festphasenkörper ist.

14. Nucleinsäureamplifizierungsset, enthaltend
einen ersten Nucleinsäureamplifizierungsprimer mit einer einzelsträngigen Markierungssequenz nur am 5'-Ende, die ausschließlich aus natürlichen Nucleinsäuren besteht, einer ersten Erkennungssequenz, die eine erste Basensequenz in einer Zielnucleinsäure erkennt, und einer Linkerstelle, die zu Hemmung oder Anhalten einer DNA-Polymerasereaktion in der Lage ist und zwischen der Markierungssequenz und der ersten Erkennungssequenz angeordnet ist, wobei die Linkerstelle eine gegebenenfalls substituierte Alkylenkette mit einer Elementzahl von 2 bis 40 umfasst, die über eine Phosphatdiesterbindung an ein Nucleotid in dem Primer angrenzt, und wobei die Linkerstelle durch die folgende Formel dargestellt ist:
5'-O-CₘH₂ₘ-O-3' Formel (1)
(wobei 5' für das Sauerstoffatom einer Phosphatdiesterbindung am 5'-Ende steht, 3' für das Phosphoratom einer Phosphatdiesterbindung am 3'-Ende steht und m eine ganze Zahl von 3 bis 12 ist); und
einen zweiten Nucleinsäureamplifizierungsprimer mit einer zweiten Erkennungssequenz, die eine zweite Basensequenz in einer Zielnucleinsäure erkennt, und einer Markierung oder markierungsbindenden Substanz am 5'-Ende.

15. Zusammensetzung zur Sondenhybridisierung, umfassend ein doppelsträngiges DNA-Fragment mit
einem einzelsträngigen Markierungsteil an der 5'-Seite eines ersten Strangs, der ausschließlich aus natürlichen Nucleinsäuren besteht und eine Markierungssequenz ist, die zur spezifischen Hybridisierung mit der Sonde in der Lage ist,
einem doppelsträngigen Teil, der durch Basenpaarung gebildet wird, und einer Markierung oder markierungsbindenden Substanz am 5'-Ende des zweiten Strangs,
wobei der erste Strang eine Linkerstelle aufweist, die zu Hemmung oder Anhalten einer DNA-Polymerasereaktion in der Lage ist und zwischen der Markierungssequenz und dem doppelsträngigen Teil angeordnet ist;
wobei die Linkerstelle eine gegebenenfalls substituierte Alkylenkette mit einer Elementzahl von 2 bis 40 umfasst, die über eine Phosphatdiesterbindung an ein Nucleotid in dem Primer angrenzt; und
wobei die Linkerstelle durch die folgende Formel dargestellt ist:
5'-O-CₘH₂ₘ-O-3' Formel (1)
(wobei 5' für das Sauerstoffatom einer Phosphatdiesterbindung am 5'-Ende steht, 3' für das Phosphoratom einer Phosphatdiesterbindung am 3'-Ende steht und m eine ganze Zahl von 3 bis 12 ist).

16. Verfahren nach einem der Ansprüche 1 bis 13, wobei m = 3 bis 6 ist.

17. Verfahren nach Anspruch 16, wobei m = 3 ist.

## Revendications

1. Procédé de détection d'un acide nucléique cible,
le procédé comprenant :
une étape d'hybridation dans laquelle un ou deux acides nucléiques partiellement double brin ou plus associés à un ou deux acides nucléiques cibles ou plus sont mis en contact avec une ou deux sondes ou plus qui sont sur un support de corps en phase solide et sont associées aux un ou deux acides nucléiques cibles ou plus, dans des conditions qui permettent une hybridation, où l'étape d'hybridation est réalisé par une chromatographie des acides nucléiques ; et
une étape de détection dans laquelle le produit d'hybridation produit à l'étape d'hybridation est détecté, où
chacun des un ou deux acides nucléiques partiellement double brin ou plus comporte une partie de marqueur simple brin uniquement à l'extrémité 5' d'un premier brin, qui consiste uniquement en des acides nucléiques naturels et qui est une séquence de marqueur capable de spécifiquement s'hybrider à une des sondes et comporte un marqueur ou une substance se liant à un marqueur à l'extrémité 5' d'un second brin,
avant l'étape d'hybridation, une étape d'amplification dans laquelle l'acide nucléique partiellement double brin est obtenu en tant que produit d'une réaction d'amplification réalisée sur un acide nucléique cible en utilisant une première amorce comportant la séquence de marqueur et une première séquence de reconnaissance qui reconnaît une première séquence de bases dans l'acide nucléique cible et comportant un site de liaison capable d'inhiber ou d'arrêter une réaction d'ADN polymérase disposé entre la séquence de marqueur et la première séquence de reconnaissance, et une deuxième amorce comportant une deuxième séquence de reconnaissance qui reconnaît une deuxième séquence de bases dans l'acide nucléique cible et le marqueur ou la substance se liant à un marqueur ;
où le site de liaison comprend une chaîne alkylène facultativement substituée ayant un nombre d'éléments compris entre 2 et 40, adjacente à un nucléotide dans l'amorce via une liaison phosphodiester ; et
où le site de liaison est représenté par la formule suivante :
5'-O-CₘH₂ₘ-O-3' Formule (1)
(dans laquelle 5' représente l'atome d'oxygène d'une liaison phosphodiester à l'extrémité 5', 3' représente l'atome de phosphore d'une liaison phosphodiester à l'extrémité 3', et m est un nombre entier compris entre 3 et 12).

2. Procédé selon la revendication 1, dans lequel l'étape d'hybridation est réalisée en préparant une solution d'hybridation contenant les un ou deux acides nucléiques partiellement double brin ou plus avec un marqueur pour une liaison à la substance se liant à un marqueur, et en mettant cette solution d'hybridation en contact avec au moins une partie du support de corps en phase solide.

3. Procédé selon la revendication 1, dans lequel l'étape d'hybridation est réalisée en mettant une solution d'hybridation comprenant une solution de réaction d'amplification contenant le produit d'amplification de l'étape d'amplification en contact avec une partie du support de corps en phase solide.

4. Procédé selon la revendication 1, dans lequel l'étape d'hybridation est réalisée en mettant la solution d'hybridation comprenant la solution de réaction d'amplification contenant les un ou deux acides nucléiques partiellement double brin ou plus en tant que produits d'amplification comportant le marqueur lié à l'avance à la substance se liant à un marqueur en contact avec la partie du support de corps en phase solide.

5. Procédé selon l'une quelconque des revendications 1, 3 et 4, dans lequel l'étape d'hybridation est réalisée en préparant un milieu de développement comprenant une solution de réaction d'amplification contenant le produit d'amplification de l'étape d'amplification avec un marqueur pour une liaison à la substance se liant à un marqueur, et en mettant ce milieu de développement en contact avec au moins une partie du support de corps en phase solide.

6. Procédé selon la revendication 5, dans lequel l'étape d'amplification est réalisée dans une cavité, le milieu de développement est préparé en introduisant au moins le marqueur dans la cavité contenant la solution de réaction d'amplification, et le milieu de développement est mis en contact avec une partie du support de corps en phase solide dans la cavité.

7. Procédé selon l'une quelconque des revendications 1 à 6, dans lequel
la substance de liaison à un marqueur est une ou deux substances ou plus sélectionnées dans le groupe consistant en les anticorps dans des réactions antigène-anticorps et la biotine, la digoxigénine, et le FITC et d'autres haptènes, et
le marqueur comporte un site capable de liaison à la substance se liant à un marqueur, et est un marqueur qui utilise une ou deux éléments ou plus sélectionnés parmi la fluorescence, la radioactivité, des enzymes, la phosphorescence, la luminescence chimique et la coloration.

8. Procédé selon l'une quelconque des revendications 1 à 7, dans lequel la deuxième amorce comporte un site de liaison disposé entre la région se liant à un marqueur et la deuxième séquence de reconnaissance.

9. Procédé selon l'une quelconque des revendications 1 à 8, dans lequel le site de liaison ne contient pas de bases naturelles ou de dérivés de base naturelle qui s'apparient avec des bases naturelles.

10. Procédé selon l'une quelconque des revendications 1 à 9, dans lequel
l'étape d'amplification est une étape de réalisation d'une amplification d'acide nucléique en utilisant de multiples jeux d'amorces constitués chacun de la première amorce et la deuxième amorce, de sorte à permettre une détection par une pluralité des sondes de détection pré-associées à une pluralité des acides nucléiques cibles,
l'étape d'hybridation est une étape de mise en contact d'une pluralité du fragment d'amplification obtenu à l'étape d'amplification avec la pluralité de sondes de détection de sorte à permettre une hybridation, et
l'étape de détection est une étape de détection des produits d'hybridation entre la pluralité de fragments d'amplification et la pluralité de sondes de détection sur le support de corps en phase solide.

11. Procédé selon l'une quelconque des revendications 1 à 10, dans lequel la séquence spécifique de la sonde de détection est sélectionnée parmi les séquences de bases représentées par SEQ ID NO: 1 à 100 et des séquences complémentaires de celles-ci.

12. Procédé selon l'une quelconque des revendications 1 à 11, dans lequel la séquence spécifique de la sonde de détection est sélectionnée parmi les séquences de bases représentées par les NOS de SEQ ID du **tableau** suivant et des séquences complémentaires de celles-ci.
**[Tableau 27]**
| Nom | Séq (5→3') | SEQ.ID. |
|---|---|---|
| D1-001 | TGTTCTCTGACCAATGAATCTGC | 1 |
| D1-002 | TGGAACTGGGAACGCTTTAGATG | 2 |
| D1-003 | TTCGCTTCGTTGTAATTTCGGAC | 3 |
| D1-005 | TAGCCCAGTGATTTATGACATGC | 5 |
| D1-006 | CGCTCTGGTTACTATTGGACGTT | 6 |
| D1-010 | GAGTAGCAGGCAAATACCCTAGA | 10 |
| D1-012 | AGTCATACAGTGAGGACCAAATG | 12 |
| D1-014 | TGCTCACTTACATTACGTCCATG | 14 |
| D1-016 | AGGTCCGGTAGTAATTTAGGTGC | 16 |
| D1-020 | TATTCTACCAACGACATCACTGC | 20 |
| D1-023 | CATCTCCAAGAATTGACCCACCA | 23 |
| D1-025 | GAAGGATCGCTTTTATCTGGCAT | 25 |
| D1-026 | CATTTGTCAGGTACAGTCCACTT | 26 |
| D1-027 | GCCCACACTCTTACTTATCGACT | 27 |
| D1-030 | CCGTCTGGGTTAAAGATTGCTAG | 30 |
| D1-035 | ATGCCGTTGTCAAGAGTTATGGT | 35 |
| D1-038 | CGCGACATTTAGTCCAGGAGATG | 38 |
| D1-040 | AGACAATTAGAATCAGTGCCCCT | 40 |
| D1-041 | GCATTGAGGTATTGTTGCTCCCA | 41 |
| D1-044 | GAGTCCGCAAAAATATAGGAGGC | 44 |
| D1-045 | GCCTCACATAACTGGAGAAACCT | 45 |
| D1-050 | GGGATAGGTATTATGCTCCAGCC | 50 |
| D1-052 | GCCTATATGAACCAAGCCACTGC | 52 |
| D1-062 | CTAGCACAATTAATCAATCCGCC | 62 |
| D1-064 | GCCTATAGTGTCGATTGTCCTCG | 64 |
| D1-065 | CGATCACGGATTAATGTCACCCC | 65 |
| D1-077 | CGCAGTTTGCAAGAACGAACAAA | 77 |
| D1-084 | CCGTGTGTATGAGTATGACAGCA | 84 |
| D1-089 | GAGTCGAAGACCTCCTCCTACTC | 89 |
| D1-090 | ATGCCAATATGTACTCGTGACTC | 90 |
| D1-095 | TGCCGGTTATACCTTTAAGGACG | 95 |
| D1-097 | CGCGGTACTATTAGAAAGGGCTA | 97 |
| D1-100 | TGCAGTGTAAGCAACTATTGTCT | 100 |

13. Procédé selon l'une quelconque des revendications 1 à 12, dans lequel l'étape d'hybridation est une étape de mise à disposition d'un liquide contenant le fragment amplifié en tant que phase mobile sur un corps en phase solide contenant une pluralité de la sonde de détection, et d'expansion de la phase mobile dans le corps en phase solide.

14. Kit d'amplification d'acide nucléique, contenant
une première amorce d'amplification d'acide nucléique comportant une séquence de marqueur simple brin seulement à l'extrémité 5' qui consiste uniquement en des acides nucléiques naturels, une première séquence de reconnaissance qui reconnaît une première séquence de bases dans un acide nucléique cible, et un site de liaison capable d'inhiber ou d'arrêter une réaction d'ADN polymérase disposé entre la séquence de marqueur et la première séquence de reconnaissance, où le site de liaison comprend une chaîne alkylène facultativement substituée ayant un nombre d'éléments compris entre 2 et 40, adjacente à un nucléotide dans l'amorce via une liaison phosphodiester, et où le site de liaison est représenté par la formule suivante :
5'-O-CₘH₂ₘ-O-3' Formule (1)
(dans laquelle 5' représente l'atome d'oxygène d'une liaison phosphodiester à l'extrémité 5', 3' représente l'atome de phosphore d'une liaison phosphodiester à l'extrémité 3', et m est un nombre entier compris entre 3 et 12) ; et
une deuxième amorce d'amplification d'acide nucléique comportant une deuxième séquence de reconnaissance qui reconnaît une deuxième séquence de bases dans un acide nucléique cible et un marqueur ou une substance se liant à un marqueur à l'extrémité 5'.

15. Composition pour l'hybridation d'une sonde, comprenant un fragment d'ADN double brin comportant
une partie de marqueur simple brin du côté 5' d'un premier brin, qui consiste uniquement en des acides nucléiques naturels et qui est une séquence de marqueur capable de spécifiquement s'hybrider à la sonde,
une partie double brin formée par un appariement de bases, et
un marqueur ou une substance se liant à un marqueur à l'extrémité 5' du second brin,
le premier brin comportant un site de liaison capable d'inhiber ou d'arrêter une réaction d'ADN polymérase disposé entre la séquence de marqueur et la partie double brin ;
où le site de liaison comprend une chaîne alkylène facultativement substituée ayant un nombre d'éléments compris entre 2 et 40, adjacente à un nucléotide dans l'amorce via une liaison phosphodiester ; et
où le site de liaison est représenté par la formule suivante :
5'-O-CₘH₂ₘ-O-3' Formule (1)
(dans laquelle 5' représente l'atome d'oxygène d'une liaison phosphodiester à l'extrémité 5', 3' représente l'atome de phosphore d'une liaison phosphodiester à l'extrémité 3', et m est un nombre entier compris entre 3 et 12).

16. Procédé selon l'une quelconque des revendications 1 à 13, dans lequel m est 3 à 6.

17. Procédé selon la revendication 16, dans lequel m est 3.
